# EUROPEAN PATENT APPLICATION

(11) **EP 3 766 529 A1**
(43) Date of publication of application: **20.01.2021**
(21) Application number: 19767462.5
(22) Date of filing: 12.03.2019
(51) Int. Cl.: A61M 1/16, B01D 61/00

(54) **COMPOSITION FOR PURIFICATION OF BIOFLUIDS**

(30) Priority: 14.03.2018 CN 201810208561
(71) Applicant: Beacon Medcare (HK) Limited, Wanchai, Hong Kong (HK)
(72) Inventor: JIANG, Chen, Shanghai 201203 (CN); SUN, Tao, Shanghai 201203 (CN)
(74) Representative: Lahrtz, Fritz
(86) International application number: PCT/CN2019/077749
(87) International publication number: WO 2019/174560

(57) **Abstract**

Disclosed is a composition for the purification of biofluids, for example, for hemodialysis and peritoneal dialysis, wherein same comprises a penetrant and a toxin-removal reagent that can remove toxins from biofluids under osmotic exchange conditions. Provided are a dialysis solution and a kit comprising the aforementioned composition, a method for removing toxins from biofluids using the aforementioned composition, and a method for treating toxin-related diseases.

## Description

### FIELD OF THE INVENTION

The present invention relates to novel compositions and devices that can be used for purification of biofluids, and methods for purification of biofluids.

### BACKGROUND

Various toxins, such as metabolic waste, generated in organisms can be normally removed through metabolism and kidney clearance. If the metabolic waste cannot be removed normally, it will accumulate in the body and cause toxicity. At present, hemodialysis or peritoneal dialysis is mainly used to reduce the content of metabolic waste in the blood, but the current effect is still relatively limited, especially for certain metabolic waste and toxins that are difficult to remove by osmotic equilibrium. Thus, there remains a need in the art for compositions and methods that are more effective in reducing toxins in the body.

### SUMMARY

In one aspect, the present application provides a composition comprising an osmotic agent and a toxin-removal reagent, wherein the osmotic agent provides an osmotic pressure substantially equal to or higher than that of a biofluid, and the toxin-removal reagent reduces a toxin in the biofluid under a condition for osmosis.

In some embodiments, the toxin-removal reagent reduces the free amount, non-free amount, and/or total amount of the toxin in the biofluid. In some embodiments, the toxin-removal reagent reduces the total amount or non-free amount of the toxin in the biofluid by at least 10%, at least 20%, at least 30%, at least 40%, at least 50%, at least 60%, at least 70%, at least 80%, at least 90%, or at least 95%.

In some embodiments, the toxin-removal reagent adsorbs, non-covalently binds to, covalently binds to, and/or degrades the toxin in the biofluid. In some embodiments, the toxin-removal reagent has one or more characteristics selected from the group consisting of: 1) having a porous structure; 2) capable of forming a charged structure; 3) capable of binding to the toxin through a non-covalent bond or a covalent bond or an ionic bond; and 4) capable of degrading the toxin.

In some embodiments, the toxin-removal reagent has a porous structure, and the porous structure has one or more of the following characteristics: a) having a specific surface area of 70 cm²/g-1000 m²/g; b) having a pore size in a range of 0.1 nm-10 µm; c) having a pore size distribution of 0.1 nm-100 µm; d) having a porosity of about 5-95%; and e) capable of adsorbing the toxin at an adsorption rate of at least 0.2 mg/g.

In some embodiments, the toxin-removal reagent having a porous structure is selected from the group consisting of a silicon-based porous material, a carbon-based porous material, a metal oxide porous material, a polymer porous material, and a metal-organic framework compound based porous material.

In some embodiments, the toxin-removal reagent is capable of forming a charged structure, and the charged structure has a charge density of 0.2-50 µC·cm⁻². In some embodiments, the charged structure comprises charged ions or charged colloids. In some embodiments, the toxin-removal reagent is selected from the group consisting of povidone, crospovidone, silica colloid, micronized silica gel, diatomaceous earth, polyethylene caprolactam-polyvinyl acetate-polyethylene glycol graft copolymer, and nano alumina.

In some embodiments, the toxin-removal reagent has a group capable of forming a non-covalent bond with the toxin (e.g., hydrogen atom, hydroxyl group, amino group, amine group, and carboxyl group, etc.), has a group capable of forming a covalent bond with the toxin (e.g., sulfhydryl group, aldehyde group, hydroxyl group, carboxyl group, etc.), or has a group capable of forming an ionic bond with the toxin (e.g., chloride ion, sulfate ion, calcium ion, and carbonate ion, etc.).

In some embodiments, the toxin-removal reagent is capable of degrading the toxin, and the toxin-removal reagent is a biocatalyst or a chemical catalyst.

In some embodiments, the toxin-removal reagent is selected from the group consisting of activated carbon, povidone, crospovidone, polyethylene caprolactam-polyvinyl acetate-polyethylene glycol graft copolymer, micronized silica gel, diatomaceous earth, and any combination thereof.

In some embodiments, the presence or excessive presence of the toxin in the biofluid can increase the risk of disease, aggravate the disease condition, or impair normal physiological functions. In some embodiments, the toxin comprise a metabolite *in vivo,* an exogenous poisonous substance, or a disease-inducing molecule.

In some embodiments, the toxin is present in the biofluid in a free state, in a bound state with a substance in the biofluid, or both. In some embodiments, the toxin is at least in part reversibly bound with the substance in the biofluid. In some embodiments, the toxin is bound to the substance in the biofluid at a Kd value of at least 10² µmol/L, 10³ µmol/L, 10⁴ µmol/L, 10⁵ µmol/L, 10⁶ µmol/L, or 10⁷ µmol/L. In some embodiments, the Kd value at which the toxin is bound to the substance in the biofluid is at least 10⁵-10⁷ µmol/L.

In some embodiments, at least 10%, at least 20%, at least 30%, at least 40%, at least 50%, at least 60%, at least 70%, at least 80%, or at least 90% of the toxin in the biofluid is present in a bound form. In some embodiments, the toxin comprises indoxyl sulfate, asymmetric arginine, homocysteine, phenylacetic acid, p-cresol, AGE products (3-deoxyglucosone, fructoselysine, glyoxal, pyruvaldehyde, pentosidine), hippuric acid, uremic toxins, hydrogen sulfide, or bilirubin. In some embodiments, the substances bound to the toxin comprises a component in blood tissue, a component in an adipose tissue, a component in a connective tissue, a component in a bone tissue, or the like.

In some embodiments, the toxin-removal reagent binds to the toxin via an adsorption or binding interaction that is different from the binding interaction between the toxin and the substance in the biofluid. In some embodiments, the toxin-removal reagent is different from the substance bound to the toxin in the biofluid.

In some embodiments, the osmotic agent provides an osmotic pressure substantially equal to or higher than 280 mOsm/L, 300 mOsm/L, or 330 mOsm/L. In some embodiments, the osmotic agent comprises a saccharide, an amino acid, a polypeptide, a glycerol, a carbonate, a bicarbonate or an analog thereof, and any combination thereof. In some embodiments, the osmotic agent which is saccharide is selected from a monosaccharide, a oligosaccharide and a polysaccharide; the osmotic agent which is an amino acid is selected from a natural amino acid, an unnatural amino acid, an analog thereof, a derivative thereof, and any combination thereof. In some embodiments, the monosaccharide is selected from glucose, fructose, sorbitol, xylitol, aminosaccharide and a derivative thereof; the oligosaccharide comprises an oligomer of one or more of the monosaccharides; and/or the polysaccharide comprises a polymer of one or more of the monosaccharides. In some embodiments, the osmotic agent which is a saccharide comprises a glucose polymer.

In some embodiments, the amount ratio (weight/weight) of the toxin-removal reagent to the osmotic agent in the composition ranges from 1:1750 to 1:4 (e.g., 1:1750 to 1:5, 1:1500 to 1:4, 1:1500 to 1:5, 1:1000 to 1:5, 1:750 to 1:6; 1:300 to 1:6; 1:75 to 1:6; 1:30 to 1:6; 1:10 to 1:6; 1:1500 to 1:5; 1:1500 to 1:10; 1:1500 to 1:30; 1:1500 to 1:75; 1:1500 to 1:150; 1:1500 to 1:300; or 1:1500 to 1:750).

In some embodiments, when the composition provided herein is formulated into a dialysis solution directly used for osmosis, the content (weight/volume) of the osmotic agent in the dialysis solution is in a range of 0.05%-10%, 0.5%-10%, 1%-10%, 1.5%-10%, 1.5%-9%, 1.5%-8%, 1.5%-7.5%, 1.5%-6%, 1.5%-5%, and the like.

In some embodiments, when the composition provided herein is formulated into a dialysis solution directly used for osmosis, the content (weight/volume) of the toxin-removal reagent in the dialysis solution is in a range of at least 0.0001%, at least 0.0005%, at least 0.001%, at least 0.005%, at least 0.01%, at least 0.025%, at least 0.05%, at least 0.075%, at least 0.1%, at least 0.125%, at least 0.15%, at least 0.175%, at least 0.2%, or at least 0.25%. In some embodiments, the content (weight/volume) of the toxin-removal reagent in the dialysis solution is in a range of not higher than 4%, not higher than 3.5%, not higher than 3.3%, not higher than 3.0%, not higher than 2.8%, not higher than 2.5%, not higher than 2.3%, not higher than 2.0%, not higher than 1.8%, not higher than 1.6%, not higher than 1.4%, not higher than 1.2%, not higher than 1.0%, not higher than 0.8%, not higher than 0.6%, or not higher than 0.4%.

In some embodiments, the composition further contains one or more of buffers, electrolytes and other dialysis components. In some embodiments, the composition is sterilized.

In some embodiments, the composition is a solid preparation, a semi-solid preparation or a liquid preparation.

In some embodiments, the toxin-removal reagent induces osmosis with the biofluid through a semi-permeable substrate. In some embodiments, the semi-permeable substrate is an artificial semi-permeable membrane or a biological semi-permeable membrane. In some embodiments, the biological semi-permeable membrane is selected from the group consisting of blood vessel wall membrane, lymphatic vessel wall membrane, peritoneum, lung membrane, glandular envelope and mucosa.

In some embodiments, the biofluid is selected from blood, tissue fluid, lymph fluid, plasma, serum, a blood product, or a biological product. In some embodiments, the biofluid is inside the body of a subject or outside the body of a subject.

In another aspect, the present application further provides a dialysis solution comprising the composition according to the present application. In some embodiments, the dialysis solution provides pH and/or electrolytes at a physiologically acceptable level.

In another aspect, the present application further provides a kit for purification of a biofluid, comprising the composition according to the present application. In some embodiments, the composition is present in the kit in the form of a single composition, or in the form of two or more components. In some embodiments, the composition or at least one of the components is a solid preparation, a semi-solid preparation or a liquid preparation. In some embodiments, the composition is present in the form of two or more components that are contained in different containers respectively. In some embodiments, the two or more components are respectively contained in two or more containers that can be operably connected via fluid. In some embodiments, the composition is sterilized. In some embodiments, the kit further comprises a semi-permeable substrate that can be used for the purification of the biofluid.

In another aspect, the present application further provides a dialysis device, comprising the composition according to the present application, wherein the device is configured to allow osmosis between the composition and the biofluid to be dialyzed. In some embodiments, the device further comprises a semi-permeable substrate that allows osmosis between the composition and the biofluid. In some embodiments, the dialysis device can be installed on a dialysis host.

In another aspect, the present application further provides a method for reducing a toxins in a biofluid, comprising: a) contacting the biofluid with an osmotic solution comprising the composition according to the present application under a condition to allow osmosis, and b) allowing the composition to reduce the amount of the toxin in the biofluid. In some embodiments, in the method, the toxin in the biofluid is transferred to the osmotic solution through osmosis under the condition to allow osmosis. In some embodiments, the step a) comprises placing the biofluid and the composition respectively at two sides of a semi-permeable substrate. In some embodiments, the osmotic solution is substantially isotonic or hypertonic to the biofluid. In some embodiments, the semi-permeable substrate is an artificial semi-permeable membrane or a biological semi-permeable membrane (such as blood vessel wall membrane, lymphatic vessel wall membrane, peritoneum, lung membrane, glandular envelope and mucosa). In some embodiments, he biofluid is inside the body of a subject. In some embodiments, the step a) comprises applying the osmotic solution to the subject by intraperitoneal infusion. In some embodiments, the biofluid is outside the body. In some embodiments, the step a) comprises applying the osmotic solution to the subject by hemodialysis.

In another aspect, the present application further provides a method of treating or preventing a toxin-related disease or condition, comprising contacting the composition according to the present application with the biofluid of a subject under a condition to allow osmosis, such that the toxin in the biofluid is reduced.

In another aspect, the present application further provides use of the composition according to the present application in the manufacture of a medicament for treating or preventing a toxin-related disease or condition, wherein the composition reduces the toxin in a biofluid in a subject.

### BRIF DESCRIPTION OF DRAWINGS

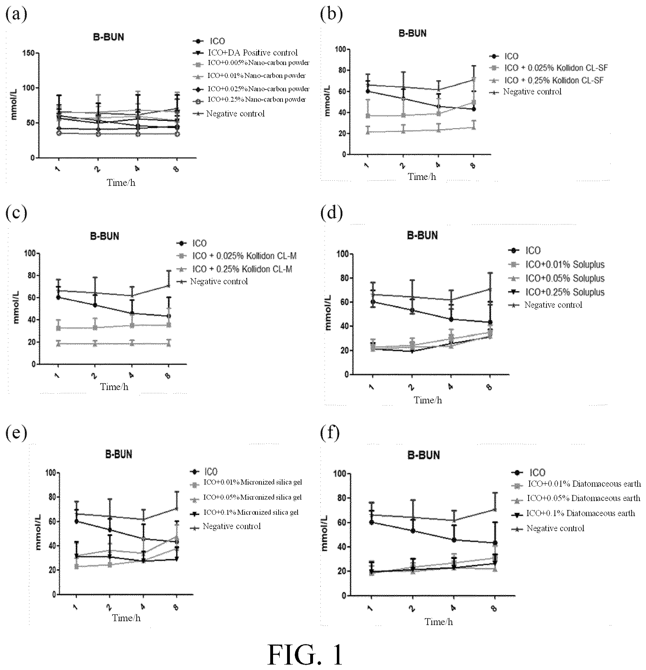

Fig. 1 shows the removal results of urea nitrogen from the blood of rats in an experimental group subjected to rat peritoneal dialysis using icodextrin peritoneal dialysis solutions containing different amounts of toxin-removal reagents, and in the corresponding positive control group (ICO+DA), blank control group (ICO) and negative control group. Fig. 1(a) shows the removal results using icodextrin peritoneal dialysis solutions to which different amounts of nano-carbon powder is added, Fig. 1(b) shows the removal results using icodextrin peritoneal dialysis solutions to which different amounts of Kollidon CL-SF is added, Fig. 1(c) shows the removal results using icodextrin peritoneal dialysis solutions to which different amounts of Kollidon CL-M is added, Fig. 1(d) shows the removal results using icodextrin peritoneal dialysis solutions to which different amounts of Soluplus is added, Fig. 1(e) shows the removal results using icodextrin peritoneal dialysis solutions to which different amounts of micronized silica gel is added, and Fig. 1(f) shows the removal results using icodextrin peritoneal dialysis solutions to which different amounts of diatomaceous earth is added.

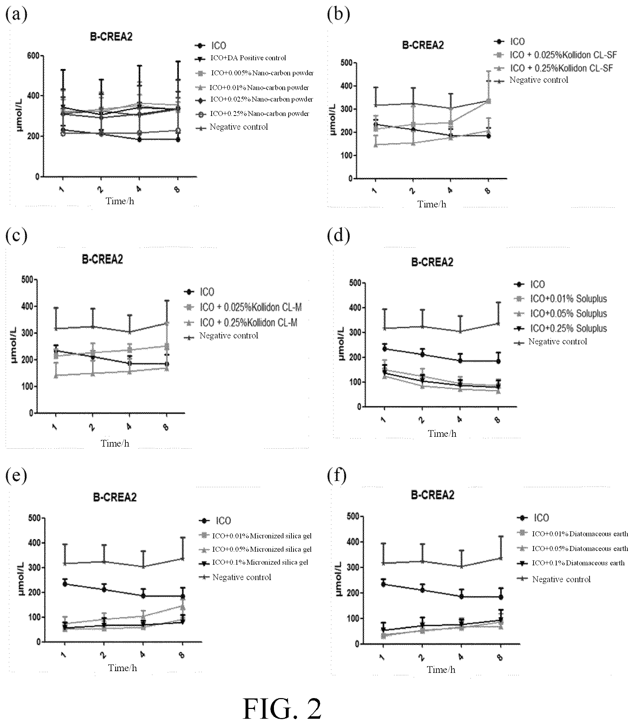

Fig. 2 shows the removal results of creatinine from the blood of rats in an experimental group subjected to rat peritoneal dialysis using icodextrin peritoneal dialysis solutions containing different amounts of toxin-removal reagents, and in the corresponding positive control group (ICO+DA), blank control group (ICO) and negative control group. Fig. 2(a) shows the removal results using icodextrin peritoneal dialysis solutions to which different amounts of nano-carbon powder is added, Fig. 2(b) shows the removal results using icodextrin peritoneal dialysis solutions to which different amounts of Kollidon CL-SF is added, Fig. 2(c) shows the removal results using icodextrin peritoneal dialysis solutions to which different amounts of Kollidon CL-M is added, Fig. 2(d) shows the removal results using icodextrin peritoneal dialysis solutions to which different amounts of Soluplus is added, Fig. 2(e) shows the removal results using icodextrin peritoneal dialysis solutions to which different amounts of micronized silica gel is added, and Fig. 2(f) shows the removal results using icodextrin peritoneal dialysis solutions to which different amounts of diatomaceous earth is added.

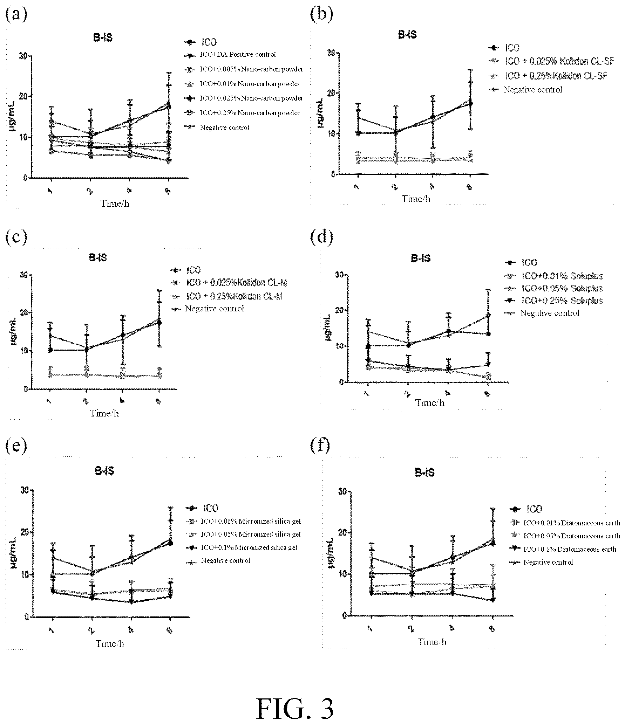

Fig. 3 shows the removal results of indoxyl sulfate from the blood of rats in an experimental group subjected to rat peritoneal dialysis using icodextrin peritoneal dialysis solutions containing different amounts of toxin-removal reagents, and in the corresponding positive control group (ICO+DA), blank control group (ICO) and negative control group. Fig. 3(a) shows the removal results using icodextrin peritoneal dialysis solutions to which different amounts of nano-carbon powder is added, Fig. 3(b) shows the removal results using icodextrin peritoneal dialysis solutions to which different amounts of Kollidon CL-SF is added, Fig. 3(c) shows the removal results using icodextrin peritoneal dialysis solutions to which different amounts of Kollidon CL-M is added, Fig. 3(d) shows the removal results using icodextrin peritoneal dialysis solutions to which different amounts of Soluplus is added, Fig. 3(e) shows the removal results using icodextrin peritoneal dialysis solutions to which different amounts of micronized silica gel is added, and Fig. 3(f) shows the removal results using icodextrin peritoneal dialysis solutions to which different amounts of diatomaceous earth is added.

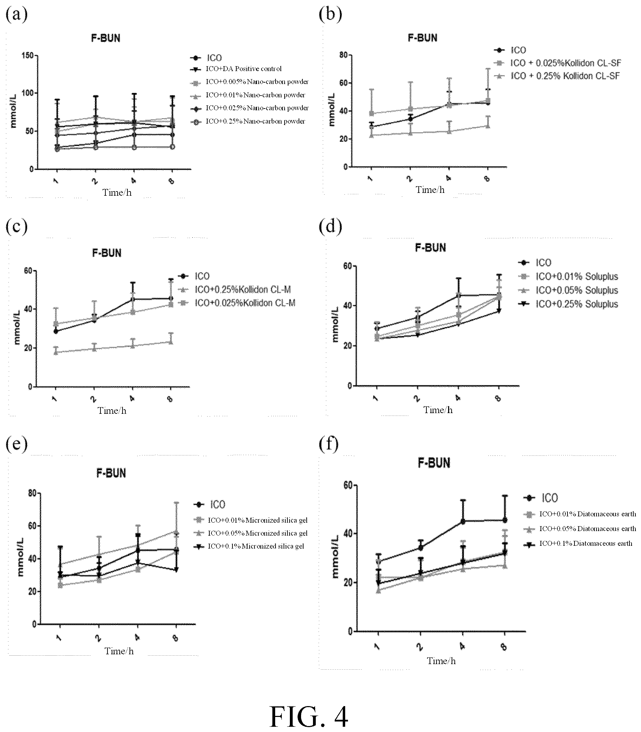

Fig. 4 shows the changes in the content of urea nitrogen in the peritoneal fluid of rats in an experimental group subjected to rat peritoneal dialysis using icodextrin peritoneal dialysis solutions containing different amounts of toxin-removal reagents, and in the corresponding positive control group (ICO+DA), blank control group (ICO) and negative control group. Fig. 4(a) shows the results using icodextrin peritoneal dialysis solutions to which different amounts of nano-carbon powder is added, Fig. 4(b) shows the results using icodextrin peritoneal dialysis solutions to which different amounts of Kollidon CL-SF is added, Fig. 4(c) shows the results using icodextrin peritoneal dialysis solutions to which different amounts of Kollidon CL-M is added, Fig. 4(d) shows the results using icodextrin peritoneal dialysis solutions to which different amounts of Soluplus is added, Fig. 4(e) shows the results using icodextrin peritoneal dialysis solutions to which different amounts of micronized silica gel is added, and Fig. 4(f) shows the results using icodextrin peritoneal dialysis solutions to which different amounts of diatomaceous earth is added.

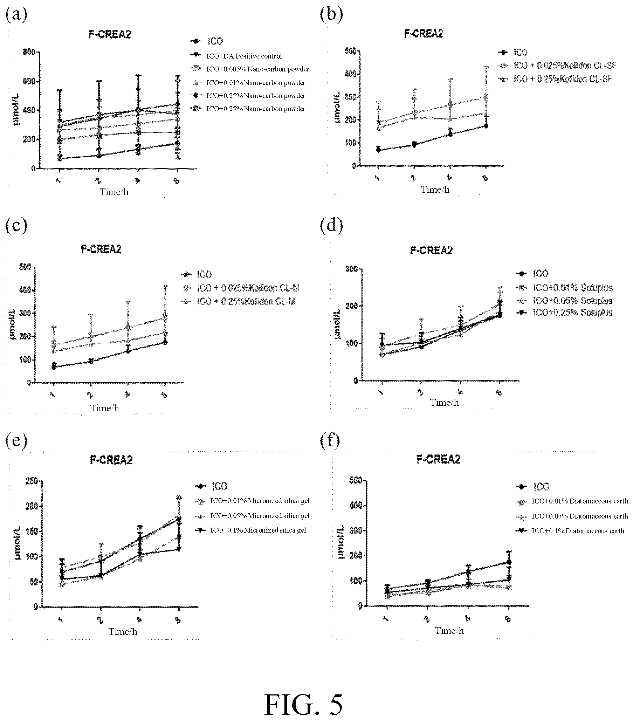

Fig. 5 shows the changes in the content of creatinine in the peritoneal fluid of rats in an experimental group subjected to rat peritoneal dialysis using icodextrin peritoneal dialysis solutions containing different amounts of toxin-removal reagents, and in the corresponding positive control group (ICO+DA), blank control group (ICO) and negative control group. Fig. 5(a) shows the results using icodextrin peritoneal dialysis solutions to which different amounts of nano-carbon powder is added, Fig. 5(b) shows the results using icodextrin peritoneal dialysis solutions to which different amounts of Kollidon CL-SF is added, Fig. 5(c) shows the results using icodextrin peritoneal dialysis solutions to which different amounts of Kollidon CL-M is added, Fig. 5(d) shows the results using icodextrin peritoneal dialysis solutions to which different amounts of Soluplus is added, Fig. 5(e) shows the results using icodextrin peritoneal dialysis solutions to which different amounts of micronized silica gel is added, and Fig. 5(f) shows the results using icodextrin peritoneal dialysis solutions to which different amounts of diatomaceous earth is added.

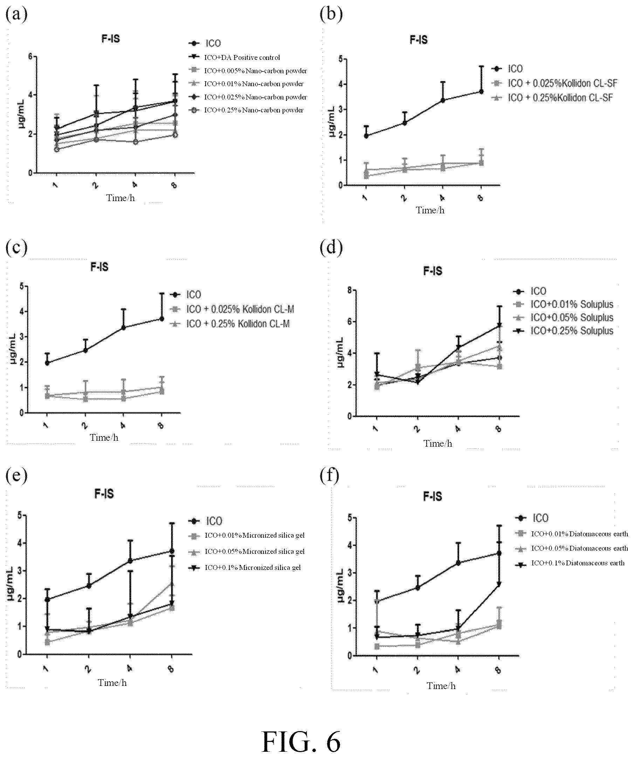

Fig. 6 shows the changes in the content of indoxyl sulfate in the peritoneal fluid of rats in an experimental group subjected to rat peritoneal dialysis using icodextrin peritoneal dialysis solutions containing different amounts of toxin-removal reagents, and in the corresponding positive control group (ICO+DA), blank control group (ICO) and negative control group. Fig. 6(a) shows the results using icodextrin peritoneal dialysis solutions to which different amounts of nano-carbon powder is added, Fig. 6(b) shows the results using icodextrin peritoneal dialysis solutions to which different amounts of Kollidon CL-SF is added, Fig. 6(c) shows the results using icodextrin peritoneal dialysis solutions to which different amounts of Kollidon CL-M is added, Fig. 6(d) shows the results using icodextrin peritoneal dialysis solutions to which different amounts of Soluplus is added, Fig. 6(e) shows the results using icodextrin peritoneal dialysis solutions to which different amounts of micronized silica gel is added, and Fig. 6(f) shows the results using icodextrin peritoneal dialysis solutions to which different amounts of diatomaceous earth is added.

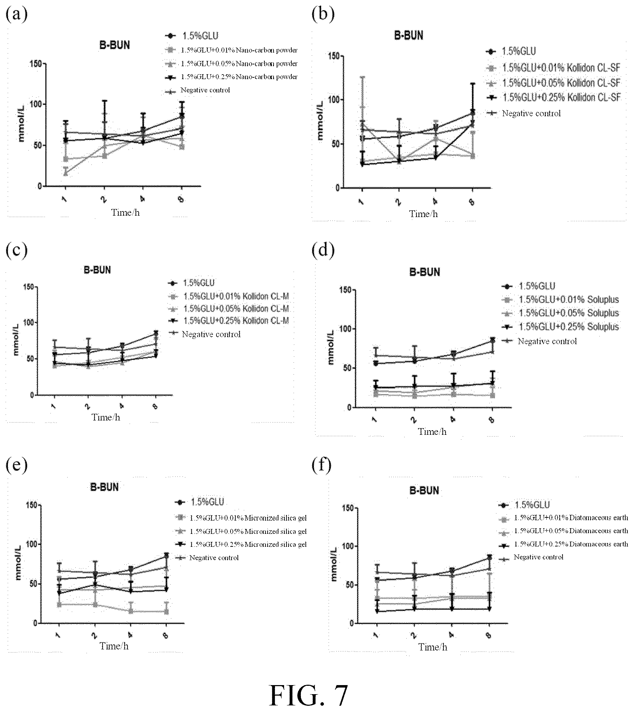

Fig. 7 shows the removal results of urea nitrogen from the blood of rats in an experimental group subjected to rat peritoneal dialysis using glucose peritoneal dialysis solutions containing different amounts of toxin-removal reagents, and in the corresponding blank control group (GLU) and negative control group. Fig. 7(a) shows the removal results using glucose peritoneal dialysis solutions to which different amounts of nano-carbon powder is added, Fig. 7(b) shows the removal results using glucose peritoneal dialysis solutions to which different amounts of Kollidon CL-SF is added, Fig. 7(c) shows the removal results using glucose peritoneal dialysis solutions to which different amounts of Kollidon CL-M is added, Fig. 7(d) shows the removal results using glucose peritoneal dialysis solutions to which different amounts of Soluplus is added, Fig. 7(e) shows the removal results using glucose peritoneal dialysis solutions to which different amounts of micronized silica gel is added, and Fig. 7(f) shows the removal results using glucose peritoneal dialysis solutions to which different amounts of diatomaceous earth is added.

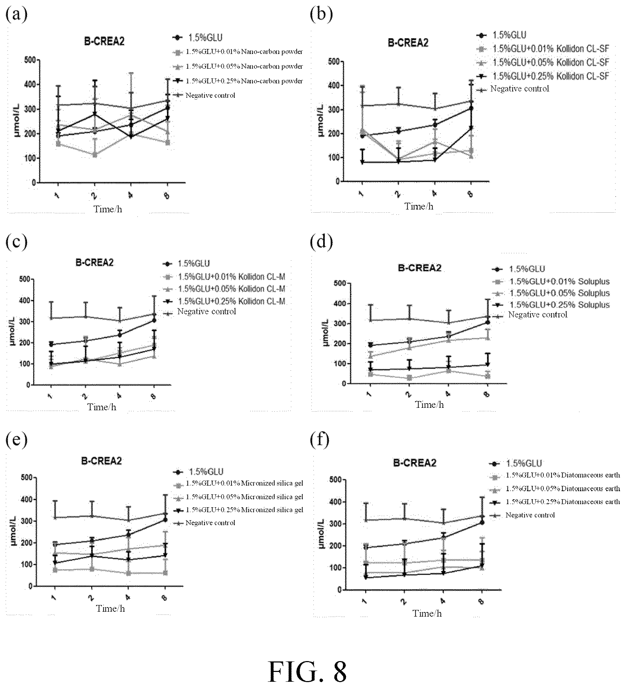

Fig. 8 shows the removal results of creatinine from the blood of rats in an experimental group subjected to rat peritoneal dialysis using glucose peritoneal dialysis solutions containing different amounts of toxin-removal reagents, and in the corresponding blank control group (GLU) and negative control group. Fig. 8(a) shows the removal results using glucose peritoneal dialysis solutions to which different amounts of nano-carbon powder is added, Fig. 8(b) shows the removal results using glucose peritoneal dialysis solutions to which different amounts of Kollidon CL-SF is added, Fig. 8(c) shows the removal results using glucose peritoneal dialysis solutions to which different amounts of Kollidon CL-M is added, Fig. 8(d) shows the removal results using glucose peritoneal dialysis solutions to which different amounts of Soluplus is added, Fig. 8(e) shows the removal results using glucose peritoneal dialysis solutions to which different amounts of micronized silica gel is added, and Fig. 8(f) shows the removal results using glucose peritoneal dialysis solutions to which different amounts of diatomaceous earth is added.

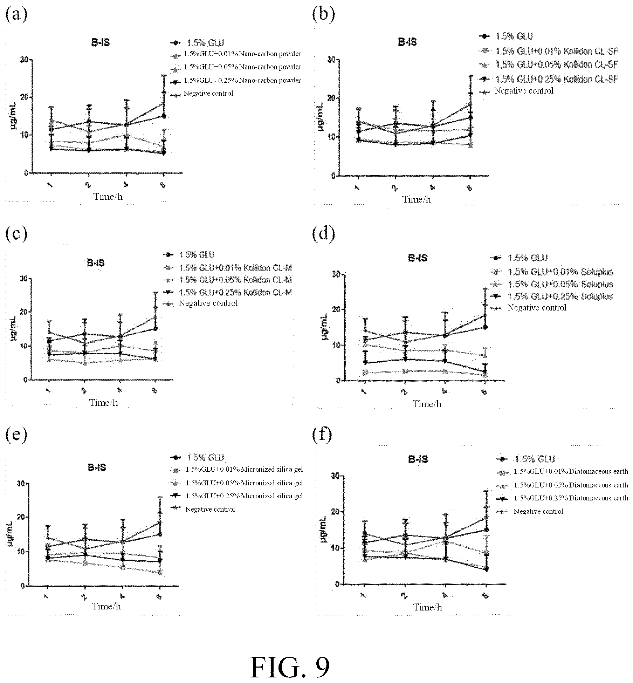

Fig. 9 shows the removal results of indoxyl sulfate from the blood of rats in an experimental group subjected to rat peritoneal dialysis using glucose peritoneal dialysis solutions containing different amounts of toxin-removal reagents, and in the corresponding blank control group (GLU) and negative control group. Fig. 9(a) shows the removal results using glucose peritoneal dialysis solutions to which different amounts of nano-carbon powder is added, Fig. 9(b) shows the removal results using glucose peritoneal dialysis solutions to which different amounts of Kollidon CL-SF is added, Fig. 9(c) shows the removal results using glucose peritoneal dialysis solutions to which different amounts of Kollidon CL-M is added, Fig. 9(d) shows the removal results using glucose peritoneal dialysis solutions to which different amounts of Soluplus is added, Fig. 9(e) shows the removal results using glucose peritoneal dialysis solutions to which different amounts of micronized silica gel is added, and Fig. 9(f) shows the removal results using glucose peritoneal dialysis solutions to which different amounts of diatomaceous earth is added.

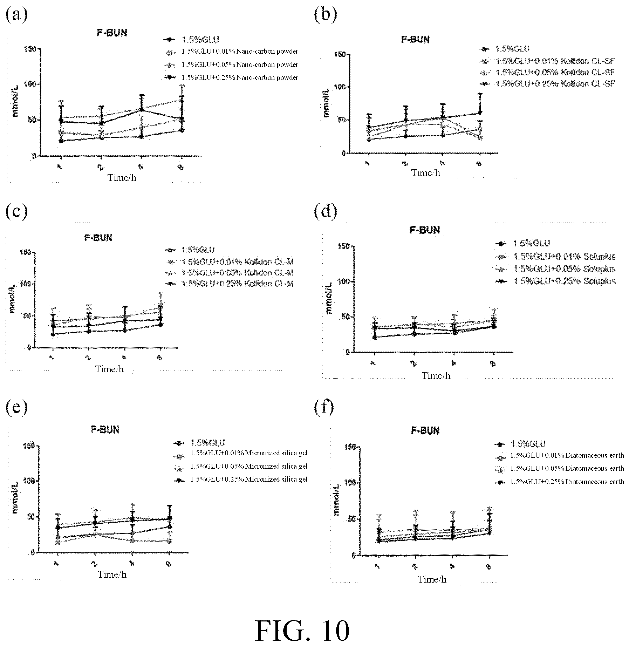

Fig. 10 shows the changes in the content of urea nitrogen in the peritoneal fluid of rats in an experimental group subjected to rat peritoneal dialysis using glucose peritoneal dialysis solutions containing different amounts of toxin-removal reagents, and in the corresponding blank control group (GLU) and negative control group. Fig. 10(a) shows the results using glucose peritoneal dialysis solutions to which different amounts of nano-carbon powder is added, Fig. 10(b) shows the results using glucose peritoneal dialysis solutions to which different amounts of Kollidon CL-SF is added, Fig. 10(c) shows the results using glucose peritoneal dialysis solutions to which different amounts of Kollidon CL-M is added, Fig. 10(d) shows the results using glucose peritoneal dialysis solutions to which different amounts of Soluplus is added, Fig. 10(e) shows the results using glucose peritoneal dialysis solutions to which different amounts of micronized silica gel is added, and Fig. 10(f) shows the results using glucose peritoneal dialysis solutions to which different amounts of diatomaceous earth is added.

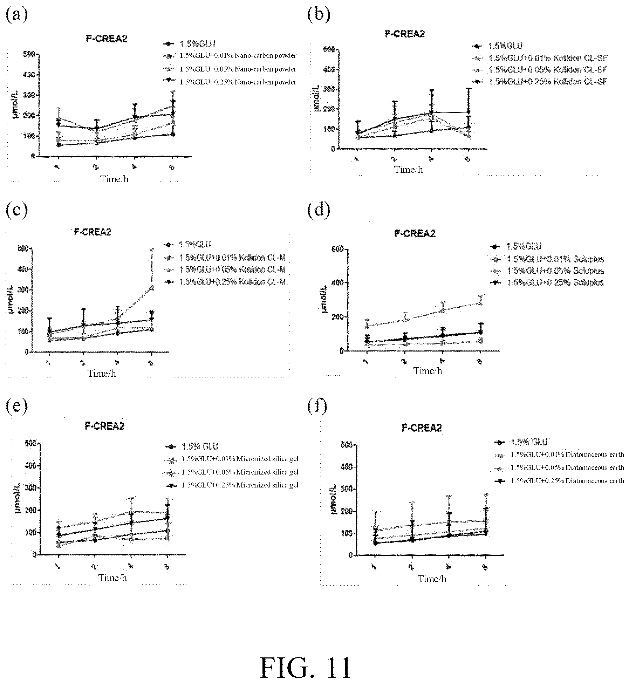

Fig. 11 shows the changes in the content of creatinine in the peritoneal fluid of rats in an experimental group subjected to rat peritoneal dialysis using glucose peritoneal dialysis solutions containing different amounts of toxin-removal reagents, and in the corresponding blank control group (GLU) and negative control group. Fig. 11(a) shows the results using glucose peritoneal dialysis solutions to which different amounts of nano-carbon powder is added, Fig. 11(b) shows the results using glucose peritoneal dialysis solutions to which different amounts of Kollidon CL-SF is added, Fig. 11(c) shows the results using glucose peritoneal dialysis solutions to which different amounts of Kollidon CL-M is added, Fig. 11(d) shows the results using glucose peritoneal dialysis solutions to which different amounts of Soluplus is added, Fig. 11(e) shows the results using glucose peritoneal dialysis solutions to which different amounts of micronized silica gel is added, and Fig. 11(f) shows the results using glucose peritoneal dialysis solutions to which different amounts of diatomaceous earth is added.

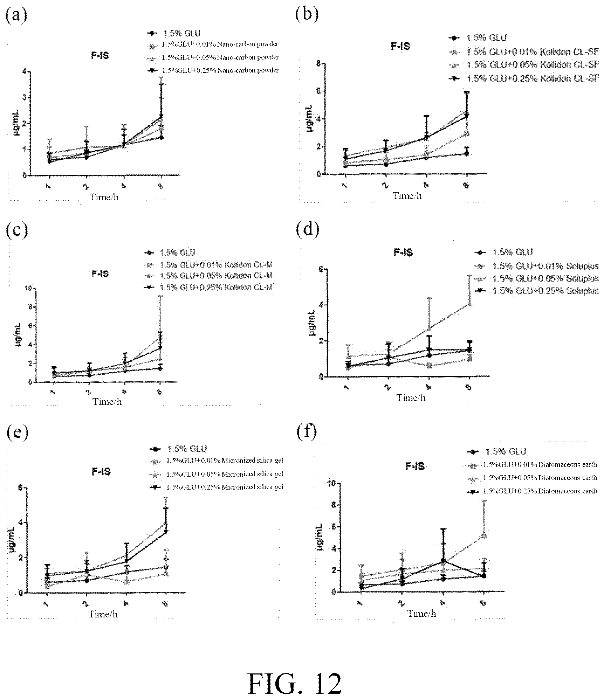

Fig. 12 shows the changes in the content of indoxyl sulfate in the peritoneal fluid of rats in an experimental group subjected to rat peritoneal dialysis using glucose peritoneal dialysis solutions containing different amounts of toxin-removal reagents, and in the corresponding blank control group (GLU) and negative control group. Fig. 12(a) shows the results using glucose peritoneal dialysis solutions to which different amounts of nano-carbon powder is added, Fig. 12(b) shows the results using glucose peritoneal dialysis solutions to which different amounts of Kollidon CL-SF is added, Fig. 12(c) shows the results using glucose peritoneal dialysis solutions to which different amounts of Kollidon CL-M is added, Fig. 12(d) shows the results using glucose peritoneal dialysis solutions to which different amounts of Soluplus is added, Fig. 12(e) shows the results using glucose peritoneal dialysis solutions to which different amounts of micronized silica gel is added, and Fig. 12(f) shows the results using glucose peritoneal dialysis solutions to which different amounts of diatomaceous earth is added.

### DETAILED DESCRIPTION

### Definition

"Osmotic agent" in the present application refers to a reagent that can provide a certain osmotic pressure in a solution.
"Osmotic pressure" in the present application refers to additional pressure that needs to be applied to the liquid surface of a solution to just prevent infiltration. For example, for a semi-permeable substrate (such as a semi-permeable membrane) having different aqueous solution concentrations on both sides, osmotic pressure means the minimum additional pressure applied on the high concentration side to prevent water from permeating from the low concentration side to the high concentration side. The osmotic pressure of a solution is directly proportional to the number of solute particles (number of molecules or ions), which cannot pass through the semi-permeable membrane, contained in a monomer volume solution. The more the solute particles, i.e., the higher the solution concentration, the greater the attraction to water, and the higher the osmotic pressure of the solution. Conversely, the less the solute particles, i.e., the lower the solution concentration, the weaker the attraction to water, and the lower the osmotic pressure of the solution.
A "semi-permeable substrate" in the present application refers to a substrate that selectively allows only a portion of the solute to pass through without allowing another portion of the solute to pass. The semi-permeable substrate may be a material having a certain porosity, where the pores therein can allow passage of molecules small enough, such as water, electrolytes and saccharides. A common example of a semi-permeable substrate is a semi-permeable membrane. However, it should be understood that the semi-permeable substrate is not limited to semi-permeable membranes, but may also be in other forms, such as hollow fibers with semi-permeable properties, or tubular permeable membranes (for example, see Yu Xuemin et al., Preparation Modification of Hemodialysis Membrane and Component Design, Membrane Science and Technology, Vol. 35 No. 4, Pages 110-122).
"Isotonic" in the present application means that the osmotic pressures of two solutions or liquids or fluids are the same. In some embodiments of the present application, the solution containing the composition provided herein is substantially isotonic to, or is hypertonic to the biofluid, so that when the solution and the biofluid are on either side of a semi-permeable substrate, the electrolytes, inorganic salts, saccharides and the like in the biofluid cannot diffuse out or excessively diffuse out to affect or destroy normal biological functions of the biofluid. Substantially isotonic means that the osmotic pressure is close to that of the biofluid, for example, having a difference within a range of no more than plus or minus 10%, plus or minus 8%, plus or minus 5%, plus or minus 3%, or plus or minus 1% from the osmotic pressure of the biofluid. For example, the normal osmotic pressure of human plasma is about 290-310 mmol/L, and the osmotic pressures of human plasma, gastric juice, pancreatic juice, intestinal juice, bile, spinal fluid, and tears are substantially equal. Therefore, the osmotic pressure that is substantially isotonic to the above-mentioned biofluids, such as human blood and plasma, is 290-310 mmol/L, or has a difference within a range of plus or minus 10% (that is, within the range of 260-340 mmol/L).
"Concentration gradient" in the present application refers to a gradient of concentration distribution resulting from a substance that has different free concentrations in fluids on two sides of a semi-permeable substrate. Concentration gradient is the driving force for transporting the substance from the high concentration side to the low concentration side through the semi-permeable substrate. When the concentration of the substance in the fluids on two sides of the semi-permeable substrate are equal, the concentration gradient disappears, and the transport of the substances through the semi-permeable substrate stops.
"Osmosis" in the present application refers to the movement of a substance from the high free concentration side to the low free concentration side through a semi-permeable substrate.
"Toxin" in the present application refers to a substance that adversely affects physiological functions when it is present in an organism (e.g., a human body) or when it is present in an organism at a level higher than a threshold level. The toxins may increase risk of disease, aggravate disease conditions, or impair normal physiological functions. The toxins may include a metabolite in the body, an exogenous poisonous substance, or a disease-causing molecule.
"Biofluid" in the present application may include any fluid from or derived from an organism that may contain toxins. The biofluid may be treated or untreated. For example, examples of biofluid include, but are not limited to, tissue fluid, lymph fluid, blood, plasma, serum, blood products, biological products, and the like.
By "free amount" as used herein refers to the amount of a toxin present in a biofluid that is in a free state.
By "non-free amount" as used herein refers to the amount of a toxin present in the biofluid that is in a non-free state (for example, in a bound (such as bound to a protein), complexed, or chelated state).
By "total amount of toxin" as used herein refers to the total amount of a toxin present in the biofluid, which is the sum of the amount of the toxin in its free state and non-free state.
"Dialysis solution" or "dialysate" in the present application refers to a solution preparation that can be used for dialysis treatment. The dialysis solution may be a liquid preparation suitable for application as it is, or a liquid preparation suitable for formulation before use.

### Composition

In one aspect, the present application provides compositions comprising an osmotic agent and a toxin-removal reagent. The compositions provided herein can be used to remove or reduce toxins present in a biofluid by osmosis with the biofluid.

In the case of osmosis, the biofluid and the osmotic agent are placed on two sides of a semi-permeable substrate. A concentration gradient exists on the two sides of the semi-permeable substrate for a toxin. For example, the toxin is present in the biofluid but not present in the osmotic agent. Therefore, the concentration difference of the toxin on the two sides of the semi-permeable substrate results in a concentration gradient, which promotes the toxin to diffuse from the biofluid side to the osmotic agent side. Where there is only the osmotic agent (without the toxin-removal reagent), after the toxin diffuses into the osmotic agent side, the toxin concentration in the osmotic agent side will increase while the concentration on the biofluid side will decrease. This decreases the concentration difference, and reduces the concentration gradient until the concentrations at both sides are equal, where the concentration gradient becomes zero. When the concentration gradient is zero, the toxin in the biofluid no longer decreases, but is in a state of dynamic equilibrium. At this moment, the concentration of free toxins in the biofluid is the final concentration, and the amount of toxins in the biofluid no longer decreases. Theoretically, assuming that the fluids at the two sides of the semi-permeable substrate have the same volume, the osmotic agent can remove up to 50% of free toxins in the biofluid by concentration gradient itself. However, considering that the volume on the osmotic agent side is always much smaller than the volume of biofluids (e.g., blood), in fact, the free toxin that can be removed by osmosis is always far less than 50%, even less than 20% (for example, in the case of hemodialysis in human). For non-free toxins, such as toxins bound to proteins, the removal amount often depends on the strength of the toxin binding (that is, whether it can readily dissociate to release free toxins), and the amount of toxins in bound state (that is, the ratio of toxins in non-free state to toxins in a free state). For toxins that are mainly present in a bound state and/or are relatively strongly bound (difficult to dissociate), it is difficult to obtain a satisfactory toxin-removal effect by just relying on the concentration gradient provided by the osmotic agent.

Without wishing to be bound by any theory, the compositions provided herein comprise an osmotic agent and a toxin-removal reagent. In the case of osmosis, the biofluid and the composition provided herein are placed on two sides of a semi-permeable substrate. The toxin-removal reagent in the composition provided herein can effectively adsorb, bind and/or degrade the toxins that diffuse into the composition side, thereby continuously reducing the free amount of toxins on the composition side. In turn, the toxins in the biofluid continuously diffuse into the composition side, thereby continuously reducing the total amount of toxins (e.g., the free amount of toxins, the protein-bound amount of toxins and/or the total amount of toxins) in the biofluid, until the toxin-removal reagent can no longer remove more toxins (e.g., reaching a state of adsorption saturation). Compared with the case where only the osmotic agent is present, the toxin-removal reagent in the composition of the present application can remove more free toxins from the biofluid, and can remove the toxins originally present in a bound state in the biofluid.

In some embodiments, the composition provided herein can reduce the free amount, non-free amount, and/or total amount of the toxin in the biofluid. In some embodiments, the composition provided herein can reduce the free amount of toxin in a biofluid to at most 50%, at most 40%, at most 30%, at most 20%, at most 10%, or at most 5% of its initial free amount. The initial free amount refers to the free amount of toxins measured before the biofluid is treated with the composition provided herein.

In some embodiments, the composition provided herein can reduce the total amount of toxins (or non-free amount) in biofluids by at least 10%, at least 20%, at least 30%, at least 40%, at least 50%, at least 60%, at least 70%, at least 80%, at least 90%, or at least 95%. Compared with the biofluid before treatment with the composition provided herein, the total amount of toxins (or non-free amount) in the biofluid after treatment is at most 90%, at most 80%, at most 70%, at most 60%, at most 50%, at most 40%, at most 30%, at most 20%, at most 10%, or at most 5% of that before treatment.

In some embodiments, the composition provided herein can reduce the total amount of a toxin (or free amount or non-free amount) in biofluids to a physiologically beneficial level. "Physiologically beneficial level" refers to the level that can reduce the risk of disease or alleviate symptoms caused by the toxin in the organism, and "disease level" refers to the level found in the organism in a disease state. The physiologically beneficial level may be any level that is greater than or equal to the normal physiological level but is below the disease level, for example, but not limited to, at least 10%, at least 20%, at least 30%, at least 40%, and at least 50% lower than the disease level. For example, the normal physiological level of indoxyl sulfate in normal human plasma is 0.59±0.26 mg/L, but the level of indoxyl sulfate in the uremic state is 53±4.5 mg/L or higher. Therefore, the physiologically beneficial level of indoxyl sulfate may be any level that is greater than or equal to 0.59 but less than 53 mg/L, for example 0.59-48 mg/L, 0.59-42 mg/L, 0.59-36 mg/L, 0.59-32 mg/L, 0.59-26 mg/L, 0.59-20 mg/L, 0.59-15 mg/L, 0.59-10 mg/L, or 0.59-5 mg/L. Similarly, the physiologically beneficial level of creatinine in plasma may be any level that is greater than or equal to 0.5 mg/dL but below 136 mg/dL, for example 0.5-120 mg/dL, 0.5-100 mg/dL, 0.5-80 mg/dL, 0.5-60 mg/dL, 0.5-40 mg/dL, or 0.5-20 mg/dL. The normal physiological level of urea nitrogen (BUN) in normal human plasma is 44-133 µmol/L, and the level in the compensatory stage of renal failure is 133-177 µmol/L, the level in the decompensatory stage of renal failure is 177-442 µmol/L, the level in the renal failure stage is 442-707 µmol/L, and the level in the uremia stage is greater than 707 µmοl/L. Therefore its physiologically beneficial level may be any level below the level of different development stages of renal dysfunction, for example, below 133 µmol/L in the compensatory stage of renal failure, below 177 µmol/L in the decompensatory stage of renal failure, below 442 µmol/L in the renal failure stage, and below 707 µmol/L in the uremia stage.

The free amount, non-free amount, and/or total amount (i.e., the free amount plus the non-free amount) of a toxin in the biofluid can be measured by detection and/or analysis methods known in the art. The useful methods can be, for example, equilibrium dialysis, ultrafiltration, ultracentrifugation, gel filtration, spectroscopy (including ultraviolet-visible spectroscopy, fluorescence spectroscopy, infrared spectroscopy, circular dichroism spectroscopy, Raman spectroscopy, and polarimetry), nuclear magnetic resonance method, optical biosensor method, biochemical analysis method, mass spectrometry, high efficiency affinity chromatography, microcalorimetry, etc. (for details, please refer to "Biopharmaceutics and Pharmacokinetics" Fifth Edition, Editor-in-Chief Liu Jianping, page 96; "Analytical Chemistry" Wuhan University Fifth Edition).

For example, equilibrium dialysis can be used, where biofluid and an isotonic solution are placed on two sides of a semi-permeable membrane that only allows toxins but not biological macromolecules to pass through, so that the toxins in the biofluid diffuse through the semi-permeable membrane without external driving force. When the equilibrium is reached, the concentrations of the toxins in the solutions on two sides of the membrane are measured, and the data on the binding of the biological macromolecules to the toxins can be analyzed by calculation.

Ultrafiltration is a membrane separation technique driven by pressure. For the purpose of separating large and small molecules, the membrane pore size is between 20-1000 Å. A hollow fiber ultrafilter (membrane) has the advantages of high filling density in a unit container, small floor space, etc. In an ultrafiltration process, an aqueous solution is driven by pressure and flows through the membrane surface. The solvent (water) and small molecular solutes smaller than the membrane pores pass through the membrane to become the purified fluid (filtrate), and solutes and solute groups larger than the membrane pores are blocked and discharged with the water flow to become a concentrated solution.

Ultracentrifugation refers to the method of applying powerful centrifugal force to separate, prepare and analyze substances in an ultracentrifuge. The centrifugal speed of the ultracentrifuge is 60000 rpm or higher, and the centrifugal force is about 500000 times the acceleration of gravity. There are two types of ultracentrifuges: preparative ultracentrifuge and analytical ultracentrifuge. The method uses a solvent system that can form a density gradient (in a centrifuge tube, the density continuously increases from the top to bottom) without coagulating or inactivating the separated biologically active substances, and after centrifugation, the substance particles can balance in the corresponding solvent density to form a zone according to the respective specific gravity.

The gel filtration method, also known as the molecular exclusion method, uses a certain type of gel with pores of a certain size, which only allow molecules with the corresponding size to enter the inside of gel particles, while large molecules are excluded.

Ultraviolet-visible spectroscopy are all produced by the transition of valence electrons. The composition, content and structure of substances can be analyzed, determined, and inferred by using the ultraviolet-visible spectrum generated by the molecules or ions of the substances absorbing ultraviolet and visible light and the degree of absorption.

Fluorescent spectroscopy refers to the method for qualitative and quantitative analysis of substances by using the characteristics and intensity of fluorescence generated by certain substances under ultraviolet light irradiation.

Infrared spectroscopy uses the ability of molecules to selectively absorb infrared rays of certain wavelengths, causing the transition of vibrational and rotational energy levels in the molecules. The infrared absorption spectrum of substances can be obtained by detecting the absorption of infrared rays, which is also referred to as molecular vibration spectrum or vibratory rotation spectrum, and qualitative analysis of the substances is carried out by analyzing these spectra.

Circular dichroism uses the phenomenon that the absorption degrees of two circularly polarized lights R and L are different. The relationship between the difference in absorption degree and the wavelength is referred to as circular dichroism, which is a spectroscopic method for determining the asymmetric structure of molecules. In the field of molecular biology, the method is mainly used to determine the three-dimensional structure of proteins, and can also be used to determine the three-dimensional structure of nucleic acids and polysaccharides.

Raman spectroscopy is a kind of scattering spectrum, which analyzes the scattering spectrum with a different frequency from the incident light to obtain information on molecular vibration and rotation, and is an analytical method used in molecular structure research.

Many substances are optically active (also known as optical activity), such as organic compounds containing chiral carbon atoms. When plane-polarized light passes through these substances (liquids or solutions), the plane of vibration of the polarized light rotates to the left or right. This phenomenon is referred to as optical rotation. Polarimetry is a method for measuring the optical activity of a compound using the optical rotation of a substance.

Nuclear magnetic resonance spectroscopy is an analysis method that studies the absorption of radio frequency radiation by atomic nuclei in a strong magnetic field to obtain the information about the molecular structure of compounds.

A biosensor is an instrument that is sensitive to biological substances and converts the concentration thereof into electrical signals for detection. The biosensor is an analytical tool or system made of immobilized biosensitive materials as identification elements (including enzymes, antibodies, antigens, microorganisms, cells, tissues, nucleic acids and other bioactive substances), appropriate physical and chemical transducers (such as oxygen electrodes, photosensitive tubes, field effect tubes, and piezoelectric crystals) and signal amplification devices. The biosensor has the functions of a receiver and a converter.

A biochemical analyzer is one of the important analytical instruments often used in clinical testing. It measures various biochemical indicators, such as transaminase, hemoglobin, albumin, total protein, cholesterol, creatinine, glucose, inorganic phosphorus, amylase and calcium by analyzing blood or other body fluids. Combined with other clinical data, comprehensive analysis can help diagnose diseases, evaluate organ functions, identify concomitant factors, and determine future treatment benchmarks.

Mass spectrometry is a method that uses electric and magnetic fields to separate moving ions (charged atoms, molecules or molecular fragments, including molecular ions, isotope ions, fragment ions, rearrangement ions, multi-charged ions, metastable ions, negative ions and ions produced by ion-molecule interaction) according to their mass-to-charge ratios for detection. The compound composition of the ion can be determined by measuring the accurate mass of the ion. This is because the exact mass of a nuclide is a multidigit decimal, there will never be two nuclides with the same mass, and there will never be a nuclide the mass of which is exactly an integer multiple of the mass of another nuclide. Analyzing these ions can obtain information such as the molecular weight, chemical structure and fragmentation regularity of the compound, and certain relationship between certain ions formed by the decomposition of single molecules.

Affinity chromatography is a chromatography that uses one of two substances with high specific affinity to each other as a stationary phase, and uses different degrees of affinity with the stationary phase to separate components from impurities.

High-performance liquid chromatography refers to that mobile phases such as single solvents with different polarities or mixed solvents in different proportions and buffers are pumped by a high-pressure infusion pump into a chromatographic column equipped with a stationary phase, a sample to be tested is injected through an injection valve and brought into the column by a mobile phase, and after the components in the column are separated, they enter a detector in turn for detection, thereby realizing the analysis of the composition of the sample.

Microcalorimetry (including isothermal titration calorimetry and differential scanning calorimetry) is an important structural biological method developed in recent years to study biothermodynamics and biokinetics. It continuously and accurately monitors and records a calorimetric curve of a changing process through a highly sensitive and highly automated microcalorimeter, providing thermodynamic and kinetic information at the same time in situ, online and without damage.

Before the specific detection or analysis, if necessary, the biofluid can be pretreated by, for example, adding appropriate reagents (e.g., anticoagulants) to avoid undesirable changes (e.g., coagulation) in the biofluid, separating or extracting the supernatant, freeing and releasing the toxins in the bound state, and/or removing irrelevant biological components that may affect detection.

### 1. Toxin-removal reagent

In some embodiments, the toxin-removal reagent in the composition provided herein reduces a toxin in the biofluid under a condition for osmosis. In some embodiments, the toxin-removal reagent in the present application adsorbs, non-covalently binds to, covalently binds to, and/or degrades the toxin. For example, in the case of osmosis with the biofluid to be treated, the toxin-removal reagent can adsorb and bind to toxins that diffuses through from the biofluid, for example, by a porous structure, a charged structure, or a group capable of forming a non-covalent bond, a covalent bond, or an ionic bond. For another example, the toxin-removal reagent can also degrade the toxin diffuses from the biofluid.

In some embodiments, the toxin-removal reagent has a porous structure. Without being bound by any theory, it is believed that the porous structure can increase the surface area of the toxin-removal reagent, thereby better adsorbing or binding to the toxin.

In some embodiments, the porous structure has a specific surface area of 70 cm²/g-1000 cm²/g, (for example, 70 cm²/g-900 m²/g, 70 cm²/g-850 m²/g, 70 cm²/g-800 m²/g, 70 cm²/g-750 m²/g, 70 cm²/g-700 m²/g, 70 cm²/g-650 m²/g, 70 cm²/g-600 m²/g, 70 cm²/g-550 m²/g, 70 cm²/g-500 m²/g, 70 cm²/g-450 m²/g, 70 cm²/g-400 m²/g, 70 cm²/g-350 m²/g, 70 cm²/g-300 m²/g, 70 cm²/g-250 m²/g, 70 cm²/g-200 m²/g, 70 cm²/g-180 m²/g, 70 cm²/g-160 m²/g, 70 cm²/g-140 m²/g, 70 cm²/g-120 m²/g, 70 cm²/g-100 m²/g, 70 cm²/g-80 m²/g, 70 cm²/g-60 m²/g, 70 cm²/g-40 m²/g, 700 cm²/g-160 m²/g, 7000 cm²/g-160 m²/g, 7 m²/g-160 m²/g, 20 m²/g-160 m²/g, 40 m²/g-160 m²/g, etc.). For example, the specific surface area of diatomaceous earth is 40-65 m²/g, and the specific surface area of porous silica may be 70-600 m²/g (for example 70-500 m²/g, 70-400 m²/g, 70-300 m²/g, 70-200 m²/g, 70-100 m²/g, 70-90 m²/g, 70-85 m²/g, etc.). The specific surface area of a material having porous structure can be measured by known methods (such as gas adsorption method, fluid permeation method, and mercury intrusion method), see, for example, Lowell, S., et al., Characterization of porous solids and powders: surface area, pore size and density, published by Springer, 2004.

In some embodiments, the toxin-removal reagent having the porous structure may have a pore size of 0.1 nm-10 µm. Depending on the pore size, materials having a porous structure can also be divided into macroporous materials (for example, having a pore size greater than 50 nm), mesoporous materials (for example, having a pore size in a range of 2- 50 nm), and microporous materials (for example, having a pore size less than 2 nm). Microporous materials suitable for use in the present disclosure include, but are not limited to, amorphous silica, inorganic sol, crystalline molecular sieve, activated carbon and the like. Mesoporous materials suitable for use in the present disclosure include, for example, but are not limited to, silica mesoporous materials. Macroporous materials suitable for use in the present disclosure include, for example, but are not limited to, macroporous molecular sieve materials (for example, see Science (2011), 333: 1131), and macroporous silica gel (for example, having a pore size of about 50 nm, a pore volume of about 2.5-3.0 ml/g, a bulk density of about 180-220 g/L, a specific surface area of about 150-200 m²/g, and a particle size of about 80 mesh).

The characterization of the pore size can be determined by a known method (for example, direct section observation method (transmission electron microscope), bubble method, diffusion method, mercury intrusion method, gas adsorption method, centrifugal force method, suspension filtration method, and X-ray small angle scattering method).

In some embodiments, the toxin-removal reagent having the porous structure has a suitable pore size distribution. For example, at least 80% of the pores have a pore size distribution of 0.1 nm-100 µm (for example, 0.1 nm-80 µm, 0.1 nm-60 µm, 0.1 nm-40 µm, 0.1 nm-20 µm, 0.1 nm-10 µm, 0.1 nm-1 µm, 0.1 nm-100 nm, 0.1 nm-10 nm, 0.1 nm-1 nm, 1 µm-80 µm, 10 µm-80 µm, 20 µm-80 µm, 40 µm-80 µm, 60 µm-80 µm).

In some embodiments, the toxin-removal reagent having the porous structure has a porosity of 5-95% (for example, 5-90%, 5-80%, 5-70%, 5-60%, 5-50%, 5-40%, 10-90%, 20-90%, 30-90%, 40-90%, 50-90%, and 60-90%).

In some embodiments, the toxin-removal reagent having the porous structure can adsorb the toxins at an adsorption rate of at least 0.2 mg/g (for example, at least 0.3 mg/g, at least 0.5 mg/g, at least 0.7 mg/g, at least 0.9 mg/g, at least 1.0 mg/g, at least 2 mg/g, at least 3 mg/g, at least 4 mg/g, at least 5 mg/g, at least 6 mg/g, at least 7 mg/g, at least 8 mg/g, and at least 9 mg/g). The adsorption rate refers to the weight of the toxin adsorbed by the toxin adsorption reagent per unit weight. For example, the adsorption rate of 0.2 mg/g means that each gram of toxin adsorption reagent can adsorb 0.2 mg of toxin.

In some embodiments, the toxin-removal reagent having a porous structure may include, for example, but are not limited to, a silicon-based porous material, a carbon-based porous material, a metal oxide porous material (for example, porous alumina, hydrotalcite-like material), a polymer porous material, and a metal-organic framework compound based porous material.

The silicon-based porous material provided herein refers to a silicon-based multiphase material composed of a solid phase and a large number of pores. Similarly, the carbon-based porous material in the present application refers to a carbon-based multiphase material composed of a solid phase and a large number of pores. The silicon-based porous materials and carbon-based porous materials generally have a stable framework structure, have a certain specific surface area and pore size distribution, and preferably have a regular porous structure.

Examples of the silicon-based porous materials include, but are not limited to, diatomaceous earth, porous silica (e.g., Parteck® SLC 500), zeolite, quartz sand, carclazyte, microporous glass, porous ceramics, mesoporous silica, clay, and molecular sieves.

Examples of the carbon-based porous materials include, but are not limited to, activated carbon, expanded graphite, and mesoporous carbon.

Examples of the metal oxide porous materials include, but are not limited to, porous alumina, hydrotalcite-like materials, and the like.

Examples of the polymer porous materials include, but are not limited to, polystyrene porous beads, inulin, polyethylene, polypropylene, polytetrafluoroethylene, polyvinylidene fluoride, polyethyl vinyl acetate, polycarbonate, polyether ether ketone, and polyether sulfone.

Examples of the metal-organic framework compound based porous materials include, but are not limited to, MOF-5, MOF-177, MOF-180, MOF-205, MOF-210, [COII(BPB)]•3DMF, ED-MIL-101, and SZ/MIL-101.

An exemplary toxin-removal reagent having a porous structure is activated carbon. In some embodiments, the effective radius of the pores of activated carbon may be 1-10000 nm, the radius of small pores may be less than 2 nm, the radius of mesopores may be 2-100 nm, and the radius of macropores may be 100-10000 nm. The small pore volume may be 0.15-0.90 mL/g, the mesopore volume may be 0.02-0.10 mL/g, and the macropore volume may be 0.2-0.5 mL/g. In some embodiments, the adsorption capacity of activated carbon to small molecule sulfadiazine is 0.54 mg/g.

Another exemplary toxin-removal reagent having a porous structure is diatomaceous earth. In some embodiments, the density of the diatomaceous earth is 1.9-2.3 g/cm³, the bulk density is 0.3-0.65 g/cm³, the specific surface area is 40-65 m²/g, the pore volume is 0.45-0.98 m³/g, the water absorption is 2-4 times of its own volume, and the fineness is 100-2000 mesh.

Another exemplary toxin-removal reagent having a porous structure is porous silica. In some embodiments, the specific surface area of porous silica is 10-1000 m²/g (for example, 70-600 m²/g) (for example, Parteck® SLC 500 is 500 m²/g), the density is 100-1000 kg/m³, and the pore size distribution is 1-1000 nm.

In some embodiments, the toxin-removal reagent can form a charged structure. Without being bound by any theory, it is believed that the toxin-removal reagent with a charged structure can adsorb toxins with opposite charges through for example, electrostatic adsorption. For example, positively charged toxin-removal reagents (such as spermidine, spermine, and nano alumina) can remove negatively charged toxins (such as indoxyl sulfate) by adsorption, or negatively charged toxin-removal reagents (such as porous silica gel, and micronized silica gel) can remove positively charged toxins (such as spermidine, and spermine) by adsorption.

In some embodiments, the charged structure comprises charged ions or charged colloids. Some sub-micron or nano-scale particles or polymers can form colloidal solutions when dispersed in a medium (such as water or aqueous solutions). The colloidal particles in the colloidal solution can have certain charges due to the adsorption of some charged ions or the ionization of the groups on the surface. In some embodiments, the charged structure has a charge density of at least 0.2 µC•cm⁻² or 0.2-50 µC•cm⁻² (for example: 0.2-40 µC•cm⁻², 0.2-30 µC•cm⁻², 0.2-20 µC•cm⁻², 0.2-10 µC•cm⁻², 2-50 µC•cm⁻², 10-50 µC•cm⁻² 20-50 µC•cm⁻², 30-50 µC•cm⁻², or 40-50 µC•cm⁻²). The charged properties of the charged structure can be detected by known methods, for example, by measuring the conductivity of a colloidal solution (for example, see: Zhou Hongwei et al., Acta Phys. -Chim. Sin. 2013, 29 (6), 1260-1265).

In some embodiments, the toxin-removal reagents that can form a charged structure (such as a charged colloidal structure) include povidone (e.g., a polymer of 1-vinyl-2-pyrrolidone), crospovidone (e.g., a cross-linked polymer of 1-vinyl-2-pyrrolidone), silica colloid, micronized silica gel (also referred to as colloidal silica), diatomaceous earth, polyethylene caprolactam-polyvinyl acetate-polyethylene glycol graft copolymer (for example, see US patents US8,636,929, US9,011,912, PCT international patent application WO/2013/090842A3, CAS No. 402932-23-4, trade name Soluplus®) etc.

In some embodiments, the toxin-removal reagent is polyethylene caprolactam-polyvinyl acetate-polyethylene glycol graft copolymer. In some embodiments, the appropriate molecular weight range can be 90,000-140,000 g/mol. In some embodiments, in the copolymer, the weight ratio of polyethylene glycol to polyethylene caprolactam to polyvinyl acetate is 13:57:30.

In some embodiments, the toxin-removal reagent can bind to the toxins by a non-covalent bond, a covalent bond, or an ionic bond. Non-covalent binding may be based on any possible non-covalent bonds, such as hydrogen bond, hydrophobic interaction, electrostatic interaction, chelation, van der Waals force, π-π stacking interaction, etc. For example, when colloidal silica is used as the toxin-removal reagent, it can form hydrogen bonds with the toxin creatinine, thereby removing creatinine from biofluid. For another example, when polyethylene caprolactam-polyvinyl acetate-polyethylene glycol graft copolymer (e.g., Soloplus) is used as the toxin-removal reagent, it can bind to the toxin indoxyl sulfate through electrostatic interaction, hydrogen bonding and the like, thereby removing indoxyl sulfate from biofluid. For yet another example, a metal chelating agent may be used as the toxin-removal reagent to form a chelate with the toxin copper ions, thereby removing copper ions from biofluid.

In some embodiments, the toxin-removal reagent has a group capable of forming a non-covalent bond with toxins, such as hydrogen atom, hydroxyl group, amino group, amine group, carboxyl group, etc.

In some embodiments, the toxin-removal reagent can bind to toxins in a covalent form. Covalent binding may be based on any covalent bonds that may be formed under a condition for osmosis, such as disulfide bonds, ester bonds, hydrazone bonds, acylhydrazone bonds, acylhydrazide bonds, etc. For example, when sulfhydryl-modified silica is used as the toxin-removal reagent, it can form disulfide bonds with toxins having glutathione or cysteine, thereby removing such toxins from the biofluid. For another example, when aldehyde-modified silica is used as the toxin-removal reagent, it can form Schiff bases (hydrazone bonds) with compounds having amino groups (such as amino acid and polypeptide toxins), thereby removing such toxins from biofluid.

In some embodiments, the toxin-removal reagent has a group capable of forming a covalent bond with toxins, such as hydrogen atom, hydroxyl group, carboxyl group, sulfhydryl group, aldehyde group, amino group, acylhydrazone bond, and acylhydrazine bond. In some embodiments, the toxin-removal reagent is silica modified with the hydroxyl group, carboxyl group, sulfhydryl group, aldehyde group, amino group, acylhydrazone bond, or acylhydrazine bond.

In some embodiments, the toxin-removal reagent can bind to toxins through ionic bonds. The ionic bonds may be any combination based on ion exchange that may be formed under a condition for osmosis. In some embodiments, the toxin-removal reagent has a group capable of forming an ionic bond with toxins, such as chloride ion, sulfate ion, calcium ion, and carbonate ion. For example, chloride ions in ion exchange resin can form ionic bonds with the toxin silver ions, thereby removing the toxin silver ions from biofluid. The toxins suitable for removal in such a way may be toxic metal ions, such as copper ions, aluminum ions, mercury ions, barium ions, lead ions, chromium ions, cadmium ions, and silver ions.

In some embodiments, the toxin-removal reagent degrades the toxin. In some embodiments, the toxin-removal reagent is a biocatalyst or a chemical catalyst. The biocatalyst may be a biological macromolecule with catalytic reaction function, such as an enzyme. For example, the toxin-removal reagent may be an enzyme and the toxin may be a substrate. Through the interaction between the enzyme and the substrate, the enzyme as the toxin-removal reagent can decompose or digest the substrate. For example, as the toxin-removal reagent, hydrolase can hydrolyze ester toxins and remove ester toxins from biofluid. As the toxin-removal reagent, pancreatin can digest and hydrolyze protein toxins. As the toxin-removal reagent, nuclease can digest and hydrolyze nucleic acid toxins. The chemical catalyst may be chemical molecules with catalytic reaction functions. An exemplary chemical catalyst may be manganese dioxide, which can decompose and remove reactive oxygen species (ROS) from biofluid when used as a toxin-removal reagent. In some embodiments, the toxin-removal reagent has a group or domain capable of degrading or destroying toxin molecules, such as the catalytic domain of an enzyme, or the catalytic group of a chemical molecule.

In some embodiments, the toxin-removal reagent is selected from activated carbon, povidone, crospovidone, polyethylene caprolactam-polyvinyl acetate-polyethylene glycol graft copolymer(for example, see US patents US8,636,929, US9,011,912, PCT international patent application WO/2013/090842A3, CAS No. 402932-23-4, trade name Soluplus®), micronized silica gel, diatomaceous earth, and any combination thereof.

In some embodiments, the toxin-removal reagent is not necessarily a physiologically acceptable material. Those skilled in the art can understand that under the condition for osmosis, the toxin-removal reagent is physically separated from the biofluid, but toxins in the biofluid can diffuse to the side of the toxin-removal reagent. The toxin-removal reagent preferably does not diffuse to the biofluid under the condition for osmosis, so the toxin-removal reagent is not necessarily physiologically acceptable.

### 2. Toxins

The toxin-removal reagent in the composition provided herein can remove toxins from biofluid.

### 2.1 Types of toxins

In some embodiments, the toxins comprise one or more metabolites *in vivo,* exogenous poisonous substances (such as drugs, pesticides, chemical poisons, food-borne poisons, or exogenous biological toxins), or disease-inducing molecules.

"Metabolites *in vivo"* are products derived from the metabolism of cells in an organism or the digestion and metabolism of food *in vivo,* or the products produced by the organism in a disease state. The metabolites *in vivo* include, for example, urea (produced by protein metabolism), creatinine (produced by muscle metabolism), indoxyl sulfate (produced at least partly by the metabolism of indole substances in food), etc. The metabolites *in vivo* can usually be excreted through urine, but when renal function declines and renal clearance decreases, the metabolites accumulate in the blood and tissues and participate in the development of uremic syndrome. Toxins that participate in or cause uremia due to accumulation *in vivo* are also referred to as uremic toxins. In some embodiments, the composition of the present application can remove uremic toxins.

In some embodiments of the present application, the metabolites *in vivo* may include urea, creatinine, uric acid, guanidine-ADMA, β₂-microglobulin, cytokines, parathyroid hormone, indoxyl sulfate, homocysteine, p-cresol, hippuric acid, reactive oxygen species (ROS, such as peroxides, superoxides, hydroxyl radicals, and single oxygen atoms), uremic toxins (such as AGE products (3-deoxyglucosone, fructoselysine, glyoxal, pyruvaldehyde, pentosidine), 1-methyladenosine, 1-methylguanosine, 1-methylinosine, asymmetric dimethylarginine, α-keto-δ-guanidinovaleric acid, α-N-acetylarginine, arabitol, arginine, benzyl alcohol, β-guanidinopropionic acid, β-lipotrophic hormone, creatine, cytidine, sodium N,N-dimethylglycinate, erythritol, γ-guanidinobutyric acid, hypoxanthine, malondialdehyde, mannitol, methylguanidine, inositol, N,N-dimethylguanosine, N-acetyl cytosine nucleoside, N-threonyl carbamoyl adenosine phosphate, orotic acid, orotidine, oxalate, phenylacetylglutamine, pseudouridine, symmetric dimethylarginine, sorbitol, taurocyamine, threitol, thymine, uracil, uridine, xanthosine, 2-methoxyresorcinol, 3-deoxyglucosone, 3-carboxy-4-methyl-5-propyl-2-furanpropionic acid (CMPF), fructosyllysine, homocysteine, hydroquinone, indole-3-acetic acid, kynurenine, kynurenic acid, leptin, melatonin, methylglyoxal, Nε-carboxymethyllysine, cresol, pentoside, phenol, p-hydroxyhippuric acid, butanediamine, quinolinic acid, retinol conjugated protein, spermidine, spermine, adrenomedullin, atrial natriuretic peptide, β-endorphin, cholecystokinin, Clara cell protein (CC16), human complement factor D, cystatin C, degranulation inhibitor protein Ic, δ-sleep-inducing peptide, endothelin, hyaluronic acid, interleukin-1β, interleukin-6, κ-immunoglobulin light chain, λ-immunoglobulin light chain, methionine-enkephalin, neuropeptide Y, parathyroid hormone, tumor necrosis factor-α), hydrogen sulfide, bilirubin, and the like.

In addition to the metabolites *in vivo,* the toxins described in the present application may also be exogenous poisonous substances. Exogenous substances may be substances (such as chemical poisons) that are not naturally present in the organism, or be mainly ingested into the organism through exogenous routes (such as natural hormone drugs taken). In some embodiments, the exogenous poisonous substances include drugs, pesticides, chemical poisons, food-borne poisons, or exogenous biological toxins.

Misuse, overdose or abuse of drugs may cause poisoning. In some embodiments of the present invention, the poisonous drug may include a sedative and hypnotic drug, an antipsychotic drug, a cardiovascular and cerebrovascular drug, an antipyretic and analgesic drug, an antiparasitic drug, an antimicrobial drug, an anesthetics and anesthetic assistant, a respiratory system drug, a circulatory system drug, a digestive system drug, a urinary system drug, a blood system drug, a metabolism and endocrine drug, an antiallergic drug, a tumor treatment drug, an immunomodulatory drug, an obstetrics and gynecology drug, a male drug, an anti-inflammatory drug, a traditional Chinese medicine, and the like.

Sedative and hypnotic drugs include, for example, barbiturates, anxiolytics, antihistamines, antipsychotics, analgesics, and other sedative and hypnotic drugs. Barbiturates are derivatives of barbituric acid, such as barbital, phenobarbital, amobarbital, secobarbital, and thiopental sodium. Anxiolytic drugs include benzodiazepines (such as diazepam, nitrazepam, estazolam, alprazolam, and zopiclone), tranquilizers (such as meprobamate, carisoprodol, and clonazepam), diphenylmethanes (such as atarax, captodiamine, and piperilate), and others (such as fenarol, trimeprazine tartrate, trimetozine, oryzanol, and tacitin). Other sedative and hypnotic drugs may include, for example, chloral hydrate, amigin, glutethimide, methyprylon, adalin, ethinamate, gastrodine, bromide, etc.

Antipsychotic drugs, also known as major tranquilizers or neuroleptic, are drugs used to treat schizophrenia and other psychotic disorders. Common antipsychotic drugs include phenothiazines (chlorpromazine, thioridazine, perphenazine, trifluoperazine, fluphenazine, and fluphenazine decanoate), butyrophenones (haloperidol, haloperidol decanoate, and penfluridol), benzamides (sulpiride), dibenzodiazepines (clozapine, and olanzapine), benzisoxazoles (risperidone), benzisothiazoles (ziprasidone), dibenzothiazepines (quetiapine) and quinolones (aripiprazole).

Cardiovascular and cerebrovascular drugs are drugs that act on the cardiovascular system. Common cardiovascular and cerebrovascular drugs include antianginal drugs (such as nitroglycerin, β-adrenergic receptor antagonists, and calcium ion antagonists), antiarrhythmic drugs (such as lidocaine, amiodarone, and verapamil), antihypertensive drugs (such as valsartan, benazepril, metoprolol, and nifedipine), anticardiac insufficiency drugs (such as spironolactone, furosemide, and irbesartan), peripheral vasodilators (such as hydralazine, sodium nitroprusside, nitrates, α-adrenergic receptor blocking agents, calcium antagonists, and angiotensin converting enzyme inhibitors), etc.

Antipyretic and analgesic drugs are drugs that can restore the body temperature of fever patients, but have no effect on the body temperature of normal people. Such drugs also have moderate analgesic effects, but the strength is not as strong as morphine and synthetic substitutes thereof. Commonly used antipyretic and analgesic drugs can be divided according to chemical structures into salicylic acids (such as aspirin, and lysine aspirin), anilines (such as acetaminophen), pyrazolones (such as analgin), indoles (such as indomethacin), aryl acetic acids (such as diclofenac sodium), aryl propionic acids (such as ibuprofen), and selective cyclooxygenase-2 inhibitors (such as celebrex, nimesulide, and meloxicam).

Common poisonous pesticides include organophosphates, herbicides, pyrethroids, abamectins, and rodenticides. Other common pesticides include paraquat, dichlorvos, dimethoate, glyphosate, glufosinate, chlorpyrifos, omethoate, bromadiolone, brodifacoum, phoxim, imidacloprid, dimehypo, parathion, phorate, demeton, trichlorfon, malathion, benzene hexachloride, clofenotane, dieldrin, endrin, aldrin, aluminum phosphide pesticide, fenvalerate, fluoroacetamide, tetramine, pentachlorophenol, dinitrocresol, diuron, dichlobenil, nereistoxin, rotenone, nicotine, methanonaphthalene chloride, formamidine, bacillus thuringiensis preparation, diphacinone sodium salt, warfarin, pyrinuron, amobam, ziram, methylmercury, and 2,4D butyl ester.

Examples of the chemical poisons may include, but are not limited to, a toxin derived from poisonous plants (such as Abrus precatorius, oleander, datura, colchicum, palythoa toxica, lupin, and rattlebush), a cyanide (such as sodium cyanide, potassium cyanide, calcium cyanide, barium cyanide, cobaltous cyanide, cobalthous cyanide, potassium cobalt cyanide, nickel cyanide, nickel potassium cyanide, copper cyanide, silver cyanide, silver potassium cyanide, zinc cyanide, cadmium cyanide, mercury cyanide, mercuric potassium cyanide, lead cyanide, cerium cyanide, cuprous cyanide, potassium gold cyanide, bromine cyanide, hydrogen cyanide, and hydrocyanic acid), xylene, automobile anti-freeze fluid, arsenic trioxide, sodium arsenite, potassium arsenite, arsenic pentoxide, arsenic trichloride, potassium selenite, sodium selenate, potassium selenate, selenium oxychloride, mercury chloride, mercuric oxycyanide, cadmium oxide, nickel carbonyl, iron pentacarbonyl, sodium azide, barium azide, hydrazoic acid, hydrogen fluoride, yellow phosphorus, sodium phosphide, potassium phosphide, magnesium phosphide, aluminum phosphide, fluorine, phosphine, arsine, hydrogen selenide, stibine, nitrogen monoxide, dinitrogen tetroxide, sulfur dioxide, chlorine dioxide, oxygen difluoride, chlorine trifluoride, phosphorus trifluoride, sulfur tetrafluoride, silicon tetrafluoride, chlorine pentafluoride, phosphorus pentafluoride, selenium hexafluoride, tellurium hexafluoride, tungsten hexafluoride, bromine chloride, cyanogen chloride, bromophosgene, cyanogen, cyanogen iodide, arsenic, calcium arsenite, strontium arsenite, barium arsenite, iron arsenite, copper arsenite, silver arsenite, zinc arsenite, lead arsenite, antimony arsenite, copper acetoarsenite, arsenic acid, meta-arsenic acid, pyroarsenic acid, ammonium arsenate, sodium arsenate, sodium meta-arsenate, disodium hydrogen arsenate, disodium hydrogen arsenate, sodium dihydrogen arsenate, potassium arsenate, potassium dihydrogen arsenate, magnesium arsenate, calcium arsenate, barium arsenate, iron arsenate, ferrous arsenate, copper arsenate, silver arsenate, zinc arsenate, mercury arsenate, lead arsenate, antimony arsenate, arsenic trifluoride, arsenic tribromide, arsenic triiodide, selenium dioxide, selenous acid, sodium hydrogen selenite, magnesium selenite, calcium selenite, barium selenite, aluminum selenite, copper selenite, silver selenite, cerium selenite, barium selenate, copper selenate, iron selenide, zinc selenide, cadmium selenide, lead selenide, selenium chloride, selenium tetrachloride, selenium bromide, selenium tetrabromide, barium chloride, thallium, thallous oxide, thallium oxide, thallium hydroxide, thallous chloride, thallous bromide, thallous iodide, thallium triiodide, thallium nitrate, thallous sulfate, thallium (thallous) carbonate, thallous phosphate, beryllium, beryllium oxide, beryllium hydroxide, beryllium chloride, beryllium carbonate, beryllium sulfate, potassium beryllium sulfate, beryllium chromate, ammonium fluoberyllate, sodium fluoberyllate, osmium tetroxide, ammonium chlorosmate, vanadium pentoxide, vanadium chloride, potassium vanadate, potassium metavanadate, sodium metavanadate, ammonium metavanadate, ammonium polyvanadate, sodium ammonium vanadate, mercury arsenide, mercury nitrate, mercury fluoride, mercury iodide, mercury oxide, sodium tellurite, sodium nitroprusside, zinc phosphide, bromine, hydrogen bromide, germane, boron trifluoride, boron trichloride, and toxic metal ions (such as copper ion, aluminum ion, mercury ion, barium ion, lead ion, chromium ion, cadmium ion, and silver ion).

Examples of the food-borne poisons may include, but are not limited to, puffer fish toxin, ginkgo toxin, dolphin toxin, paralytic shellfish toxin, diarrheal shellfish toxin, neurotic shellfish toxin, amnestic shellfish toxin, ciguatoxin, scombroid toxin, conotoxin, polyether toxin, ciguatoxin, saxitoxin, maitotoxin, botulinum toxin, cyanogenic glycosides, linamarin, amygdalin, hydrocyanic acid, benzaldehyde, gossypols, solanine, muscarine, phallotoxin, amanita toxins, nitrite, histamines, lead, cadmium, mercury, arsenic, fluorine, polycyclic aromatic hydrocarbons, polychlorinated biphenyls, methanol, bongkrek acid, toxoflavin, deoxynivalenol, fusarenon-X, T2 toxin, triticum gibberellic disease toxin, aflatoxin, ochratoxin, phorbol esters, aplysiatoxin, okadaic acid, teleocidin, 3-nitropropionic acid, gyromitrin, ethanol, clenbuterol hydrochloride (lean meat powder), caffeine, heroin, and theophylline.

Examples of the exogenous biological toxins may include, but are not limited to, snake venom, scorpion venom, and jellyfish venom.

In some embodiments of the present invention, the disease-inducing molecules are selected from the group consisting of free DNA, free RNA, inflammatory factors, antibodies (such as antibodies for self-antigens), antigens (such as allergenic antigens), protein fragments, pathogenic microorganisms (such as viruses and bacteria), etc.

### 2.2 Presence form of toxins

In some embodiments, the toxin is present in the biofluid in a free state, in a bound state with a substance in the biofluid, or both.

There are usually other biological components in biofluid, such as proteins (such as plasma proteins), saccharides, lipids, nucleic acids and other molecules, which may bind to toxins. For example, albumin is present in a large amount in the blood, and can bind various metabolites *in vivo,* such as indoxyl sulfate, resulting in the indoxyl sulfate difficult to remove. In some embodiments, the substances bound to the toxins include components in blood tissue (such as plasma protein, albumin, and red blood cells), components in adipose tissue (such as fat, unilocular adipose cells, and multilocular adipose cells), components in connective tissue (such as cellulite , collagen fibers, elastic fibers, and reticular fibers), components in bone tissue (such as calcium phosphate), etc.

In some embodiments, the toxin is at least in part reversibly bound with the substance in the biofluid. Reversible binding means that the toxins can spontaneously bind to and dissociate from the bound substances, and such binding and dissociation can reach a dynamic equilibrium. When the dynamic equilibrium is reached, the concentration or amount of toxins in free and bound states remains substantially unchanged. If the concentration of toxins in a free state is lower than the concentration in dynamic equilibrium, the bound toxins will be released through dissociation. Conversely, if the concentration of toxins in a free state is higher than the concentration in dynamic equilibrium, the binding of toxins in a free state are promoted.

In some embodiments, the reversible binding includes reversible binding formed by non-covalent bonds (such as ion-dipole interaction, dipole-dipole interaction, hydrogen bond, cation-π system interaction, π-π stacking interaction, hydrophobic interaction, and/or van der Waals force). The acting force of the non-covalent bonds usually has the strength as follows: ion-dipole interaction force (50-200 kJ/mol); dipole-dipole interaction (5-50 kJ/mol), which belongs to intermolecular interaction; hydrogen bond (4-120 kJ/mol); cation-π system interaction (5-80 kJ/mol); π-π stacking interaction (0-50 kJ/mol); hydrophobic interaction (0-50 kJ/mol); and Van der Waals force interaction (less than 5 kJ/mol). For the description and introduction of the above-mentioned non-covalent bond force, please refer to, for example, "Biopharmaceuticals and Pharmacokinetics" Fifth Edition, People's Medical Publishing House, editor-in-chief Liu Jianping, page 94; Supramolecular Chemistry: From Molecules to Nanomaterials, Online 2012 John Wiley & Sons, Ltd.

The binding strength of the toxins and the substances in the biofluid (such as protein) can be expressed by the Kd value. In some embodiments, the toxin is bound to the substance in the biofluid at a Kd value of at least 10² µmol/L, 10³ µmol/L, 10⁴ µmol/L, 10⁵ µmol/L, 10⁶ µmol/L, or 10⁷ µmol/L. The greater the Kd value, the stronger the binding. Generally speaking, protein is bound to the toxin at a high Kd value of 10⁵-10⁷ µmol/L, and at a low or medium binding strength Kd value of 10²-10⁴ µmol/L.

In some embodiments, the Kd value at which the toxin is bound to the substance (such as protein) in the biofluid is at least 10⁵-10⁷ µmol/L, 10^{5.5}-10⁷ µmol/L, or 10⁶-10⁷ µmol/L.

Those skilled in the art can understand that the stronger the binding of the toxin, the harder it is to remove. Certain toxins tend to be strongly bind to plasma proteins, for example, with a high Kd value, which makes it difficult to remove the toxins by the methods in the prior art.

In some embodiments, the toxins in biofluid is present mainly in a bound state. In such case, the free amount of toxins in biofluid is relatively low, and only part of the free toxins can be removed by the simple osmosis in the prior art. However, most of the bound toxins are still not effectively eliminated from the biofluid.

In some embodiments, at least 10%, at least 20%, at least 30%, at least 40%, at least 50%, at least 60%, at least 70%, at least 80% or at least 90% of the toxins in the biofluid are present in a bound state. In some embodiments, at least 10%, at least 20%, at least 30%, at least 40%, at least 50%, at least 60%, at least 70%, at least 80% or at least 90% of the toxins in the biofluid are present in a state bound to plasma proteins. The binding of toxins with substances (such as proteins or plasma proteins) in biofluid can be detected by various methods known in the art, for example, but not limited to, equilibrium dialysis, ultrafiltration, ultracentrifugation, gel filtration, spectroscopy (including ultraviolet-visible spectroscopy, fluorescence spectroscopy, infrared spectroscopy, circular dichroism spectroscopy, and Raman spectroscopy), optical biosensor method, etc.

In some embodiments, the toxins can reversibly bind to protein in biofluid. Such toxins include but are not limited to indoxyl sulfate, asymmetric arginine, homocysteine, phenylacetic acid, p-cresol, AGE products (3-deoxyglucosone, fructoselysine, glyoxal, pyruvaldehyde, pentosidine), hippuric acid, uremic toxins, hydrogen sulfide, bilirubin, etc.

In some embodiments, the toxins are present mainly in a bound state (for example, at least 50%, 60%, 70%, 80%, or more than 90% are present in a bound state), and are strongly bound to the substances (such as protein) in the biofluid (for example, at a Kd value of at least 10⁴ µmol/L, 10⁵ µmol/L or higher). For example, the binding rate of tenoxicam (an anti-inflammatory drug) to plasma protein is 99%, and the binding rate of digitoxin (a drug used to treat congestive heart failure) to plasma protein is 91%.

In some embodiments, the toxin-removal reagent in the composition provided herein is different from the substance bound to the toxin in the biofluid. For example, when the toxins in the biofluid to be treated is bound to plasma proteins (such as albumin), the toxin-removal reagent in the composition of the present application may not use plasma proteins (such as albumin). In some embodiments, the toxin-removal reagent in the composition provided herein is not a substance present in the biofluid (for example, it may be a synthetic polymer, an inorganic substance, etc.).

In some embodiments, the toxin-removal reagent in the composition provided herein is not immunogenic. Such compositions have advantages when used in direct contact with organisms (for example, when used in peritoneal dialysis).

In some embodiments, the toxin-removal reagent in the composition provided herein is an artificial material. Artificial materials refer to materials that do not present in biofluid, or materials that are artificially synthesized or prepared. For example, diatomaceous earth, silica, activated carbon and the like are all artificial materials.

In some embodiments, the toxin-removal reagent in the composition provided herein binds to the toxin via an interaction that is different from the binding interaction between the substance in the biofluid and the toxin. For example, the toxin-removal reagent is a porous material that binds to the toxins through porous adsorption, while the toxins in biofluid bind to albumin mainly through, for example, hydrogen bond. Without being bound by theory, it is believed that the use of toxin-removal reagents that provide different binding interactions can help break the original dynamic equilibrium of toxin binding in the biofluid, thereby promoting the dissociation and removal of the bound toxins from the biofluid.

In some embodiments, the toxin-removal reagent in the composition provided herein binds to the toxins via a stronger adsorption or binding interaction than the binding interaction between the toxins and the substances in the biofluid. For example, compared to the binding between toxins and substances (e.g., plasma proteins) in biofluid, the toxin-removal reagent binds to the toxins at a higher Kd value (for example, the toxins bind to the toxin-removal reagent at a Kd value of 10⁵ µmol/L, while the toxins bind to the substances in the biofluid at a Kd value of 10⁴ µmol/L), at a greater binding ratio (for example, the toxins bind to the toxin-removal reagent at a binding ratio of greater than 80%, while the toxins bind to the substance in the biofluid at a binding ratio of 50%), and/or in a more stable binding type (for example, the toxins bind to the toxin-removal reagent via a covalently bond, while the toxins bind to the substances in the biofluid via a non-covalently bond).

### 3. Osmotic agent

In some embodiments of the present invention, the composition of the present invention includes an osmotic agent. The osmotic agent provides an osmotic pressure substantially equal to or higher than that of biofluid. In some embodiments, the osmotic agent can provide an osmotic pressure of about 290-310 mmol/L, 260-340 mmol/L, or 260-350 mmol/L (for example, about 280 mOsm/L, 283 mOsm/L, 284 mOsm/L, 285 mOsm/L, 287 mOsm/L, 290 mOsm/L, 295 mOsm/L, 300 mOsm/L, 310 mOsm/L, 315 mOsm/L, 320 mOsm/L, 325 mOsm/L, 330 mOsm/L, 340 mOsm/L or 350 mOsm/L). The osmotic agent provides an osmotic pressure substantially equal to that of the biofluid, which can prevent electrolytes, saccharides, water and other substances in the biofluid from passing as much as possible, or does not have excessively adverse effects on the biological properties of the biofluid. The osmotic agent provides an osmotic pressure higher than that of the biofluid, which allows water in the biofluid to pass, and is beneficial to some patients with impaired kidney function to excrete excess water from the body.

When used to treat biofluid, suitable osmotic agents may be non-toxic, biologically inert, and/or metabolizable.

In some embodiments, the osmotic agent may include saccharides, amino acids, polypeptides, glycerol, carbonates, or analogs thereof, or any combination thereof. The osmotic agent can have a small molecular weight (for example, the molecular weight is not greater than 500 g/mol), or a large molecular weight (for example, the molecular weight is greater than 500 g/mol).

Various saccharides suitable as osmotic agents can be used as osmotic agents, which can be monosaccharides, oligosaccharides (for example, disaccharides, and trisaccharides) and polysaccharides. Examples of the monosaccharides include, but are not limited to, glucose, fructose, sorbitol, xylitol, aminosaccharides and derivatives thereof. The oligosaccharides or polysaccharides include oligomers or polymers of one or more monosaccharides, and the monosaccharides may be linked by, for example, α-1,4 glycosidic bonds, or α-1,6 glycosidic bonds. In some embodiments, the oligosaccharides or polysaccharides can be metabolized by, for example, the human or animal.

In some embodiments, suitable osmotic agent which is a saccharide may include, for example, glucose, non-glucose monosaccharide (such as sorbitol, fructose, and xylitol), or a monosaccharide polymer (such as glucose polymer, maltodextrin, icodextrin, and chitosan oligosaccharide (for example, see Chinese patent application CN107375318A)). In some embodiments, the glucose polymer include icodextrin.

The osmotic agent which is an amino acid may be selected from a natural amino acid, an unnatural amino acid, an analog thereof, a derivative thereof, and any combination thereof. Examples of osmotic agent which is an amino acid include, but are not limited to, leucine, valine, threonine, isoleucine, lysine, histidine, methionine, phenylalanine, tryptophan, alanine, proline, arginine, glycine, serine, tyrosine, aspartic acid, glutamic acid, and derivatives and any combination thereof. An exemplary osmotic agent which is an amino acid can be found in US Patent 5,629,025.

The osmotic agent which is a peptide can also be used as possible osmotic agent (for example, see, US Patent 4,906,616, US Patent 6,380,163, and US Patent 5,039,609).

### 4. Content and other components

In some embodiments, the amount ratio (weight/weight) of the toxin-removal reagent to the osmotic agent in the composition provided herein ranges from 1:1750 to 1:4 (for example, 1:1750 to 1:5, 1:1500 to 1:4, 1:1500 to 1:5, 1:1000 to 1:5, 1:750 to 1:6, 1:300 to 1:6, 1:75 to 1:6, 1:30 to 1:6, 1:10 to 1:6, 1:1500 to 1:5, 1:1500 to 1:10, 1:1500 to 1:30, 1:1500 to 1:75, 1:1500 to 1:150, 1:1500 to 1:300, and 1:1500 to 1:750).

In some embodiments, when the composition provided herein is formulated into a dialysis solution directly used for osmosis, the content (weight/volume) of the osmotic agent in the dialysis solution is in a range of 0.05%-10%, 0.5%-10%, 1%-10%, 1.5%-10%, 1.5%-9%, 1.5%-8%, 1.5%-7.5%, 1.5%-6%, 1.5%-5%, etc. For example, when the osmotic agent is icodextrin, the content (weight/volume) of the osmotic agent in the dialysis solution for osmosis may be in a range of 3%-8%, 5-7.5%, or about 7.5%. For example, when the osmotic agent is glucose, the content (weight/volume) of the osmotic agent in the dialysis solution for osmosis may be in a range of 0.05%-2%, 0.05%-1.5%, 0.05%-1.0%, 0.05%-0.75%, 0.5%-1.5%, or about 1.5%.

In some embodiments, when the composition provided herein is formulated into a dialysis solution directly used for osmosis, the content (weight/volume) of the toxin-removal reagent in the dialysis solution is in a range of at least 0.0001%, at least 0.0005%, at least 0.001%, at least 0.005%, at least 0.01%, at least 0.025%, at least 0.05%, at least 0.075%, at least 0.1%, at least 0.125%, at least 0.15%, at least 0.175%, at least 0.2%, or at least 0.25%. In some embodiments, the content (weight/volume) of the toxin-removal reagent in the dialysis solution is in a range of not higher than 4%, not higher than 3.5%, not higher than 3.3%, not higher than 3.0%, not higher than 2.8%, not higher than 2.5%, not higher than 2.3%, not higher than 2.0%, not higher than 1.8%, not higher than 1.6%, not higher than 1.4%, not higher than 1.2%, not higher than 1.0%, not higher than 0.8%, not higher than 0.6%, or not higher than 0.4%. In some embodiments, the content range (weight/volume) of the toxin-removal reagent in the dialysis solution is a numerical range obtained by any combination of the two end values selected from the above combinations respectively, for example, but not limited to, at least 0.0001% to not higher than 4%, at least 0.005% to not higher than 4%, at least 0.01% to not higher than 4% and so on, which are not listed one by one herein for brevity. In some embodiments, the toxin-removal reagent is activated carbon, povidone, diatomaceous earth, polyethylene caprolactam-polyvinyl acetate-polyethylene glycol graft copolymer (e.g., Soloplus), or micronized silica gel or any combination thereof, and when the composition is formulated into a dialysis solution directly used for osmosis, the content range (weight/volume) of the toxin-removal reagent in the dialysis solution meets the various ranges listed above.

In some embodiments of the present application, the composition of the present invention may also comprise one or more components suitable for use under a condition for osmosis, such as buffers, electrolytes, and other components suitable for osmosis.

In some embodiments, the composition provided herein may further contain a suitable buffer to provide the required pH value. Non-limiting examples of buffers include one or more of lactate, bicarbonate, citrate, isocitrate, dihydrogen phosphate, hydrogen phosphate, phosphate, pyruvate, succinate, fumarate, acetate, malate, oxaloacetate, chloride, amino acids (histidine, glycine, alanine) with pK₁ of 7-13, and analogs thereof.

In some embodiments, when the composition of the present application is formulated into a solution for osmosis with biofluid, the pH value of the solution may be close to that of the biofluid, or at least is at a physiologically acceptable level. For example, the pH of the solution measured at room temperature (20-25°C) may be 4.0-8.0, 5.0-8.0, 6.0-8.0, 4.0-7.8, 4.0-7.0, 5.0-7.5, or 6.5-7.5. The concentration of the buffer in the solution can be, for example, but not limited to, 1.0-200 mM, 2.5-150 mM, 5-100 mM, 5-75 mM, 10-50 mM, and 20-30 mM.

Electrolytes refer to substances that contain free ions and have conductivity. The electrolytes can be completely dissociated into cations and anions, and preferably do not significantly change the pH of the composition. Non-limiting examples of cations of the electrolytes include alkali metal cations (such as Na⁺ and K⁺), and alkali earth metal cations (such as Ca²⁺ and Mg²⁺). Non-limiting examples of the anions of the electrolytes include Cl⁻. In some embodiments, the electrolytes may be selected from one or more of sodium salt, calcium salt, magnesium salt and potassium salt.

In some embodiments, the composition is sterilized. Various methods known in the art can be used for sterilization, such as, but not limited to, autoclaved sterilization, steam sterilization, ultraviolet sterilization, filter sterilization, or any combination thereof.

In some embodiments, the composition is a solid preparation, a semi-solid preparation or a liquid preparation. The composition in a solid or semi-solid form can be conveniently formulated with a suitable solvent (e.g., sterile water) into a desired liquid preparation. The liquid preparation may also be a liquid concentrate with different degrees of concentration that can be mixed with a solvent in an appropriate manner to prepare a desired liquid preparation.

### Dialysis solution

In another aspect, a dialysis solution containing the composition of the present application is provided herein. The dialysis solution may be a small molecule solution, and may also include a solution or colloidal solution containing large molecules (e.g., polymers). In some embodiments, the dialysis solution can be used as a peritoneal dialysis solution or a hemodialysis solution. In some embodiments, the pH value and/or electrolyte concentration of the dialysis solution provided herein is physiologically acceptable.

In some embodiments, the dialysis solution is in the form of a concentrate or a ready-to-use form. In some embodiments, the dialysis solution may be a single solution (for example, contained in a single container), or may contains two or more components (for example, contained in two or more containers respectively). Without being bound by theory, dividing the dialysis solution into different components helps to avoid unnecessary interaction of the components in the composition (for example, a neutral or alkaline pH may cause glucose polymers to degrade during high-temperature sterilization).

In some embodiments, the dialysis solution is divided into two or more components, which are kept separate from each other until it is needed for use, for example, until it is needed to be formulated into a ready-to-use dialysis solution. The components can be in different forms, for example, they can all be in the form of a fluid, or at least one of the components can be in the form of a dry powder (e.g., a buffer component), or at least one of the components can be in the form of a fluid concentrate (e.g., an electrolyte concentrate). Optionally, the component in the form of dry powder is dissolved with a diluent before being mixed with other components, or the concentrate can be diluted with a diluent in a certain ratio (for example, but not limited to 1:35, 1:45 or 1:200). Any suitable diluent can be used, such as sterile water, and optionally, without an electrolyte.

In some embodiments, the first component of the dialysis solution contains glucose polymers, and has a pH value in a range of 3.5-5.5. One or more physiologically acceptable acids (such as lactic acid, pyruvic acid, acetic acid, citric acid, and hydrochloric acid) can be used to adjust the pH value. Alternatively, acids may be replaced with carbon dioxide for pH adjustment. In some embodiments, the second component of the dialysis solution may contain a buffer solution. The buffer solution can provide a pH in a range of about 7 to about 9. A suitable buffer solution may contain, for example, sodium bicarbonate, sodium chloride, sodium lactate, one or more amino acids with a pK₁ value of 7-13 (for example, histidine, glycine, and alanine) or combination thereof.

The toxin-removal reagent provided herein may be present in the first component, or in the second component, or in an additional third component.

In some embodiments, the two or more components of the dialysis solution can be contained in containers in any suitable manner. For example, different components of the dialysis solution are respectively contained in a plurality of containers that can be connected via fluid. When the components needs to be formulated into a dialysis solution for direct use, the fluids in containers are operably connected (for example, by opening a valve for fluid connection, or breaking the seal between a multi-compartment bag) to obtain a ready-to-use dialysis solution.

In some embodiments, the dialysis solution may further contain anticoagulant regents, such as heparin and citrate.

### Kit

In another aspect, the present application further provides kits for purification of a biofluid, comprising the composition of the present application. In some embodiments, the composition is sterilized.

In some embodiments, the composition in the kit may be present in the form of a single composition, or may be in the form of two or more components. In some embodiments, the composition or the components thereof are sterilized. In some embodiments, the composition or at least one of the components is a solid preparation, a semi-solid preparation or a liquid preparation. The components can be in different forms, for example, they can all be in the form of a fluid, or at least one of the components can be in the form of a dry powder (e.g., a buffer component), or can be in the form of a fluid concentrate (e.g., an electrolyte concentrate). Optionally, the component in the form of dry powder is dissolved with a diluent before being mixed with other components, or the concentrate can be diluted with a diluent in a certain ratio (for example, but not limited to 1:35, 1:45 or 1:200).

In some embodiments, the two or more components are contained in different containers. In some embodiments, the two or more components are kept separate from each other. In some embodiments, the two or more components are respectively contained in two or more containers that can be operably connected via fluid. When needed for use, the components can be mixed to prepare the product needed, such as a ready-to-use dialysis solution. Any suitable diluent can be used, such as sterile water, and optionally, the diluent contains no electrolytes.

In some embodiments, the kit further contains a semi-permeable substrate that can be used for purification of a biofluid.

### Dialysis device

In another aspect, the present application provides a dialysis device, comprising the composition provided herein. The device is configured to allow osmosis between the composition and the biofluid to be dialyzed. In some embodiments, the dialysis device can be suitable for hemodialysis or peritoneal dialysis.

In some embodiments, the dialysis device provided herein comprises a first passage for receiving the biofluid to be dialyzed, a second passage for containing the composition provided herein, and a semi-permeable substrate separating the first passage from the second passage. The semi-permeable substrate can allow osmosis between toxins in the biofluid to be dialyzed and the composition.

In some embodiments, the dialysis device is disposable. For example, the dialysis device can be installed on a dialysis host, and disassembled and discarded after the dialysis process is completed. The dialysis host can include various devices and components necessary for dialysis, such as one or more of a dialysis pump, an ultrafiltration pump, a proportional dosing device, a liquid constant temperature device, a transmembrane pressure monitor, a conductivity monitor, a blood pump, a heparin pump, an arteriovenous pressure monitor, an air bubble monitor, a blood leakage monitor, a replacement fluid balance scale, and a two-pipe pump. When the dialysis device is installed on the dialysis host, the dialysis device can be configured to be operably connected to the dialysis host, so that the biofluid to be dialyzed enters the first passage for receiving biofluid in the dialysis device through the dialysis host, and passes through the semi-permeable substrate in the dialysis device, and allows osmosis with the composition of the present application in the dialysis device. In some embodiments, after osmosis is completed, the biofluid can be returned to the organism through the dialysis host. In some embodiments, the dialysis host may further be provided with dedicated software, which can control the dialysis process (for example, temperature, pressure, and addition of anticoagulant reagents).

In some embodiments, the dialysis device can be used for artificial kidneys (for example, see US Patent 8,834,400, US Patent 8,277,407, US Patent 8,012,118, US Patent 5,545,131, and US Patent 4,623,450 for details), artificial livers (for example, see US Patent 9,650,609, and U.S. Patent 9,775,863 for details), or artificial lungs (for example, see U.S. Patent 9,717,835, U.S. Patent 9,827,534, U.S. Patent 9,814,821, U.S. Patent 9,795,730, and U.S. Patent 9,717,839 for details), and the like to achieve the removal of toxins from the biofluid.

An artificial kidney is a device that replaces kidney function and is used to help patients with uremia, etc. The artificial kidney is mainly used to treat renal failure and uremia. The artificial kidney takes the blood out of the body, eliminates excess nitrogenous compounds, metabolites or excess drugs *in vivo* by the principles of dialysis, filtration, adsorption and membrane separation, adjusts the electrolyte balance, and then returns the purified blood to the body.

An artificial liver refers to a device that uses an external mechanical, physicochemical or biological reactor device to remove all kinds of harmful substances produced or increased due to liver failure, to supplement essential substances such as proteins that need to be synthesized or metabolized by the liver, to improve the patient's internal environment such as water, electrolyte and acid-base balance, temporarily assist or replace the corresponding main functions of the liver until autologous liver cell regeneration or liver function recovery, or to alleviate the symptoms of patients with advanced liver disease, become a "bridge" for liver transplantation, so as to improve the survival rate of patients.

An artificial lung, also known as oxygenator or gas exchanger, is an artificial organ that replaces human lungs to discharge carbon dioxide, uptake oxygen, and exchange gas.

### A purification method of biofluid

In another aspect, the present application further provides methods for reducing a toxin in a biofluid. The method of the present application may comprise: a) contacting the biofluid with an osmotic solution comprising the composition of the present application under a condition to allow osmosis, and b) allowing the composition to reduce the amount of the toxin in the biofluid.

In some embodiments, the methods provided by the present application can reduce the free amount of the toxin in biofluid to at most 50%, at most 40%, at most 30%, at most 20%, at most 10%, or at most 5% of its initial free amount. In some embodiments, the method provided by the present application can reduce the total amount of the toxin (or the non-free amount) in a biofluid by at least 10%, at least 20%, at least 30%, at least 40%, at least 50%, at least 60%, at least 70%, at least 80%, at least 90%, or at least 95%. Compared with the biofluid before treatment with the composition provided by the present application, the total amount of toxins (or non-free amount) in the biofluid after treatment is at most 90%, at most 80%, at most 70%, at most 60%, at most 50%, at most 40%, at most 30%, at most 20%, at most 10%, or at most 5% of that before treatment.

In some embodiments, the method provided by the present application can reduce the total amount of toxins (or free amount or non-free amount) in biofluid to a physiologically beneficial level. "Physiologically beneficial level " refers to the level that can reduce the risk of disease or alleviate symptoms caused by toxins in the organism.

In some embodiments, the toxin is present in the biofluid in a free state, in a bound state with a substance in the biofluid, or both. In some embodiments, the toxin is at least in part reversibly bound with the substance in the biofluid. In some embodiments, the toxin is bound to the substance (e.g., protein) in the biofluid at a Kd value of at least 10⁵-10⁷ µmol/L, 10^{5.5}-10⁷ µmol/L, or 10⁶-10⁷ µmol/L. In some embodiments, the toxin in the biofluid is present mainly in a bound state. In some embodiments, at least 10%, at least 20%, at least 30%, at least 40%, at least 50%, at least 60%, at least 70%, at least 80% or at least 90% of the toxin in the biofluid is present in a bound state. In some embodiments, at least 10%, at least 20%, at least 30%, at least 40%, at least 50%, at least 60%, at least 70%, at least 80% or at least 90% of the toxin in the biofluid is present in a bound state with plasma proteins. In some embodiments, the toxin can reversibly bind to proteins in the biofluid. Such toxin includes but is not limited to indoxyl sulfate, asymmetric arginine, homocysteine, phenylacetic acid, p-cresol, AGE products (3-deoxyglucosone, fructoselysine, glyoxal, pyruvaldehyde, pentosidine), hippuric acid, uremic toxins, hydrogen sulfide, bilirubin, etc.

In some embodiments, the toxin in the biofluid is transferred to the osmotic solution through osmosis under the condition to allow osmosis. The condition to allow osmosis in the present application means that there is a toxin concentration difference between the biofluid to be purified and the osmotic solution containing the composition of the present application. That is, the concentration of the toxins in the biofluid is higher than the concentration in the osmotic solution. In the method provided by the present application, the osmotic solution containing the composition of the present application can be used to purify biofluid. Through the osmosis of substances between the osmotic solution and the biofluid, the toxins (and/or metabolic waste, and optionally, excess liquid) in the biofluid are discharged to the osmotic solution. In some embodiments, the substances required in the biofluid may also be transferred from the osmotic solution to the biofluid. A wellknown method for purifying biofluid is dialysis, in which toxins diffuse from the biofluid and pass through a semi-permeable substrate into the osmotic solution through the concentration gradient of the toxin between the biofluid and the osmotic solution. In some embodiments, the osmotic solution is at least isotonic to the biofluid. In some embodiments, the osmotic pressure of the osmotic solution is higher than that of the biofluid, so as to allow the water in the biofluid to be discharged from the biofluid through osmotic diffusion.

In some embodiments, the step a) comprises placing the biofluid and the composition respectively at two sides of a semi-permeable substrate.

In some embodiments, the semi-permeable substrate is an artificial semi-permeable membrane. The artificial semi-permeable membrane may be made of any suitable material, such as, but not limited to, ceramics, graphite, metals, metal oxides, and polymers. In some embodiments, the artificial semi-permeable membrane may be made of polymers selected from the group consisting of polysulfone, polyamide, polycarbonate, polyester, acrylonitrile polymer, vinyl alcohol polymer, acrylate polymer, methacrylate polymer, cellulose acetate polymer, etc.

In some embodiments, the semi-permeable substrate is a biological semi-permeable membrane. The biological semi-permeable membrane may be blood vessel wall membrane, lymphatic vessel wall membrane, peritoneum, lung membrane, glandular envelope and mucosae (such as oral mucosa, nasal mucosa, gastric mucosa, intestinal mucosa, and vaginal mucosa).

In some embodiments, the biofluid is inside the body of a subject. By removing the toxins from the biofluid in the subject, the amount of toxins in the subject can be reduced, thereby improving the health of the subject. The biofluid in a subject may be blood in the blood circulation system in the body (for example, in the case of peritoneal dialysis), lymph in the lymphatic system, and the like, or the biofluid that is in connection with the circulation system in the body and is transferred to the outside of the body and then returned into the body. The biofluid inside the body of a subject may include, for example, blood and lymph in the organism.

In some embodiments, the biofluid inside the body of the subject can directly undergo osmosis with the osmotic solution *in vivo* (for example, peritoneal dialysis). Alternatively, in some other embodiments, the biofluid inside the body of the subject can be drawn out of the body, and after osmosis with the osmotic solution and toxin removal *in vitro,* the biofluid is transfused into the body (for example, hemodialysis).

For example, in hemodialysis, blood can be drawn from an organism and purified in an extracorporeal blood circuit, and then the purified blood can be transfused into the organism. The blood flows on one side of a semi-permeable membrane, and the osmotic solution flows on the other side of the semi-permeable membrane. Due to the difference in the transmembrane concentration of toxins between the blood and the osmotic solution, the toxins in the blood (and optionally, various harmful and excess metabolic waste and excessive electrolytes) are transferred to the osmotic solution by diffusion through the semi-permeable membrane. At the same time, excess fluid in the blood generates a transmembrane pressure difference on the semi-permeable membrane, thereby passing through the semi-permeable membrane and being transferred to the osmotic solution through ultrafiltration.

For example, in peritoneal dialysis, the peritoneum of an organism (such as a human or an animal) can be used as a semi-permeable membrane, thereby purifying the blood in the organism. In peritoneal dialysis, the aseptic osmotic solution in the method of the present application can be introduced into the abdominal cavity through a catheter, and allow solute exchange between the osmotic solution and the blood within a sufficient time to remove toxins from the blood in the organism. By providing a suitable osmotic gradient from the osmotic solution to the blood, the flow of liquid is achieved to allow water to flow out of the blood. This enables proper acid-base, electrolyte and fluid balance in the blood. After a period of time, the osmotic solution is discharged from the body cavity through the catheter. Examples of different types of peritoneal dialysis include continuous ambulatory peritoneal dialysis, automatic peritoneal dialysis, and continuous flow peritoneal dialysis.

In some embodiments, the step a) comprises applying the osmotic solution to the subject by intraperitoneal infusion. In such embodiments, the peritoneum in the subject is used as a semi-permeable substrate. In some embodiments, the step a) comprises applying the osmotic solution to the subject by hemodialysis.

In some embodiments, the biofluid is outside the body. In some embodiments, the biofluid outside the body includes a fluid that does not form a fluid circuit with the biofluid inside the body. For example, the biofluid outside the body may be a blood sample taken from an organism, or a treated blood product or biological product. By removing the toxins from the biofluid outside the body of the subject (such as blood products and biological products), the biofluid can be purified to achieve the purpose of subsequent application. Examples of the biofluid outside the body include, but are not limited to, whole blood, warm blood or cold blood, umbilical cord blood and stored or fresh blood, treated blood, for example, blood diluted with at least one physiological solution (including but not limited to saline, nutrients, additives and/or anticoagulant solutions), blood components (such as platelet concentrate (PC), platelet-rich plasma (PRP), platelet-poor plasma (PPP), platelet-free plasma, plasma, fresh frozen plasma (FFP), components obtained from plasma, packed red cells (PRC), transition zone material or buffy coat (BC)), blood products derived from blood or blood components or bone marrow, stem cells, cell cultures, physiological solutions containing bone marrow aspirates, etc.

### Methods for treating diseases

In another aspect, the present application also relates to methods of treating or preventing a toxin-related disease or condition, comprising contacting the composition of the present application with the biofluid of a subject under a condition to allow osmosis, such that the toxin in the biofluid is reduced. In another aspect, the present application further provides use of the composition of the present application in the manufacture of a medicament for treating or preventing a toxin-related disease or condition. In some embodiments, the composition can reduce the toxins in the biofluid inside the body of the subj ect.

Those skilled in the art can understand that when certain toxins are present in the biofluid (for example, blood) in a subject, the presence or excessive presence of the toxins will adversely affect the physiological function of the subject, for example, leading to metabolic dysfunction, and/or affecting various organs and organ systems, such as the cardiovascular system (hypertension, pericarditis and heart failure), peripheral nervous system (polyneuropathy), bone and joint system, central nervous system (poor memory, loss of concentration and mental ability retardation), blood system (anemia, hemorrhagic tendency, coagulation, immune status (immunosuppression)), etc.

In some embodiments, the toxin-related diseases or conditions include kidney disease (such as uremia and renal insufficiency), cardio-cerebrovascular diseases, blood diseases (such as anemia, hemorrhagic tendency and coagulation), autoimmune diseases (autoimmunity, immunosuppression, etc.), metabolic diseases (hyperlipidemia, diabetes, etc.), orthopedic diseases, digestive system diseases (such as liver and gallbladder diseases), drug overdose or poisoning.

The toxin related to the above diseases includes, but is not limited to, urea, creatinine, uric acid, guanidine-ADMA, β₂-microglobulin, cytokines, parathyroid hormone, indoxyl sulfate, homocysteine, p-cresol, hippuric acid, reactive oxygen species (ROS), uremic toxins (such as AGE products (3-deoxyglucosone, fructoselysine, glyoxal, pyruvaldehyde, pentosidine), 1-methyladenosine, 1-methylguanosine, 1-methylinosine, asymmetric dimethylarginine, α-keto-δ-guanidinovaleric acid, α-N-acetylarginine, arabitol, arginine, benzyl alcohol, β-guanidinopropionic acid, β-lipotrophic hormone, creatine, cytidine, sodium N,N-dimethylglycinate, erythritol, γ-guanidinobutyric acid, hypoxanthine, malondialdehyde, mannitol, methylguanidine, inositol, N,N-dimethylguanosine, N-acetyl cytosine nucleoside, N-threonyl carbamoyl adenosine phosphate, orotic acid, orotidine, oxalate, phenylacetylglutamine, pseudouridine, symmetric dimethylarginine, sorbitol, taurocyamine, threitol, thymine, uracil, uridine, xanthosine, 2-methoxyresorcinol, 3-deoxyglucosone, 3-carboxy-4-methyl-5-propyl-2-furanpropionic acid (CMPF), fructosyllysine, homocysteine, hydroquinone, indole-3-acetic acid, kynurenine, kynurenic acid, leptin, melatonin, methylglyoxal, Nε-carboxymethyllysine, cresol, pentoside, phenol, p-hydroxyhippuric acid, butanediamine, quinolinic acid, retinol conjugated protein, spermidine, spermine, adrenomedullin, atrial natriuretic peptide, β-endorphin, cholecystokinin, Clara cell protein (CC16), human complement factor D, cystatin C, degranulation inhibitor protein Ic, δ-sleep-inducing peptide, endothelin, hyaluronic acid, interleukin-1β, interleukin-6, κ-immunoglobulin light chain, λ-immunoglobulin light chain, methionine-enkephalin, neuropeptide Y, parathyroid hormone, tumor necrosis factor-α), hydrogen sulfide, bilirubin, exogenous poisonous drug (a sedative and hypnotic drug, an antipsychotic drug, a cardiovascular and cerebrovascular drug, an antipyretic and analgesic drug, an antiparasitic drug, an antimicrobial drug, an anesthetics and anesthetic assistant, a respiratory system drug, a circulatory system drug, a digestive system drug, a urinary system drug, a blood system drug, a metabolism and endocrine drug, an antiallergic drug, a tumor treatment drug, an immunomodulatory drug, an obstetrics and gynecology drug, a male drug, an anti-inflammatory drug, a traditional Chinese medicine), a pesticide (such as paraquat, dichlorvos, dimethoate, glyphosate, methamidophos, chlorpyrifos, omethoate, bromadiolone, brodifacoum, phoxim, imidacloprid, abamectin, silatrane, dimehypo, parathion, phorate, demeton, trichlorfon, malathion, benzene hexachloride, clofenotane, dieldrin, endrin, aldrin, fenvalerate, fluoroacetamide, tetramine, pentachlorophenol, dinitrocresol, diuron, dichlobenil, nereistoxin, rotenone, nicotine, methanonaphthalene chloride, formamidine, bacillus thuringiensis preparation, diphacinone sodium salt, warfarin, pyrinuron, amobam, ziram, methylmercury, and 2,4D butyl ester), a chemical poison (for example, a toxin derived from poisonous plants (such as Abrus precatorius and oleander), xylene, and automobile anti-freeze fluid), a food-borne poison (such as puffer fish toxin, ginkgo toxin, dolphin toxin, paralytic shellfish toxin, diarrheal shellfish toxin, neurotic shellfish toxin, amnestic shellfish toxin, tetrodotoxin, ciguatoxin, scombroid toxin, botulinum toxin, cyanogenic glycosides, linamarin, amygdalin, hydrocyanic acid, benzaldehyde, gossypol, solanine, muscarine, phallotoxin, amanita toxins, nitrite, histamines, lead, cadmium, mercury, arsenic, fluorine, polycyclic aromatic hydrocarbons, polychlorinated biphenyls, methanol, bongkrek acid, toxoflavin, deoxynivalenol, fusarenon-X, T2 toxin, triticum gibberellic disease toxin, 3-nitropropionic acid, gyromitrin, ethanol, clenbuterol hydrochloride (lean meat powder), caffeine, and theophylline), an exogenous biological toxin (such as snake venom, scorpion venom, and jellyfish venom), or a disease-inducing molecule (for example, free DNA, an inflammatory factor, an antibody, an antigen, a protein fragment, and a pathogenic microorganism).

By using the composition of the present application to reduce the level of one or more of the toxins in the biofluid of a subject, one or more of the aforementioned diseases can be treated or prevented. In the present application, "treatment" includes any improvement of the disease or health status, such as curing the disease, alleviating symptoms, reducing the severity of the disease, delaying the progression of the disease, and improving the life quality of the patient. "Prevention" includes reducing the risk of onset of disease, delaying the onset of disease, etc.

In some embodiments, the kidney disease can be treated or prevented by reducing one or more of the above-mentioned toxins in the biofluid. Examples of kidney diseases that can be treated or prevented may be selected from the group consisting of uremia, chronic nephropathy, acute renal insufficiency, chronic pyelonephritis, acute pyelonephritis, chronic glomerulonephritis, acute progressive nephritis syndrome, nephrotic syndrome, nephrosclerosis, interstitial nephritis, diabetic nephropathy, focal glomerulosclerosis, membranous nephropathy, multiple pustular renal syndrome, renovascular hypertension and hypertension syndrome, secondary nephropathy, hyperphosphatemia, hyperkalemia, hyperuricemia or hypernatremia.

### Examples

The present invention can be better understood with reference to the following Examples. However, the following Examples are intended to illustrate the present invention and should not be construed as limiting the scope of the present invention. Considering that various modifications and changes may be made to the teachings herein, these modifications and changes are within the scope of the present invention.

Various reagents, apparatuses and measuring methods used in the Examples are as follows.

### Reagents

### 1. Basic peritoneal dialysis solution

Icodextrin peritoneal dialysis solution: Baxter Extraneal peritoneal dialysis solution, containing 7.5% of icodextrin, and having the labeled osmotic pressure of 284 osmmol/Kg.

Glucose peritoneal dialysis solution: Baxter low-calcium peritoneal dialysis solution, lactate-G1.5%, containing 1.5% of glucose, and having the labeled osmotic pressure of 343 osmmol/Kg.

### 2. Toxin-removal reagent

Nano-carbon powder: Carbon nanoparticles suspension injection produced by Chongqing Lummy Pharmaceutical Co., Ltd. under the trade name Kanalin

Kollidon CL-SF: Crospovidone CL-SF (Kollidon® CL-SF) produced by BASF

Kollidon CL-M: Crospovidone CL-M (Kollidon® CL-M) produced by BASF

Soluplus: Soluplus® with the product number 30446233 produced by BASF

Micronized silica gel: Drug delivery silica with the product number 120091 produced by Merck & Co., Ltd.

Diatomaceous earth: Diatomaceous earth for biological use of Heilongjiang Fengtao Mining Investment Co., Ltd. (with a silica content of 92.8%, a specific surface area of 78-84 m²/g, a cumulative pore volume of 0.25 cm³/g, and an average pore diameter of 12 nm)

### Test

Cryoscopic method: The osmotic pressure molar concentration of a solution is determined by measuring the freezing point depression of the solution.

High performance liquid chromatography (HPLC): An Agilent C18 reverse chromatographic column is used, the column temperature is 25°C, the mobile phase is 25% by volume acetonitrile and 75% by volume aqueous solution containing 0.1% trifluoroacetic acid, the flow rate is 1 ml/min, the injection volume is 10 µL, a fluorescence detector with excitation wavelength of 295 nm and emission wavelength of 360 nm is used to detect indoxyl sulfate, the analysis time is 10 min, and the peak time is 3.8-4.0 min.

Biochemical analyzer detection: A biochemical analyzer is used to measure the content of creatinine and urea nitrogen in body fluids (blood or other body fluids).

### Example 1 Preparation of peritoneal dialysis solution

A certain volume of commercially available basic peritoneal dialysis solution was measured under aseptic conditions, and placed in a high-pressure steam sterilization container. A certain amount of sterilized toxin-removal reagent was added. The mixed solution was shaken to mix evenly and sealed. After ultrasonic treatment at 25°C in a water bath (50 Hz, 40 KW, 30 min), the mixed solution was placed in a 37°C water bath shaker (100 rpm, 30 min) for treatment, and was then sterilized using suitable methods such as a high temperature steam method. The mixed solution needs to be shaken evenly before use.

### Example 2 Effect of the amount of toxin-removal reagent on the osmotic pressure of different peritoneal dialysis solutions

### Example 2-1: Icodextrin peritoneal dialysis solutions with different amounts of toxin-removal reagents added (wt% is the ratio of weight to volume)

The osmotic pressure of peritoneal dialysis solutions obtained by adding different amounts of toxin-removal reagents was measured by the cryoscopic method.

**Table 1. Osmotic pressure of commercially available icodextrin peritoneal dialysis solutions containing different addition amounts of toxin-removal reagents**

| **Toxin-removal reagent** | **Addition amount (wt%)** | **Measured osmotic pressure (osmmol/Kg)** | | | **Average of measured osmotic pressure (osmmol/Kg)** |
|---|---|---|---|---|---|
| Null | 0 | 289 | 291 | 292 | 290.7 |
| Nano-carbon powder | 0.005 | 292 | 296 | 287 | 291.7 |
| | 0.01 | 297 | 299 | 296 | 297.3 |
| | 0.025 | 292 | 296 | 288 | 292.0 |
| | 0.25 | 291 | 290 | 288 | 289.7 |
| Kollidon CL-SF | 0.025 | 294 | 294 | 296 | 294.7 |
| | 0.25 | 310 | 313 | 311 | 311.3 |
| Kollidon CL-M | 0.025 | 300 | 295 | 300 | 298.3 |
| | 0.25 | 299 | 298 | 298 | 298.3 |
| Soluplus | 0.005 | 295 | 294 | 293 | 294.0 |
| | 0.01 | 303 | 305 | 281 | 296.3 |
| | 0.05 | 297 | 292 | 291 | 293.3 |
| | 0.1 | 300 | 301 | 301 | 300.7 |
| | 0.75 | 308 | 308 | 305 | 307.0 |
| | 1.5 | 313 | 303 | 303 | 306.3 |
| Micronized silica gel | 0.01 | 297 | 29 | 297 | 207.7 |
| | 0.05 | 298 | 298 | 296 | 297.3 |
| | 0.1 | 306 | 300 | 298 | 301.3 |
| Diatomaceous earth | 0.01 | 295 | 297 | 293 | 295.0 |
| | 0.05 | 292 | 290 | 299 | 293.7 |
| | 0.1 | 293 | 288 | 294 | 291.7 |

It can be seen from Table 1 that for Kollidon CL-SF, Soluplus and micronized silica gel groups, the osmotic pressure increases slightly with the increase of the addition amount. But for all groups, the osmotic pressure of the peritoneal dialysis solutions obtained by adding different amounts of toxin-removal reagents as above meets the requirements for peritoneal dialysis solutions. To reduce dehydration that may be caused by peritoneal dialysis, the amount of the toxin-removal reagent can be controlled to below 0.25%.

### Example 2-2: Glucose peritoneal dialysis solutions with different amounts of toxin-removal reagents added

The osmotic pressure of peritoneal dialysis solutions obtained by adding different amounts of toxin-removal reagents was measured by the cryoscopic method.

**Table 2. Osmotic pressure of commercially available glucose peritoneal dialysis solutions containing different addition amounts of toxin-removal reagents**

| **Toxin-removal reagent** | **Addition amount (wt%)** | **Measured osmotic pressure (osmmol/Kg)** | | | **Average of measured osmotic pressure (osmmol/Kg)** |
|---|---|---|---|---|---|
| Null | 0 | 337 | 334 | 332 | 334.3 |
| Nano-carbon powder | 0.01 | 335 | 339 | 334 | 336.0 |
| | 0.05 | 341 | 346 | 339 | 342.0 |
| | 0.25 | 344 | 349 | 343 | 345.3 |
| Kollidon CL-SF | 0.01 | 332 | 336 | 330 | 332.7 |
| | 0.05 | 341 | 337 | 335 | 337.7 |
| | 0.25 | 347 | 338 | 342 | 342.3 |
| Kollidon CL-M | 0.01 | 348 | 349 | 342 | 346.3 |
| | 0.05 | 357 | 345 | 359 | 353.7 |
| | 0.25 | 358 | 352 | 355 | 355.0 |
| Soluplus | 0.01 | 359 | 355 | 358 | 357.3 |
| | 0.05 | 367 | 359 | 364 | 363.3 |
| | 0.25 | 367 | 363 | 361 | 363.7 |
| Micronized silica gel | 0.01 | 330 | 337 | 339 | 335.3 |
| | 0.05 | 341 | 337 | 339 | 339.0 |
| | 0.25 | 335 | 341 | 338 | 338.0 |
| Diatomaceous earth | 0.01 | 349 | 351 | 347 | 349.0 |
| | 0.05 | 338 | 339 | 337 | 338.0 |
| | 0.25 | 340 | 337 | 335 | 337.3 |

It can be seen from Table 2 that for nano-carbon powder, Kollidon CL-SF and Kollidon CL-M groups, the osmotic pressure increases slightly with the increase of the addition amount. But for all groups, the osmotic pressure of the peritoneal dialysis solutions obtained by adding different amounts of toxin-removal reagents as above meets the requirements for peritoneal dialysis solutions. To reduce dehydration that may be caused by peritoneal dialysis, the amount of the toxin-removal reagent can be controlled to below 0.25%.

It can be seen from the above that the peritoneal dialysis solutions with different amounts of toxin-removal reagents still meet the requirements for peritoneal dialysis solutions and can be used for peritoneal dialysis.

### Example 3 Toxin-reducing effects of different peritoneal dialysis solutions containing toxin-removal reagents

### Example 3-1: Construction of rat model of renal failure by cisplatin method

200±5g male SD rats were selected, and a cisplatin physiological saline solution was intraperitoneally administered as 8 mg/kg body weight. Without fasting, dialysis experiments were performed on the 4th day after administration.

### Example 3-2: Peritoneal dialysis method

The rats were divided into groups. The positive control group was the rats treated with the basic peritoneal dialysis solution supplemented with 2.5 mg/L dopamine (DA), the negative control group was the rats not subjected to peritoneal dialysis treatment, the blank control group was the rats treated with the basic peritoneal dialysis solution only, and the experimental group was the rats treated with the basic peritoneal dialysis solution supplemented with different amounts of toxin-removal reagents. During peritoneal dialysis, the peritoneal dialysis solution was infused into the rats' abdominal cavity at a dose of 80 ml/kg body weight, and tail venous blood and peritoneal fluid were sampled for analysis at the time points of 1h, 2h, 4h, and 8h.

The tail venous blood sampling method was to take 0.5 ml of blood from the tail vein each time into a 1.5 ml volume EP tube containing 20 µl of 1% heparin physiological saline solution. The sampled blood was centrifuged at 3000 rpm for 10 min. 100 µl of supernatant was taken and 300 µl of methanol was added to the supernatant. The mixture was subj ected to vortex mixing for 10 s, and then centrifuged at 10000 rpm for 10 min. 100 µl of supernatant was taken and 300 µl of methanol was added to the supernatant. The mixture was subjected to vortex mixing for 10 s. 200 µl of the mixture was taken into an injection vial of high performance liquid chromatography for measuring the toxin content.

### Example 3-3: Icodextrin peritoneal dialysis solutions with different amounts of toxin-removal reagents added

The following table lists the experimental grouping of rats subjected to peritoneal dialysis treatment using icodextrin peritoneal dialysis solutions containing different amounts of toxin-removal reagents.

**Table 3. Experimental groups of rats treated with icodextrin peritoneal dialysis solutions containing different amounts of toxin-removal reagents**

| **Experimental group** | **Toxin-removal reagent** | **Amount of toxin-removal reagent (wt%)** |
|---|---|---|
| 1-1 | Nano-carbon powder | 0.005 |
| 1-2 | | 0.01 |
| 1-3 | | 0.025 |
| 1-4 | | 0.25 |
| 2-1 | Kollidon CL-SF | 0.025 |
| 2-2 | | 0.25 |
| 3-1 | Kollidon CL-M | 0.025 |
| 3-2 | | 0.25 |
| 4-1 | Soluplus | 0.01 |
| 4-2 | | 0.05 |
| 4-3 | | 0.25 |
| 5-1 | Micronized silica gel | 0.01 |
| 5-2 | | 0.05 |
| 5-3 | | 0.25 |
| 6-1 | Diatomaceous earth | 0.01 |
| 6-2 | | 0.05 |
| 6-3 | | 0.25 |

According to the method described in Example 3-2, the rats in the experimental group, positive control group, blank control group and negative control group were subjected to peritoneal dialysis, and the content of toxins (urea nitrogen (BUN), creatinine (CREA2) and indoxyl sulfate (IS)) in tail venous blood and peritoneal fluid is tested.

### Result:

### (1) Removal effect of urea nitrogen from blood

Figs. 1 (a)-(f) show the removal results of urea nitrogen from the blood of rats in the experimental groups as listed in Table 3 and in the corresponding positive control group (ICO+DA), blank control group (ICO) and negative control group.

Fig. 1(a) shows the removal results of urea nitrogen from blood using icodextrin peritoneal dialysis solutions with different amounts of nano-carbon powder added. It can be seen that compared to the blank control group treated with the icodextrin peritoneal dialysis solution only, the experimental groups 1-3 and 1-4 treated with icodextrin peritoneal dialysis solutions with 0.025 wt% and 0.25 wt% of nano-carbon powder added show a significant decrease in the content of urea nitrogen. While the experimental groups 1-1 and 1-2 treated with the icodextrin peritoneal dialysis solutions with 0.005 wt% and 0.01 wt% of nano-carbon powder added show no significant decrease in the content of urea nitrogen.

Fig. 1(b) shows the removal results of urea nitrogen from blood using icodextrin peritoneal dialysis solutions with different amounts of Kollidon CL-SF added. It can be seen that compared to the blank control group treated with the icodextrin peritoneal dialysis solution only, the experimental groups 2-1 and 2-2 treated with icodextrin peritoneal dialysis solutions with 0.025 wt% and 0.25 wt% of Kollidon CL-SF added show a significant decrease in the content of urea nitrogen, and the higher the content of Kollidon CL-SF, the more the content of urea nitrogen decreases.

Fig. 1(c) shows the removal results of urea nitrogen from blood using icodextrin peritoneal dialysis solutions with different amounts of Kollidon CL-M added. It can be seen that compared to the blank control group treated with the icodextrin peritoneal dialysis solution only, the experimental groups 3-1 and 3-2 treated with icodextrin peritoneal dialysis solutions with 0.025 wt% and 0.25 wt% of Kollidon CL-M added show a significant decrease in the content of urea nitrogen, and the higher the content of Kollidon CL-M, the more the content of urea nitrogen decreases.

Fig. 1(d) shows the removal results of urea nitrogen from blood using icodextrin peritoneal dialysis solutions with different amounts of Soluplus added. It can be seen that compared to the blank control group treated with the icodextrin peritoneal dialysis solution only, the experimental groups 4-1, 4-2 and 4-3 treated with icodextrin peritoneal dialysis solutions with 0.01 wt%, 0.05 wt% and 0.25 wt% of Soluplus added show a significant decrease in the content of urea nitrogen, and adding only 0.01 wt% of Soluplus can achieve a significant urea nitrogen-decreasing effect. However, when the amount of Soluplus is increased to 0.25 wt%, the effect of decreasing the content of urea nitrogen is not significantly improved.

Fig. 1(e) shows the removal results of urea nitrogen from blood using icodextrin peritoneal dialysis solutions with different amounts of micronized silica gel added. It can be seen that compared to the blank control group treated with the icodextrin peritoneal dialysis solution only, the experimental groups 5-1, 5-2 and 5-3 treated with icodextrin peritoneal dialysis solutions with 0.01 wt%, 0.05 wt% and 0.1 wt% of micronized silica gel added show a significant decrease in the content of urea nitrogen, and adding only 0.01 wt% of micronized silica gel can achieve a significant urea nitrogen-decreasing effect. However, when the amount of micronized silica gel is increased to 0.1 wt%, the effect of decreasing the content of urea nitrogen is not significantly improved.

Fig. 1(f) shows the removal results of urea nitrogen from blood using icodextrin peritoneal dialysis solutions with different amounts of diatomaceous earth added. It can be seen that compared to the blank control group treated with the icodextrin peritoneal dialysis solution only, the experimental groups 6-1, 6-2 and 6-3 treated with icodextrin peritoneal dialysis solutions with 0.01 wt%, 0.05 wt% and 0.1 wt% of diatomaceous earth added show a significant decrease in the content of urea nitrogen, and adding only 0.01 wt% of diatomaceous earth can achieve a significant urea nitrogen-decreasing effect. However, when the amount of diatomaceous earth is increased to 0.1 wt%, the effect of decreasing the content of urea nitrogen is not significantly improved.

### (2) Removal effect of creatinine from blood

Figs. 2 (a)-(f) show the removal results of creatinine from the blood of rats in the experimental groups as listed in Table 3 and in the corresponding positive control group (ICO+DA), blank control group (ICO) and negative control group.

Fig. 2(a) shows the removal results of creatinine from blood using icodextrin peritoneal dialysis solutions with different amounts of nano-carbon powder added. It can be seen that compared to the blank control group treated with the icodextrin peritoneal dialysis solution only, the experimental groups 1-1 to 1-4 treated with icodextrin peritoneal dialysis solutions with test amounts of nano-carbon powder added show no significant decrease in the content of creatinine.

Fig. 2(b) shows the removal results of creatinine from blood using icodextrin peritoneal dialysis solutions with different amounts of Kollidon CL-SF added. It can be seen that compared to the blank control group treated with the icodextrin peritoneal dialysis solution only, the experimental group 2-1 treated with an icodextrin peritoneal dialysis solution with 0.025 wt% of Kollidon CL-SF added shows no significant decrease in the content of creatinine, while the experimental group 2-2 treated with the icodextrin peritoneal dialysis solution with 0.25 wt% of Kollidon CL-SF added shows significant decrease in the content of creatinine in the first 4 hours.

Fig. 2(c) shows the removal results of creatinine from blood using icodextrin peritoneal dialysis solutions with different amounts of Kollidon CL-M added. It can be seen that compared to the blank control group treated with the icodextrin peritoneal dialysis solution only, the experimental group 3-1 treated with an icodextrin peritoneal dialysis solution with 0.025 wt% of Kollidon CL-M added shows no significant decrease in the content of creatinine, while the experimental group 3-2 treated with the icodextrin peritoneal dialysis solution with 0.25 wt% of Kollidon CL-SF added shows significant decrease in the content of creatinine in the first 4 hours.

Fig. 2(d) shows the removal results of creatinine from blood using icodextrin peritoneal dialysis solutions with different amounts of Soluplus added. It can be seen that compared to the blank control group treated with the icodextrin peritoneal dialysis solution only, the experimental groups 4-1, 4-2 and 4-3 treated with icodextrin peritoneal dialysis solutions with 0.01 wt%, 0.05 wt% and 0.25 wt% of Soluplus added show a significant continuous decrease in the content of creatinine, and adding only 0.01 wt% of Soluplus can achieve a significant creatinine-decreasing effect. However, when the amount of Soluplus is increased to 0.25 wt%, the effect of decreasing the content of creatinine is not significantly improved.

Fig. 2(e) shows the removal results of creatinine from blood using icodextrin peritoneal dialysis solutions with different amounts of micronized silica gel added. It can be seen that compared to the blank control group treated with the icodextrin peritoneal dialysis solution only, the experimental groups 5-1, 5-2 and 5-3 treated with icodextrin peritoneal dialysis solutions with 0.01 wt%, 0.05 wt% and 0.1 wt% of micronized silica gel added show a significant decrease in the content of creatinine, and adding only 0.01 wt% of micronized silica gel can achieve a significant creatinine-decreasing effect. However, when the amount of micronized silica gel is increased to 0.1 wt%, the effect of decreasing the content of creatinine is not significantly improved.

Fig. 2(f) shows the removal results of creatinine from blood using icodextrin peritoneal dialysis solutions with different amounts of diatomaceous earth added. It can be seen that compared to the blank control group treated with the icodextrin peritoneal dialysis solution only, the experimental groups 6-1, 6-2 and 6-3 treated with icodextrin peritoneal dialysis solutions with 0.01 wt%, 0.05 wt% and 0.1 wt% of diatomaceous earth added show a significant decrease in the content of creatinine, and adding only 0.01 wt% of diatomaceous earth can achieve a significant creatinine-decreasing effect. However, when the amount of diatomaceous earth is increased to 0.1 wt%, the effect of decreasing the content of urea nitrogen is not significantly improved.

### (3) Removal effect of indoxyl sulfate from blood

Figs. 3 (a)-(f) show the removal results of indoxyl sulfate from the blood of rats in the experimental groups as listed in Table 3 and in the corresponding positive control group (ICO+DA), blank control group (ICO) and negative control group.

Fig. 3(a) shows the removal results of indoxyl sulfate from blood using icodextrin peritoneal dialysis solutions with different amounts of nano-carbon powder added. It can be seen that compared to the blank control group treated with the icodextrin peritoneal dialysis solution only, the experimental groups 1-1 to 1-4 treated with icodextrin peritoneal dialysis solutions with 0.005 wt%, 0.1 wt%, 0.025 wt% or 0.25 wt% of nano-carbon powder added all show a significant decrease in the content of indoxyl sulfate, especially after treatment for 2 hours. The content of indoxyl sulfate continues to remain at a low level. Adding different amounts (0.005 wt%, 0.1 wt%, 0.025 wt% or 0.25 wt%) of nano-carbon powder shows similar indoxyl sulfate-decreasing effects.

Fig. 3(b) shows the removal results of indoxyl sulfate from blood using icodextrin peritoneal dialysis solutions with different amounts of Kollidon CL-SF added. It can be seen that compared to the blank control group treated with the icodextrin peritoneal dialysis solution only, the experimental groups 2-1 and 2-2 treated with icodextrin peritoneal dialysis solutions with 0.025 wt% or 0.25 wt% of Kollidon CL-SF added all show a significant decrease in the content of indoxyl sulfate, and the content of indoxyl sulfate continues to remain at a low level. Adding different amounts (0.025 wt% or 0.25 wt%) of Kollidon CL-SF shows similar indoxyl sulfate-decreasing effects.

Fig. 3(c) shows the removal results of indoxyl sulfate from blood using icodextrin peritoneal dialysis solutions with different amounts of Kollidon CL-M added. It can be seen that compared to the blank control group treated with the icodextrin peritoneal dialysis solution only, the experimental groups 3-1 and 3-2 treated with icodextrin peritoneal dialysis solutions with 0.025 wt% or 0.25 wt% of Kollidon CL-M added all show a significant decrease in the content of indoxyl sulfate, and the content of indoxyl sulfate continues to remain at a low level. Adding different amounts (0.025 wt% or 0.25 wt%) of Kollidon CL-M shows similar indoxyl sulfate-decreasing effects.

Fig. 3(d) shows the removal results of indoxyl sulfate from blood using icodextrin peritoneal dialysis solutions with different amounts of Soluplus added. It can be seen that compared to the blank control group treated with the icodextrin peritoneal dialysis solution only, the experimental groups 4-1, 4-2 and 4-3 treated with icodextrin peritoneal dialysis solutions with 0.01 wt%, 0.05 wt% and 0.25 wt% of Soluplus added all show a significant decrease in the content of indoxyl sulfate, and the content of indoxyl sulfate continues to remain at a low level. The experimental groups 4-1 and 4-2 treated with the icodextrin peritoneal dialysis solutions with 0.01 wt% and 0.05 wt% of Soluplus added show a continuous decrease trend in the content of indoxyl sulfate. In the first 4 hours, adding different amounts (0.01 wt%, 0.05 wt%, or 0.25% wt) of Soluplus shows similar indoxyl sulfate-decreasing effects.

Fig. 3(e) shows the removal results of indoxyl sulfate from blood using icodextrin peritoneal dialysis solutions with different amounts of micronized silica gel added. It can be seen that compared to the blank control group treated with the icodextrin peritoneal dialysis solution only, the experimental groups 5-1, 5-2 and 5-3 treated with icodextrin peritoneal dialysis solutions with 0.01 wt%, 0.05 wt% or 0.1 wt% of micronized silica gel added all show a significant decrease in the content of indoxyl sulfate, and the content of indoxyl sulfate continues to remain at a low level. Adding different amounts (0.01 wt%, 0.05 wt% or 0.1 wt%) of micronized silica gel shows similar indoxyl sulfate-decreasing effects.

Fig. 3(f) shows the removal results of indoxyl sulfate from blood using icodextrin peritoneal dialysis solutions with different amounts of diatomaceous earth added. It can be seen that compared to the blank control group treated with the icodextrin peritoneal dialysis solution only, the experimental groups 5-1, 5-2 and 5-3 treated with icodextrin peritoneal dialysis solutions with 0.01 wt%, 0.05 wt% or 0.1 wt% of diatomaceous earth added all show a significant decrease in the content of indoxyl sulfate, and the content of indoxyl sulfate continues to remain at a low level. Adding different amounts (0.01 wt%, 0.05 wt% or 0.1 wt%) of diatomaceous earth shows similar indoxyl sulfate-decreasing effects.

### (4) Changes in the content of urea nitrogen in peritoneal fluid

Figs. 4 (a)-(f) show the changes in the content of urea nitrogen in the peritoneal fluid of rats in the experimental group listed in Table 3 and in the corresponding positive control group (ICO+DA), blank control group (ICO) and negative control group.

Fig. 4(a) shows the changes in the content of urea nitrogen in peritoneal fluid treated with icodextrin peritoneal dialysis solutions with different amounts of nano-carbon powder added. It can be seen that compared to the blank control group treated with the icodextrin peritoneal dialysis solution only, the experimental groups 1-1 to 1-3 treated with icodextrin peritoneal dialysis solutions with 0.005 wt%, 0.1 wt% or 0.025 wt% of nano-carbon powder added show a significant increase in the content of urea nitrogen, while the experimental group 1-4 treated with the icodextrin peritoneal dialysis solution with 0.25 wt% of nano-carbon powder added shows a decrease in the content of urea nitrogen.

Fig. 4(b) shows the changes in the content of urea nitrogen in peritoneal fluid treated with icodextrin peritoneal dialysis solutions with different amounts of Kollidon CL-SF added. It can be seen that compared to the blank control group treated with the icodextrin peritoneal dialysis solution only, the experimental group 2-1 treated with the icodextrin peritoneal dialysis solution with 0.025 wt% of Kollidon CL-SF added shows a significant increase in the content of urea nitrogen in the first 2 hours, while the experimental group 2-2 treated with the icodextrin peritoneal dialysis solution with 0.25 wt% of Kollidon CL-SF added shows a significant decrease in the content of urea nitrogen.

Fig. 4(c) shows the changes in the content of urea nitrogen in peritoneal fluid treated with icodextrin peritoneal dialysis solutions with different amounts of Kollidon CL-M added. It can be seen that compared to the blank control group treated with the icodextrin peritoneal dialysis solution only, the experimental group 3-1 treated with the icodextrin peritoneal dialysis solution with 0.025 wt% of Kollidon CL-M added shows a similar change in the content of urea nitrogen, while the experimental group 3-2 treated with the icodextrin peritoneal dialysis solution with 0.25 wt% of Kollidon CL-M added shows a significant decrease in the content of urea nitrogen.

Fig. 4(d) shows the changes in the content of urea nitrogen in peritoneal fluid treated with icodextrin peritoneal dialysis solutions with different amounts of Soluplus added. It can be seen that compared to the blank control group treated with the icodextrin peritoneal dialysis solution only, the experimental groups 4-1 to 4-3 treated with icodextrin peritoneal dialysis solutions with 0.01 wt%, 0.05 wt% or 0.25 wt% of Soluplus added show a significant decrease in the content of urea nitrogen, while the three experimental groups have no significant difference from each other.

Fig. 4(e) shows the changes in the content of urea nitrogen in peritoneal fluid treated with icodextrin peritoneal dialysis solutions with different amounts of micronized silica gel added. It can be seen that compared to the blank control group treated with the icodextrin peritoneal dialysis solution only, the experimental groups 5-1 and 5-3 treated with icodextrin peritoneal dialysis solutions with 0.01 wt% or 0.1 wt% of micronized silica gel added show a decrease in the content of urea nitrogen, while the experimental group 5-2 treated with the icodextrin peritoneal dialysis solution with 0.05 wt% of micronized silica gel added shows a significant increase in the content of urea nitrogen.

Fig. 4(f) shows the changes in the content of urea nitrogen in peritoneal fluid treated with icodextrin peritoneal dialysis solutions with different amounts of diatomaceous earth added. It can be seen that compared to the blank control group treated with the icodextrin peritoneal dialysis solution only, the experimental groups 6-1 to 6-3 treated with icodextrin peritoneal dialysis solutions with 0.01 wt%, 0.05 wt% or 0.1 wt% of diatomaceous earth added show a significant decrease in the content of urea nitrogen, while the three experimental groups have no significant difference from each other.

### (5) Changes in the content of creatinine in peritoneal fluid

Figs. 5 (a)-(f) show the changes in the content of creatinine in the peritoneal fluid of rats in the experimental group listed in Table 3 and in the corresponding positive control group (ICO+DA), blank control group (ICO) and negative control group.

Fig. 5(a) shows the changes in the content of creatinine in peritoneal fluid treated with icodextrin peritoneal dialysis solutions with different amounts of nano-carbon powder added. It can be seen that compared to the blank control group treated with the icodextrin peritoneal dialysis solution only, the experimental groups 1-1 to 1-4 treated with icodextrin peritoneal dialysis solutions with 0.005 wt%, 0.1 wt%, 0.025 wt% and 0.25 wt% of nano-carbon powder added show an increase in the content of creatinine.

Fig. 5(b) shows the changes in the content of creatinine in peritoneal fluid treated with icodextrin peritoneal dialysis solutions with different amounts of Kollidon CL-SF added. It can be seen that compared to the blank control group treated with the icodextrin peritoneal dialysis solution only, the experimental groups 2-1 and 2-2 treated with icodextrin peritoneal dialysis solutions with 0.025 wt% or 0.25 wt% of Kollidon CL-SF added show a significant increase in the content of creatinine.

Fig. 5(c) shows the changes in the content of creatinine in peritoneal fluid treated with icodextrin peritoneal dialysis solutions with different amounts of Kollidon CL-M added. It can be seen that compared to the blank control group treated with the icodextrin peritoneal dialysis solution only, the experimental groups 3-1 and 3-2 treated with icodextrin peritoneal dialysis solutions with 0.025 wt% or 0.25 wt% of Kollidon CL-M added show a significant increase in the content of creatinine.

Fig. 5(d) shows the changes in the content of creatinine in peritoneal fluid treated with icodextrin peritoneal dialysis solutions with different amounts of Soluplus added. It can be seen that compared to the blank control group treated with the icodextrin peritoneal dialysis solution only, the experimental groups 4-1 to 4-3 treated with icodextrin peritoneal dialysis solutions with 0.01 wt%, 0.05 wt% or 0.25 wt% of Soluplus added show no significant difference.

Fig. 5(e) shows the changes in the content of creatinine in peritoneal fluid treated with icodextrin peritoneal dialysis solutions with different amounts of micronized silica gel added. It can be seen that compared to the blank control group treated with the icodextrin peritoneal dialysis solution only, the experimental groups 5-1 and 5-3 treated with icodextrin peritoneal dialysis solutions with 0.01 wt% or 0.1 wt% of micronized silica gel added show a significant decrease in the content of creatinine, while the experimental group 5-2 treated with the icodextrin peritoneal dialysis solution with 0.05 wt% of micronized silica gel added shows no significant difference.

Fig. 5(f) shows the changes in the content of creatinine in peritoneal fluid treated with icodextrin peritoneal dialysis solutions with different amounts of diatomaceous earth added. It can be seen that compared to the blank control group treated with the icodextrin peritoneal dialysis solution only, the experimental groups 6-1 to 6-3 treated with icodextrin peritoneal dialysis solutions with 0.01 wt%, 0.05 wt% or 0.1 wt% of diatomaceous earth added show a decrease in the content of creatinine, while the three experimental groups have no significant difference from each other.

### (6) Changes in the content of indoxyl sulfate in peritoneal fluid

Fig. 6 (a)-(f) show the changes in the content of indoxyl sulfate in the peritoneal fluid of rats in the experimental group listed in Table 3 and in the corresponding positive control group (ICO+DA), blank control group (ICO) and negative control group.

Fig. 6(a) shows the changes in the content of indoxyl sulfate in peritoneal fluid treated with icodextrin peritoneal dialysis solutions with different amounts of nano-carbon powder added. It can be seen that compared to the blank control group treated with the icodextrin peritoneal dialysis solution only, the experimental groups 1-1 to 1-4 treated with icodextrin peritoneal dialysis solutions with 0.005 wt%, 0.1 wt%, 0.025 wt% and 0.25 wt% of nano-carbon powder added show a decrease in the content of indoxyl sulfate.

Fig. 6(b) shows the changes in the content of indoxyl sulfate in peritoneal fluid treated with icodextrin peritoneal dialysis solutions with different amounts of Kollidon CL-SF added. It can be seen that compared to the blank control group treated with the icodextrin peritoneal dialysis solution only, the experimental groups 2-1 and 2-2 treated with icodextrin peritoneal dialysis solutions with 0.025 wt% or 0.25 wt% of Kollidon CL-SF added show a significant decrease in the content of indoxyl sulfate, while the two experimental groups have no significant difference from each other.

Fig. 6(c) shows the changes in the content of indoxyl sulfate in peritoneal fluid treated with icodextrin peritoneal dialysis solutions with different amounts of Kollidon CL-M added. It can be seen that compared to the blank control group treated with the icodextrin peritoneal dialysis solution only, the experimental groups 3-1 and 3-2 treated with icodextrin peritoneal dialysis solutions with 0.025 wt% or 0.25 wt% of Kollidon CL-M added show a significant decrease in the content of indoxyl sulfate, while the two experimental groups have no significant difference.

Fig. 6(d) shows the changes in the content of indoxyl sulfate in peritoneal fluid treated with icodextrin peritoneal dialysis solutions with different amounts of Soluplus added. It can be seen that compared to the blank control group treated with the icodextrin peritoneal dialysis solution only, the experimental groups 4-1 to 4-3 treated with icodextrin peritoneal dialysis solutions with 0.01 wt%, 0.05 wt% or 0.25 wt% of Soluplus added show no significant difference in the first 2 hours. However, afterward the experimental groups 4-2 and 4-3 show a significant increase in the content of indoxyl sulfate, while the experimental group 4-1 shows a decrease in the content of indoxyl sulfate.

Fig. 6(e) shows the changes in the content of indoxyl sulfate in peritoneal fluid treated with icodextrin peritoneal dialysis solutions with different amounts of micronized silica gel added. It can be seen that compared to the blank control group treated with the icodextrin peritoneal dialysis solution only, the experimental groups 5-1 to 5-3 treated with icodextrin peritoneal dialysis solutions with 0.01 wt%, 0.05 wt% or 0.1 wt% of micronized silica gel added show a significant decrease in the content of indoxyl sulfate, while the three experimental groups have no significant difference from each other.

Fig. 6(f) shows the changes in the content of indoxyl sulfate in peritoneal fluid treated with icodextrin peritoneal dialysis solutions with different amounts of diatomaceous earth added. It can be seen that compared to the blank control group treated with the icodextrin peritoneal dialysis solution only, the experimental groups 6-1 to 6-3 treated with icodextrin peritoneal dialysis solutions with 0.01 wt%, 0.05 wt% or 0.1 wt% of diatomaceous earth added show a decrease in the content of indoxyl sulfate in 8 hours. In the first 4 hours, the three experimental groups have no significant difference from each other, but from the 4^{th} hour, the experimental group 6-3 shows a significant increase in the content of indoxyl sulfate.

### Example 3-3: Glucose peritoneal dialysis solutions with different amounts of toxin-removal reagents added

The following table lists the experimental grouping of rats subjected to peritoneal dialysis treatment using glucose peritoneal dialysis solutions containing different amounts of toxin-removal reagents.

**Table 4. Experimental groups of rats treated with glucose peritoneal dialysis solutions containing different amounts of toxin-removal reagents**

| **Experimental group** | **Toxin-removal reagent** | **Amount of toxin-removal reagent (wt%)** |
|---|---|---|
| 1-1 | Nano-carbon powder | 0.01 |
| 1-2 | | 0.05 |
| 1-3 | | 0.25 |
| 2-1 | Kollidon CL-SF | 0.01 |
| 2-2 | | 0.05 |
| 2-3 | | 0.25 |
| 3-1 | Kollidon CL-M | 0.01 |
| 3-2 | | 0.05 |
| 3-3 | | 0.25 |
| 4-1 | Soluplus | 0.01 |
| 4-2 | | 0.05 |
| 4-3 | | 0.25 |
| 5-1 | Micronized silica gel | 0.01 |
| 5-2 | | 0.05 |
| 5-3 | | 0.25 |
| 6-1 | Diatomaceous earth | 0.01 |
| 6-2 | | 0.05 |
| 6-3 | | 0.25 |

According to the method described in Example 3-2, the rats in the experimental groups, blank control group and negative control group were subjected to peritoneal dialysis, and the content of the toxins (urea nitrogen (BUN), creatinine (CREA2) and indoxyl sulfate (IS)) in tail venous blood and peritoneal fluid was tested.

### Result:

### (1) Removal effect of urea nitrogen from blood

Figs. 7 (a)-(f) show the removal result of urea nitrogen from the blood of rats in the experimental groups listed in Table 4 and in the corresponding blank control group (GLU) and negative control group.

Fig. 7(a) shows the removal results of urea nitrogen from blood using glucose peritoneal dialysis solutions with different amounts of nano-carbon powder added. It can be seen that compared to the blank control group treated with the glucose peritoneal dialysis solution only, the experimental groups 1-1 and 1-2 treated with glucose peritoneal dialysis solutions with 0.01 wt% and 0.05 wt% of nano-carbon powder added show a significant decrease in the content of urea nitrogen, while the experimental group 1-3 treated with the glucose peritoneal dialysis solution with 0.25 wt% of nano-carbon powder added shows no significant decrease in the content of urea nitrogen in the first 2 hours, but after 2 hours, starts to show a certain decrease in the content of urea nitrogen.

Fig. 7(b) shows the removal results of urea nitrogen from blood using glucose peritoneal dialysis solutions with different amounts of Kollidon CL-SF added. It can be seen that compared to the blank control group treated with the glucose peritoneal dialysis solution only, the experimental groups 2-1 to 2-3 treated with glucose peritoneal dialysis solutions with 0.01 wt%, 0.05 wt% and 0.25 wt% of Kollidon CL-SF added show a significant decrease in the content of urea nitrogen, while the experimental group 2-3 treated with the glucose peritoneal dialysis solution with 0.25 wt% of Kollidon CL-SF added starts to show an increase in the content of urea nitrogen after 4 hours.

Fig. 7(c) shows the removal results of urea nitrogen from blood using glucose peritoneal dialysis solutions with different amounts of Kollidon CL-M added. It can be seen that compared to the blank control group treated with the glucose peritoneal dialysis solution only, the experimental groups 3-1 to 3-3 treated with glucose peritoneal dialysis solutions with 0.01 wt%, 0.05 wt% and 0.25 wt% of Kollidon CL-M added show a significant decrease in the content of urea nitrogen, while the three experimental groups have no significant difference from each other.

Fig. 7(d) shows the removal results of urea nitrogen from blood using glucose peritoneal dialysis solutions with different amounts of Soluplus added. It can be seen that compared to the blank control group treated with the glucose peritoneal dialysis solution only, the experimental groups 4-1 to 4-3 treated with glucose peritoneal dialysis solutions with 0.01 wt%, 0.05 wt% and 0.25 wt% of Soluplus added show a significant decrease in the content of urea nitrogen, while the three experimental groups have no significant difference from each other.

Fig. 7(e) shows the removal results of urea nitrogen from blood using glucose peritoneal dialysis solutions with different amounts of micronized silica gel added. It can be seen that compared to the blank control group treated with the glucose peritoneal dialysis solution only, the experimental groups 5-1 to 5-3 treated with glucose peritoneal dialysis solutions with 0.01 wt%, 0.05 wt% and 0.25 wt% of micronized silica gel added show a significant decrease in the content of urea nitrogen. The experimental group 5-1 with 0.01 wt% of micronized silica gel added shows the best urea nitrogen-decreasing effect.

Fig. 7(f) shows the removal results of urea nitrogen from blood using glucose peritoneal dialysis solutions with different amounts of diatomaceous earth added. It can be seen that compared to the blank control group treated with the glucose peritoneal dialysis solution only, the experimental groups 6-1 to 6-3 treated with glucose peritoneal dialysis solutions with 0.01 wt%, 0.05 wt% and 0.25 wt% of diatomaceous earth added show a significant decrease in the content of urea nitrogen. The experimental group 6-3 with 0.25 wt% of micronized silica gel added shows the best urea nitrogen-decreasing effect.

### (2) Removal result of creatinine from blood

Figs. 8 (a)-(f) show the removal results of creatinine from the blood of rats in the experimental groups listed in Table 4 and in the corresponding blank control group (GLU) and negative control group.

Fig. 8(a) shows the removal results of creatinine from blood using glucose peritoneal dialysis solutions with different amounts of nano-carbon powder added. It can be seen that compared to the blank control group treated with the glucose peritoneal dialysis solution only, the experimental groups 1-2 and 1-3 treated with glucose peritoneal dialysis solutions with 0.05 wt% and 0.25 wt% of nano-carbon powder added show no significant difference, while the experimental group 1-1 treated with glucose peritoneal dialysis solutions with 0.01 wt% of nano-carbon powder added shows a significant decrease in the content of creatinine.

Fig. 8(b) shows the removal results of creatinine from blood using glucose peritoneal dialysis solutions with different amounts of Kollidon CL-SF added. It can be seen that compared to the blank control group treated with the glucose peritoneal dialysis solution only, the experimental groups 2-1 to 2-3 treated with glucose peritoneal dialysis solutions with 0.01 wt%, 0.05 wt% and 0.25 wt% of Kollidon CL-SF added show a significant decrease in the content of creatinine, while the three experimental groups have no significant difference from each other.

Fig. 8(c) shows the removal results of creatinine from blood using glucose peritoneal dialysis solutions with different amounts of Kollidon CL-M added. It can be seen that compared to the blank control group treated with the glucose peritoneal dialysis solution only, the experimental groups 3-1 to 3-3 treated with glucose peritoneal dialysis solutions with 0.01 wt%, 0.05 wt% and 0.25 wt% of Kollidon CL-M added show a significant decrease in the content of creatinine, while the three experimental groups have no significant difference from each other.

Fig. 8(d) shows the removal results of creatinine from blood using glucose peritoneal dialysis solutions with different amounts of Soluplus added. It can be seen that compared to the blank control group treated with the glucose peritoneal dialysis solution only, the experimental groups 4-1 to 4-3 treated with glucose peritoneal dialysis solutions with 0.01 wt%, 0.05 wt% and 0.25 wt% of Soluplus added show a significant decrease in the content of creatinine. The experimental groups 4-1 and 4-3 with 0.01 wt% and 0.25 wt% of Soluplus added have similar creatinine-removal effect, which is better than that of the experimental group 4-2 with 0.05 wt% of Soluplus added.

Fig. 8(e) shows the removal results of creatinine from blood using glucose peritoneal dialysis solutions with different amounts of micronized silica gel added. It can be seen that compared to the blank control group treated with the glucose peritoneal dialysis solution only, the experimental groups 5-1 to 5-3 treated with glucose peritoneal dialysis solutions with 0.01 wt%, 0.05 wt% and 0.25 wt% of micronized silica gel added show a significant decrease in the content of creatinine. The experimental group 5-1 with 0.01 wt% of micronized silica gel added shows the best creatinine-removal effect.

Fig. 8(f) shows the removal results of creatinine from blood using glucose peritoneal dialysis solutions with different amounts of diatomaceous earth added. It can be seen that compared to the blank control group treated with the glucose peritoneal dialysis solution only, the experimental groups 6-1 to 6-3 treated with glucose peritoneal dialysis solutions with 0.01 wt%, 0.05 wt% and 0.25 wt% of diatomaceous earth added show a significant decrease in the content of creatinine, while the three experimental groups have no significant difference from each other.

### (3) Removal effect of indoxyl sulfate from blood

Figs. 9 (a)-(f) show the removal results of indoxyl sulfate from the blood of rats in the experimental groups listed in Table 4 and in the corresponding blank control group (GLU) and negative control group.

Fig. 9(a) shows the removal results of indoxyl sulfate from blood using glucose peritoneal dialysis solutions with different amounts of nano-carbon powder added. It can be seen that compared to the blank control group treated with the glucose peritoneal dialysis solution only, the experimental groups 1-1 to 1-3 treated with glucose peritoneal dialysis solutions with 0.01 wt%, 0.05 wt% and 0.25 wt% of nano-carbon powder added show a significant decrease in the content of indoxyl sulfate, while the three experimental groups have no significant difference from each other.

Fig. 9(b) shows the removal results of creatinine from blood using glucose peritoneal dialysis solutions with different amounts of Kollidon CL-SF added. It can be seen that compared to the blank control group treated with the glucose peritoneal dialysis solution only, the experimental group 2-2 treated with the glucose peritoneal dialysis solution with 0.05 wt% of Kollidon CL-SF added shows no significant difference, while the experimental groups 2-1 and 2-3 treated with the glucose peritoneal dialysis solutions with 0.01 wt% and 0.25 wt% of Kollidon CL-SF added show a significant decrease in the content of indoxyl sulfate, and the two experimental groups have no significant difference from each other.

Fig. 9(c) shows the removal results of indoxyl sulfate from blood using glucose peritoneal dialysis solutions with different amounts of Kollidon CL-M added. It can be seen that compared to the blank control group treated with the glucose peritoneal dialysis solution only, the experimental groups 3-1 to 3-3 treated with glucose peritoneal dialysis solutions with 0.01 wt%, 0.05 wt% and 0.25 wt% of Kollidon CL-M added show a significant decrease in the content of indoxyl sulfate, while the three experimental groups have no significant difference from each other.

Fig. 9(d) shows the removal results of indoxyl sulfate from blood using glucose peritoneal dialysis solutions with different amounts of Soluplus added. It can be seen that compared to the blank control group treated with the glucose peritoneal dialysis solution only, the experimental groups 4-1 to 4-3 treated with glucose peritoneal dialysis solutions with 0.01 wt%, 0.05 wt% and 0.25 wt% of Soluplus added show a significant decrease in the content of indoxyl sulfate. The experimental group 4-1 with 0.01 wt% of Soluplus added has the best indoxyl sulfate-removal effect, the experimental group 4-3 with 0.25 wt% of Soluplus added takes the second place for the indoxyl sulfate-removal effect, and the experimental group 4-2 with 0.05 wt% of Soluplus added takes the third place.

Fig. 9(e) shows the removal results of indoxyl sulfate from blood using glucose peritoneal dialysis solutions with different amounts of micronized silica gel added. It can be seen that compared to the blank control group treated with the glucose peritoneal dialysis solution only, the experimental groups 5-1 to 5-3 treated with glucose peritoneal dialysis solutions with 0.01 wt%, 0.05 wt% and 0.25 wt% of micronized silica gel added show significant decrease in the content of indoxyl sulfate. The experimental group 5-1 with 0.01 wt% of micronized silica gel added has the best indoxyl sulfate-removal effect.

Fig. 9(f) shows the removal results of creatinine from blood using glucose peritoneal dialysis solutions with different amounts of diatomaceous earth added. It can be seen that compared to the blank control group treated with the glucose peritoneal dialysis solution only, the experimental group 6-1 treated with the glucose peritoneal dialysis solution with 0.01 wt% of diatomaceous earth added shows no significant difference, while the experimental groups 6-2 and 6-3 treated with the glucose peritoneal dialysis solutions with 0.05 wt% and 0.25 wt% of diatomaceous earth added show a significant decrease in the content of indoxyl sulfate, and the two experimental groups have no significant difference from each other.

### (4) Changes in the content of urea nitrogen in peritoneal fluid

Fig. 10 (a)-(f) show the changes in the content of urea nitrogen in the peritoneal fluid of rats in the experimental groups listed in Table 4 and in the corresponding blank control group (GLU) and negative control group.

Fig. 10(a) shows the changes in the content of urea nitrogen in peritoneal fluid treated with glucose peritoneal dialysis solutions with different amounts of nano-carbon powder added. It can be seen that compared to the blank control group treated with the glucose peritoneal dialysis solution only, the experimental groups 1-1 to 1-3 treated with glucose peritoneal dialysis solutions with 0.01 wt%, 0.05 wt% or 0.25 wt% of nano-carbon powder added show a increase in the content of urea nitrogen. The experimental group 1-2 with 0.05 wt% of nano-carbon powder added shows the highest increase in the content of urea nitrogen, the experimental group 1-3 with 0.25 wt% of nano-carbon powder added takes the second place, and the experimental group 1-1 with 0.01 wt% of nano-carbon powder added takes the third place.

Fig. 10(b) shows the changes in the content of urea nitrogen in peritoneal fluid treated with glucose peritoneal dialysis solutions with different amounts of Kollidon CL-SF added. It can be seen that compared to the blank control group treated with the glucose peritoneal dialysis solution only, the experimental groups 2-1 to 2-3 treated with glucose peritoneal dialysis solutions with 0.01 wt%, 0.05 wt% or 0.25 wt% of Kollidon CL-SF added show an increase in the content of urea nitrogen. The three experimental groups have no significant difference from each other in the first 4 hours, but after 4 hours, the experimental groups 2-1 and 2-2 with 0.01 wt% and 0.05 wt% of Kollidon CL-SF added show a significant decrease in the content of urea nitrogen until the content of urea nitrogen is close to that of the negative control group.

Fig. 10(c) shows the changes in the content of urea nitrogen in peritoneal fluid treated with glucose peritoneal dialysis solutions with different amounts of Kollidon CL-M added. It can be seen that compared to the blank control group treated with the glucose peritoneal dialysis solution only, the experimental groups 3-1 to 3-3 treated with glucose peritoneal dialysis solutions with 0.01 wt%, 0.05 wt% or 0.25 wt% of Kollidon CL-M added show an increase in the content of urea nitrogen, while the three experimental groups have no significant difference from each other.

Fig. 10(d) shows the changes in the content of urea nitrogen in peritoneal fluid treated with glucose peritoneal dialysis solutions with different amounts of Soluplus added. It can be seen that compared to the blank control group treated with the glucose peritoneal dialysis solution only, the experimental groups 4-1 to 4-3 treated with glucose peritoneal dialysis solutions with 0.01 wt%, 0.05 wt% or 0.25 wt% of Soluplus added show an increase in the content of urea nitrogen in the first 2 hours, but later the three experimental groups have no significant difference from the negative control group.

Fig. 10(e) shows the changes in the content of urea nitrogen in peritoneal fluid treated with glucose peritoneal dialysis solutions with different amounts of micronized silica gel added. It can be seen that compared to the blank control group treated with the glucose peritoneal dialysis solution only, the experimental groups 5-2 and 5-3 treated with glucose peritoneal dialysis solutions with 0.05 wt% or 0.25 wt% of micronized silica gel added show a significant increase in the content of urea nitrogen, and the two experimental groups have no significant difference from each other; the experimental group 5-1 with 0.01 wt% of micronized silica gel added shows no significant difference from the negative control group in the first 2 hours, but later shows a significant decrease in the content of urea nitrogen.

Fig. 10(f) shows the changes in the content of urea nitrogen in peritoneal fluid treated with glucose peritoneal dialysis solutions with different amounts of diatomaceous earth added. It can be seen that compared to the blank control group treated with the glucose peritoneal dialysis solution only, the experimental groups 5-1 to 5-3 treated with glucose peritoneal dialysis solutions with 0.05 wt%, 0.05 wt% or 0.25 wt% of diatomaceous earth added show no significant difference.

### (5) Changes in the content of creatinine in peritoneal fluid

Fig. 11(a)-(f) show the changes in the content of creatinine in the peritoneal fluid of rats in the experimental groups listed in Table 4 and in the corresponding blank control group (GLU) and negative control group.

Fig. 11(a) shows the changes in the content of creatinine in peritoneal fluid treated with glucose peritoneal dialysis solutions with different amounts of nano-carbon powder added. It can be seen that compared to the blank control group treated with the glucose peritoneal dialysis solution only, the experimental group 1-1 treated with the glucose peritoneal dialysis solution with 0.01 wt% of nano-carbon powder added shows no significant difference, while the experimental groups 1-2 and 1-3 treated with the glucose peritoneal dialysis solution with 0.05 wt% or 0.25 wt% of nano-carbon powder added show a significant increase in the content of urea nitrogen, and the two experimental groups have no significant difference from each other.

Fig. 11(b) shows the changes in the content of creatinine in peritoneal fluid treated with glucose peritoneal dialysis solutions with different amounts of Kollidon CL-SF added. It can be seen that compared to the blank control group treated with the glucose peritoneal dialysis solution only, the experimental groups 2-1 to 2-3 treated with glucose peritoneal dialysis solutions with 0.01 wt%, 0.05 wt% or 0.25 wt% of Kollidon CL-SF added show an increase in the content of creatinine. The three experimental groups have no significant difference from each other in the first 4 hours, but after 4 hours, the experimental groups 2-1 and 2-2 with 0.01 wt% and 0.05 wt% of Kollidon CL-SF added show a significant decrease in the content of creatinine until the content of creatinine is close to that of the negative control group.

Fig. 11(c) shows the changes in the content of creatinine in peritoneal fluid treated with glucose peritoneal dialysis solutions with different amounts of Kollidon CL-M added. It can be seen that compared to the blank control group treated with the glucose peritoneal dialysis solution only, the experimental groups 3-1 to 3-3 treated with glucose peritoneal dialysis solutions with 0.01 wt%, 0.05 wt% or 0.25 wt% of Kollidon CL-SF added show no significant difference, but after 4 hours, the experimental group 2-1 with 0.01 wt% of Kollidon CL-SF added shows a significant increase in the content of creatinine.

Fig. 11(d) shows the changes in the content of urea nitrogen in peritoneal fluid treated with glucose peritoneal dialysis solutions with different amounts of Soluplus added. It can be seen that compared to the blank control group treated with the glucose peritoneal dialysis solution only, the experimental groups 4-1 and 4-3 treated with glucose peritoneal dialysis solutions with 0.01 wt% and 0.25 wt% of Soluplus added show no significant difference, while the experimental group 4-2 treated with the glucose peritoneal dialysis solution with 0.05 wt% of Soluplus added shows a significant increase in the content of creatinine.

Fig. 11(e) shows the changes in the content of creatinine in peritoneal fluid treated with glucose peritoneal dialysis solutions with different amounts of micronized silica gel added. It can be seen that compared to the blank control group treated with the glucose peritoneal dialysis solution only, the experimental group 5-1 treated with the glucose peritoneal dialysis solution with 0.01 wt% of micronized silica gel added shows no significant difference, while the experimental groups 5-2 and 5-3 with 0.05 wt% and 0.25 wt% of micronized silica gel added show a significant increase in the content of creatinine, and the two experimental groups have no significant difference from each other.

Fig. 11(f) shows the changes in the content of creatinine in peritoneal fluid treated with glucose peritoneal dialysis solutions with different amounts of diatomaceous earth added. It can be seen that compared to the blank control group treated with the glucose peritoneal dialysis solution only, the experimental groups 5-2 and 5-3 treated with the glucose peritoneal dialysis solutions with 0.05 wt% and 0.25 wt% of diatomaceous earth added show no significant difference, while the experimental group 5-1 with 0.01 wt% of diatomaceous earth added shows a significant increase in the content of creatinine.

### (6) Changes in the content of indoxyl sulfate in peritoneal fluid

Fig. 12 (a)-(f) show the changes in the content of indoxyl sulfate in the peritoneal fluid of rats in the experimental groups listed in Table 4 and in the corresponding blank control group (GLU) and negative control group.

Fig. 12(a) shows the changes in the content of indoxyl sulfate in peritoneal fluid treated with glucose peritoneal dialysis solutions with different amounts of nano-carbon powder added. It can be seen that compared to the blank control group treated with the glucose peritoneal dialysis solution only, the experimental groups 1-1 and 1-3 treated with the glucose peritoneal dialysis solutions with 0.01 wt% or 0.25 wt% of nano-carbon powder added show no significant difference, while the experimental group 1-2 treated with the glucose peritoneal dialysis solution with 0.05 wt% of nano-carbon powder added shows an increase in the content of indoxyl sulfate.

Fig. 12(b) shows the changes in the content of indoxyl sulfate in peritoneal fluid treated with glucose peritoneal dialysis solutions with different amounts of Kollidon CL-SF added. It can be seen that compared to the blank control group treated with the glucose peritoneal dialysis solution only, the experimental groups 2-1 to 2-3 treated with glucose peritoneal dialysis solutions with 0.01 wt%, 0.05 wt% or 0.25 wt% of Kollidon CL-SF added show a significant continuous increase in the content of indoxyl sulfate.

Fig. 12(c) shows the changes in the content of indoxyl sulfate in peritoneal fluid treated with glucose peritoneal dialysis solutions with different amounts of Kollidon CL-M added. It can be seen that compared to the blank control group treated with the glucose peritoneal dialysis solution only, the experimental groups 3-1 to 3-3 treated with glucose peritoneal dialysis solutions with 0.01 wt%, 0.05 wt% or 0.25 wt% of Kollidon CL-M added show a significant continuous increase in the content of indoxyl sulfate.

Fig. 12(d) shows the changes in the content of indoxyl sulfate in peritoneal fluid treated with glucose peritoneal dialysis solutions with different amounts of Soluplus added. It can be seen that compared to the blank control group treated with the glucose peritoneal dialysis solution only, the experimental groups 4-1 to 4-3 treated with glucose peritoneal dialysis solutions with 0.01 wt%, 0.05 wt% or 0.25 wt% of Soluplus added show no significant difference in the first 2 hours, but after 2 hours, the experimental group 4-2 with 0.05 wt% of Soluplus added shows a significant continuous increase in the content of indoxyl sulfate, and the experimental groups 4-1 and 4-3 with 0.01 wt% or 0.25 wt% of Soluplus added show no significant difference from the negative control group.

Fig. 12(e) shows the changes in the content of indoxyl sulfate in peritoneal fluid treated with glucose peritoneal dialysis solutions with different amounts of micronized silica gel added. It can be seen that compared to the blank control group treated with the glucose peritoneal dialysis solution only, the experimental group 5-1 treated with the glucose peritoneal dialysis solution with 0.01 wt% of micronized silica gel added shows no significant difference, while the experimental groups 5-2 and 5-3 with 0.05 wt% and 0.25 wt% of micronized silica gel added show a significant continuous increase in the content of indoxyl sulfate, and the two experimental groups have no significant difference from each other.

Fig. 12(f) shows the changes in the content of indoxyl sulfate in peritoneal fluid treated with glucose peritoneal dialysis solutions with different amounts of diatomaceous earth added. It can be seen that compared to the negative control group treated with the glucose peritoneal dialysis solution only, the experimental groups 6-1 and 6-2 treated with glucose peritoneal dialysis solutions with 0.01 wt% and 0.05 wt% of diatomaceous earth added show a significant increase in the content of indoxyl sulfate, while the experimental group 6-3 with 0.25 wt% of diatomaceous earth added shows a significant increase in the content of indoxyl sulfate in the first 4 hours, but after 4 hours, shows a significant decrease in the content of indoxyl sulfate until the content of indoxyl sulfate is close to that of the negative control group.

The present invention is not limited to the scope of the specific embodiments described herein. In fact, based on the above description, various modifications and changes of the present invention are easily conceivable for those skilled in the art. These modifications and changes also fall within the scope of the appended claims.

## Claims

1. A composition comprising an osmotic agent and a toxin-removal reagent, wherein the osmotic agent provides an osmotic pressure substantially equal to or higher than that of a biofluid, and the toxin-removal reagent reduces a toxin in the biofluid under a condition for osmosis.

2. The composition according to claim 1, wherein the toxin-removal reagent reduces free amount, non-free amount, and/or total amount of the toxin in the biofluid.

3. The composition according to claim 2, wherein the toxin-removal reagent reduces total amount or non-free amount of the toxin in the biofluid by at least 10%, at least 20%, at least 30%, at least 40%, at least 50%, at least 60%, at least 70%, at least 80%, at least 90%, or at least 95%.

4. The composition according to any one of the preceding claims, wherein the toxin-removal reagent adsorbs, non-covalently binds to, covalently binds to, and/or degrades the toxin in the biofluid.

5. The composition according to any one of the preceding claims, wherein the toxin-removal reagent has one or more characteristics selected from the group consisting of: 1) having a porous structure; 2) capable of forming a charged structure; 3) capable of binding to the toxin through a non-covalent bond or a covalent bond or an ionic bond; and 4) capable of degrading the toxin.

6. The composition according to claim 5, wherein the toxin-removal reagent has a porous structure, and the porous structure has one or more of the following characteristics:
1) having a specific surface area of 70 cm²/g-1000m²/g;
2) having a pore size in a range of 0.1 nm-10 µm;
3) having a pore size distribution of 0.1 nm-100 µm;
4) having a porosity of about 5-95%; and
5) capable of adsorbing the toxin at an adsorption rate of at least 0.2 mg/g.

7. The composition according to claim 6, wherein the toxin-removal reagent having a porous structure is selected from the group consisting of a silicon-based porous material, a carbon-based porous material, a metal oxide porous material, a polymer porous material, and a metal-organic framework compound based porous material.

8. The composition according to claim 5, wherein the toxin-removal reagent is capable of forming a charged structure, and the charged structure has a charge density of 0.2-50 µC·cm⁻².

9. The composition according to claim 8, wherein the charged structure comprises charged ions or charged colloids.

10. The composition according to claim 8, wherein the toxin-removal reagent is selected from the group consisting of povidone, crospovidone, silica colloid, micronized silica gel, diatomaceous earth, polyethylene caprolactam-polyvinyl acetate-polyethylene glycol graft copolymer, and nano alumina.

11. The composition according to claim 5, wherein the toxin-removal reagent has a group capable of forming a non-covalent bond with the toxin (e.g., hydrogen atom, hydroxyl group, amino group, amine group, and carboxyl group, etc.), has a group capable of forming a covalent bond with the toxin (e.g., sulfhydryl group, aldehyde group, hydroxyl group, carboxyl group, amino group, acylhydrazone bond, and acylhydrazine bond, etc.), or has a group capable of forming an ionic bond with the toxin (e.g., chloride ion, sulfate ion, calcium ion, and carbonate ion, etc.).

12. The composition according to claim 5, wherein the toxin-removal reagent is capable of degrading the toxin, and the toxin-removal reagent is a biocatalyst or a chemical catalyst.

13. The composition according to any one of the preceding claims, wherein the toxin-removal reagent is selected from the group consisting of activated carbon, povidone, crospovidone, polyethylene caprolactam-polyvinyl acetate-polyethylene glycol graft copolymer, micronized silica gel, diatomaceous earth, and any combination thereof.

14. The composition according to any one of the preceding claims, wherein the presence or excessive presence of the toxin in the biofluid can increase the risk of disease, aggravate the disease condition, or impair normal physiological functions.

15. The composition according to any one of the preceding claims, wherein the toxin comprise a metabolite *in vivo*, an exogenous poisonous substance, or a disease-inducing molecule.

16. The composition according to claim 15, wherein the metabolite is selected from the group consisting of urea, creatinine, uric acid, guanidine-ADMA, β₂-microglobulin, cytokines, parathyroid hormone, indoxyl sulfate, homocysteine, p-cresol, hippuric acid, reactive oxygen species (ROS), uremic toxins (such as AGE products (3-deoxyglucosone, fructoselysine, glyoxal, pyruvaldehyde, pentosidine), 1-methyladenosine, 1-methylguanosine, 1-methylinosine, asymmetric dimethylarginine, α-keto-δ-guanidinovaleric acid, α-N-acetylarginine, arabitol, arginine, benzyl alcohol, β-guanidinopropionic acid, β-lipotrophic hormone, creatine, cytidine, sodium N,N-dimethylglycinate, erythritol, γ-guanidinobutyric acid, hypoxanthine, malondialdehyde, mannitol, methylguanidine, inositol, N,N-dimethylguanosine, N-acetyl cytosine nucleoside, N-threonyl carbamoyl adenosine phosphate, orotic acid, orotidine, oxalate, phenylacetylglutamine, pseudouridine, symmetric dimethylarginine, sorbitol, taurocyamine, threitol, thymine, uracil, uridine, xanthosine, 2-methoxyresorcinol, 3-deoxyglucosone, 3-carboxy-4-methyl-5-propyl-2-furanpropionic acid (CMPF), fructosyllysine, homocysteine, hydroquinone, indole-3-acetic acid, kynurenine, kynurenic acid, leptin, melatonin, methylglyoxal, Nε-carboxymethyllysine, cresol, pentoside, phenol, p-hydroxyhippuric acid, butanediamine, quinolinic acid, retinol conjugated protein, spermidine, spermine, adrenomedullin, atrial natriuretic peptide, β-endorphin, cholecystokinin, Clara cell protein (CC16), human complement factor D, cystatin C, degranulation inhibitor protein Ic, δ-sleep-inducing peptide, endothelin, hyaluronic acid, interleukin-1β, interleukin-6, κ-immunoglobulin light chain, λ-immunoglobulin light chain, methionine-enkephalin, neuropeptide Y, parathyroid hormone, tumor necrosis factor-α), hydrogen sulfide, and bilirubin.

17. The composition according to claim 15, wherein the exogenous poisonous substance comprises a drug, a pesticide, a chemical poison, a food-borne poison, or an exogenous biological toxin.

18. The composition according to claim 17, wherein the drug is selected from the group consisting of a sedative and hypnotic drug, an antipsychotic drug, a cardiovascular and cerebrovascular drug, an antipyretic and analgesic drug, an antiparasitic drug, an antimicrobial drug, an anesthetics and anesthetic assistant, a respiratory system drug, a circulatory system drug, a digestive system drug, a urinary system drug, a blood system drug, a metabolism and endocrine drug, an antiallergic drug, a tumor treatment drug, an immunomodulatory drug, an obstetrics and gynecology drug, a male drug, an antiinflammatory drug, and a traditional Chinese medicine.

19. The composition according to claim 17, wherein the pesticide is selected from the group consisting of paraquat, dichlorvos, dimethoate, glyphosate, methamidophos, chlorpyrifos, omethoate, bromadiolone, brodifacoum, phoxim, imidacloprid, abamectin, silatrane, dimehypo, parathion, phorate, demeton, trichlorfon, malathion, benzene hexachloride, clofenotane, dieldrin, endrin, aldrin, aluminum phosphide pesticide, fenvalerate, fluoroacetamide, tetramine, pentachlorophenol, dinitrocresol, diuron, dichlobenil, nereistoxin, rotenone, nicotine, methanonaphthalene chloride, formamidine, bacillus thuringiensis preparation, diphacinone sodium salt, warfarin, pyrinuron, amobam, ziram, methylmercury, and 2,4D butyl ester.

20. The composition according to claim 17, wherein the chemical poison is selected from the group consisting of a toxin derived from poisonous plants (such as Abrus precatorius, oleander, datura, colchicum, palythoa toxica, lupin, and rattlebush), a cyanide (such as sodium cyanide, potassium cyanide, calcium cyanide, barium cyanide, cobaltous cyanide, cobalthous cyanide, potassium cobalt cyanide, nickel cyanide, nickel potassium cyanide, copper cyanide, silver cyanide, silver potassium cyanide, zinc cyanide, cadmium cyanide, mercury cyanide, mercuric potassium cyanide, lead cyanide, cerium cyanide, cuprous cyanide, potassium gold cyanide, bromine cyanide, hydrogen cyanide, and hydrocyanic acid), xylene, automobile anti-freeze fluid, arsenic trioxide, sodium arsenite, potassium arsenite, arsenic pentoxide, arsenic trichloride, potassium selenite, sodium selenate, potassium selenate, selenium oxychloride, mercury chloride, mercuric oxycyanide, cadmium oxide, nickel carbonyl, iron pentacarbonyl, sodium azide, barium azide, hydrazoic acid, hydrogen fluoride, yellow phosphorus, sodium phosphide, potassium phosphide, magnesium phosphide, aluminum phosphide, fluorine, phosphine, arsine, hydrogen selenide, stibine, nitrogen monoxide, dinitrogen tetroxide, sulfur dioxide, chlorine dioxide, oxygen difluoride, chlorine trifluoride, phosphorus trifluoride, sulfur tetrafluoride, silicon tetrafluoride, chlorine pentafluoride, phosphorus pentafluoride, selenium hexafluoride, tellurium hexafluoride, tungsten hexafluoride, bromine chloride, cyanogen chloride, bromophosgene, cyanogen, cyanogen iodide, arsenic, calcium arsenite, strontium arsenite, barium arsenite, iron arsenite, copper arsenite, silver arsenite, zinc arsenite, lead arsenite, antimony arsenite, copper acetoarsenite, arsenic acid, meta-arsenic acid, pyroarsenic acid, ammonium arsenate, sodium arsenate, sodium meta-arsenate, disodium hydrogen arsenate, disodium hydrogen arsenate, sodium dihydrogen arsenate, potassium arsenate, potassium dihydrogen arsenate, magnesium arsenate, calcium arsenate, barium arsenate, iron arsenate, ferrous arsenate, copper arsenate, silver arsenate, zinc arsenate, mercury arsenate, lead arsenate, antimony arsenate, arsenic trifluoride, arsenic tribromide, arsenic triiodide, selenium dioxide, selenous acid, sodium hydrogen selenite, magnesium selenite, calcium selenite, barium selenite, aluminum selenite, copper selenite, silver selenite, cerium selenite, barium selenate, copper selenate, iron selenide, zinc selenide, cadmium selenide, lead selenide, selenium chloride, selenium tetrachloride, selenium bromide, selenium tetrabromide, barium chloride, thallium, thallous oxide, thallium oxide, thallium hydroxide, thallous chloride, thallous bromide, thallous iodide, thallium triiodide, thallium nitrate, thallous sulfate, thallium (thallous) carbonate, thallous phosphate, beryllium, beryllium oxide, beryllium hydroxide, beryllium chloride, beryllium carbonate, beryllium sulfate, potassium beryllium sulfate, beryllium chromate, ammonium fluoberyllate, sodium fluoberyllate, osmium tetroxide, ammonium chlorosmate, vanadium pentoxide, vanadium chloride, potassium vanadate, potassium metavanadate, sodium metavanadate, ammonium metavanadate, ammonium polyvanadate, sodium ammonium vanadate, mercury arsenide, mercury nitrate, mercury fluoride, mercury iodide, mercury oxide, sodium tellurite, sodium nitroprusside, zinc phosphide, bromine, hydrogen bromide, germane, boron trifluoride, boron trichloride, and toxic metal ions (such as copper ion, aluminum ion, mercury ion, barium ion, lead ion, chromium ion, cadmium ion, and silver ion).

21. The composition according to claim 17, wherein the food-borne poison is selected from the group consisting of puffer fish toxin, ginkgo toxin, dolphin toxin, paralytic shellfish toxin, diarrheal shellfish toxin, neurotic shellfish toxin, amnestic shellfish toxin, ciguatoxin, scombroid toxin, conotoxin, polyether toxin, ciguatoxin, saxitoxin, maitotoxin, botulinum toxin, cyanogenic glycosides, linamarin, amygdalin, hydrocyanic acid, benzaldehyde, gossypols, solanine, muscarine, phallotoxin, amanita toxins, nitrite, histamines, lead, cadmium, mercury, arsenic, fluorine, polycyclic aromatic hydrocarbons, polychlorinated biphenyls, methanol, bongkrek acid, toxoflavin, deoxynivalenol, fusarenon-X, T2 toxin, triticum gibberellic disease toxin, aflatoxin, ochratoxin, phorbol esters, aplysiatoxin, okadaic acid, teleoeidin, 3-nitropropionic acid, gyromitrin, ethanol, clenbuterol hydrochloride (lean meat powder), caffeine, heroin, and theophylline.

22. The composition according to claim 17, wherein the disease-inducing molecule is selected from the group consisting of free DNA, an inflammatory factor, an antibody, an antigen, a protein fragment, and a pathogenic microorganism.

23. The composition according to any one of the preceding claims, wherein the toxin is present in the biofluid in a free state, in a bound state with a substance in the biofluid, or both.

24. The composition according to any one of the preceding claims, wherein the toxin is at least in part reversibly bound with the substance in the biofluid.

25. The composition according to claim 24, wherein the toxin is bound to the substance in the biofluid at a Kd value of at least 10² µmol/L, 10³ µmol/L, 10⁴ µmol/L, 10⁵ µmol/L, 10⁶ µmol/L, or 10⁷ µmol/L.

26. The composition according to claim 25, wherein the Kd value at which the toxin is bound to the substance in the biofluid is at least 10⁵-10⁷ µmol/L.

27. The composition according to any one of claims 23-26, wherein at least 10%, at least 20%, at least 30%, at least 40%, at least 50%, at least 60%, at least 70%, at least 80%, or at least 90% of the toxin in the biofluid is present in a bound state.

28. The composition according to claim 27, wherein the toxin comprises indoxyl sulfate, asymmetric arginine, homocysteine, phenylacetic acid, p-cresol, AGE products (3-deoxyglucosone, fructoselysine, glyoxal, pyruvaldehyde, pentosidine), hippuric acid, uremic toxins, hydrogen sulfide, or bilirubin.

29. The composition according to any one of claims 23-28, wherein the substances bound to the toxin comprises a component in blood tissue, a component in an adipose tissue, a component in a connective tissue, a component in a bone tissue, or the like.

30. The composition according to claim 29, wherein the toxin-removal reagent binds to the toxin via an adsorption or binding interaction that is different from the binding interaction between the toxin and the substance in the biofluid.

31. The composition according to claim 29, wherein the toxin-removal reagent is different from the substance bound to the toxin in the biofluid.

32. The composition according to any one of the preceding claims, wherein the osmotic agent provides an osmotic pressure substantially equal to or higher than 280 mOsm/L, 300 mOsm/L, or 330 mOsm/L.

33. The composition according to any one of the preceding claims, wherein the osmotic agent comprises a saccharide, an amino acid, a polypeptide, a glycerol, a carbonate or an analog thereof, and any combination thereof.

34. The composition according to any one of the preceding claims, wherein the osmotic agent which is saccharide is selected from a monosaccharide, a oligosaccharide and a polysaccharide; the osmotic agent which is an amino acid is selected from a natural amino acid, an unnatural amino acid, an analog thereof, a derivative thereof, and any combination thereof.

35. The composition according to claim 34, wherein the monosaccharide is selected from glucose, fructose, sorbitol, xylitol, aminosaccharide and a derivative thereof; the oligosaccharide comprises an oligomer of one or more of the monosaccharides; and/or the polysaccharide comprises a polymer of one or more of the monosaccharides.

36. The composition according to claim 35, wherein the osmotic agent which is a saccharide comprises a glucose polymer.

37. The composition according to any one of the preceding claims, wherein the amount ratio (weight/weight) of the toxin-removal reagent to the osmotic agent in the composition ranges from 1:1750 to 1:4 (e.g., 1:1750 to 1:5, 1:1500 to 1:4, 1:1500 to 1:5, 1:1000 to 1:5, 1:750 to 1:6; 1:300 to 1:6; 1:75 to 1:6; 1:30 to 1:6; 1:10 to 1:6; 1:1500 to 1:5; 1:1500 to 1:10; 1:1500 to 1:30; 1:1500 to 1:75; 1:1500 to 1:150; 1:1500 to 1:300; or 1:1500 to 1:750).

38. The composition according to any one of the preceding claims, wherein the composition further contains one or more of buffers, electrolytes and other dialysis components.

39. The composition according to claim 38, wherein the buffer is selected from one or more of lactate, bicarbonate, citrate, isocitrate, pyruvate, succinate, fumarate, malate, oxaloacetate, chloride, amino acids (histidine, glycine, alanine) with pK₁ of 7-13, and analogs thereof.

40. The composition according to claim 38, wherein the electrolyte is selected from one or more of sodium salts, calcium salts, magnesium salts, and potassium salts.

41. The composition according to any one of the preceding claims, wherein the composition is sterilized.

42. The composition according to any one of the preceding claims, wherein the composition is a solid preparation, a semi-solid preparation or a liquid preparation.

43. The composition according to claim 1, wherein the toxin-removal reagent induces osmosis with the biofluid through a semi-permeable substrate.

44. The composition according to claim 43, wherein the semi-permeable substrate is an artificial semi-permeable membrane.

45. The composition according to claim 43, wherein the semi-permeable substrate is a biological semi-permeable membrane.

46. The composition according to claim 45, wherein the biological semi-permeable membrane is selected from the group consisting of blood vessel wall membrane, lymphatic vessel wall membrane, peritoneum, lung membrane, glandular envelope and mucosa.

47. The composition according to any one of the preceding claims, wherein the biofluid is selected from blood, tissue fluid, lymph fluid, plasma, serum, a blood product, or a biological product.

48. The composition according to any one of the preceding claims, wherein the biofluid is inside the body of a subject or outside the body of a subject.

49. A dialysis solution comprising the composition according to any one of the preceding claims.

50. The dialysis solution according to claim 49, wherein the dialysis solution provides pH and/or electrolytes at a physiologically acceptable level.

51. A kit for purification of a biofluid, comprising the composition according to any one of claims 1-48.

52. The kit according to claim 51, wherein the composition is present in the form of a single composition, or in the form of two or more components.

53. The kit according to claim 52, wherein the composition or at least one of the components is a solid preparation, a semi-solid preparation or a liquid preparation.

54. The kit according to claim 52, wherein the composition is present in the form of two or more components that are contained in different containers respectively.

55. The kit according to claim 54, wherein the two or more components are respectively contained in two or more containers that can be operably connected via fluid.

56. The kit according to any one of claims 51-55, wherein the composition is sterilized.

57. The kit according to any one of claims 51-56, wherein the kit further comprises a semi-permeable substrate that can be used for the purification of the biofluid.

58. A dialysis device, comprising the composition according to any one of claims 1-48, wherein the device is configured to allow osmosis between the composition and the biofluid to be dialyzed.

59. The dialysis device according to claim 54, wherein the device further comprises a semi-permeable substrate that allows osmosis between the composition and the biofluid.

60. The dialysis device according to claim 54 or 55, wherein the dialysis device can be installed on a dialysis host.

61. A method for reducing a toxin in a biofluid, comprising:
a) contacting the biofluid with an osmotic solution comprising the composition according to any one of claims 1-48 under a condition to allow osmosis, and
b) allowing the composition to reduce the amount of the toxin in the biofluid.

62. The method according to claim 61, wherein the toxin in the biofluid is transferred to the osmotic solution through osmosis under the condition to allow osmosis.

63. The method according to any one of claims 61-62, wherein the step a) comprises placing the biofluid and the composition respectively at two sides of a semi-permeable substrate.

64. The method according to claim 63, wherein the osmotic solution is substantially isotonic or hypertonic to the biofluid.

65. The method according to claim 63, wherein the semi-permeable substrate is an artificial semi-permeable membrane.

66. The method according to claim 63, wherein the semi-permeable substrate is a biological semi-permeable membrane.

67. The method according to claim 66, wherein the biological semi-permeable membrane is blood vessel wall membrane, lymphatic vessel wall membrane, peritoneum, lung membrane, glandular envelope and mucosa.

68. The method according to any one of claims 61-62, wherein the biofluid is inside the body of a subject.

69. The method according to claim 68, wherein the step a) comprises applying the osmotic solution to the subject by intraperitoneal infusion.

70. The method according to any one of claims 61-62, wherein the biofluid is outside the body.

71. The method according to claims 70, wherein the step a) comprises applying the osmotic solution to the subject by hemodialysis.

72. A method of treating or preventing a toxin-related disease or condition, comprising contacting the composition according to any one of claims 1-48 with the biofluid of a subject under a condition to allow osmosis, such that the toxin in the biofluid is reduced.

73. Use of the composition according to any one of claims 1-48 in the manufacture of a medicament for treating or preventing a toxin-related disease or condition, wherein the composition reduces the toxin in a biofluid in a subject.

74. The method or use according to claim 72 or 73, wherein the toxin-related disease or condition comprises kidney disease, cardio-cerebrovascular disease, blood disease, autoimmune disease, metabolic disease, orthopedic disease, digestive system disease, drug overdose or poisoning.

75. The method or use according to any one of claims 72-74, wherein the kidney disease is selected from uremia, chronic nephropathy, acute renal insufficiency, chronic pyelonephritis, acute pyelonephritis, chronic glomerulonephritis, acute progressive nephritis syndrome, nephrotic syndrome, nephrosclerosis, interstitial nephritis, diabetic nephropathy, focal glomerulosclerosis, membranous nephropathy, multiple pustular renal syndrome, renovascular hypertension and hypertension syndrome, secondary nephropathy, hyperphosphatemia, hyperkalemia, hyperuricemia or hypernatremia.
